# EUROPEAN PATENT SPECIFICATION

(11) **EP 3 051 290 B1**
(45) Date of publication and mention of the grant of the patent: **14.03.2018**
(21) Application number: 16153380.7
(22) Date of filing: 29.01.2016
(51) Int. Cl.: G01N 33/497, A61B 10/00

(54) **BIOLOGICAL INFORMATION MEASUREMENT SYSTEM**
SYSTEM ZUR MESSUNG BIOLOGISCHER INFORMATIONEN
SYSTÈME DE MESURE D'INFORMATIONS BIOLOGIQUES

(30) Priority: 30.01.2015 JP 2015017448; 29.09.2015 JP 2015191420; 29.09.2015 JP 2015191421
(43) Date of publication of application: 03.08.2016
(73) Proprietor: Toto Ltd., Fukuoka 802-8601 (JP)
(72) Inventor: Kizuka, Satoko, Kitakyushu-shi, Fukuoka 8028601 (JP); Tsuboi, Hiroshi, Kitakyushu-shi, Fukuoka 8028601 (JP); Nagaishi, Masayuki, Kitakyushu-shi, Fukuoka 8028601 (JP); Sonoda, Koji, Kitakyushu-shi, Fukuoka 8028601 (JP); Oka, Hidenori, Kitakyushu-shi, Fukuoka 8028601 (JP); Takeshita, Akemi, Kitakyushu-shi, Fukuoka 8028601 (JP); Yamamoto, Masahiro, Kitakyushu-shi, Fukuoka 8028601 (JP); Sakaguchi, Akitoshi, Kitakyushu-shi, Fukuoka 8028601 (JP)
(74) Representative: Zimmermann & Partner Patentanwälte mbB

(56) References cited:
- WO-A1-2004/008953
- JP-A- 2014 160 049
- JP-B2- 5 131 646
- THOMAS SCHLEBUSCH ET AL: "Intelligent Toilet System for Health Screening", 2 September 2011 (2011-09-02), UBIQUITOUS INTELLIGENCE AND COMPUTING, SPRINGER BERLIN HEIDELBERG, BERLIN, HEIDELBERG, PAGE(S) 152 - 160, XP019162482, ISBN: 978-3-642-23640-2 * abstract; figures 1,2 *
- J. Elders: "C2V-200: a NeSSI fast micro gas chromatograph", , 27 January 2009 (2009-01-27), XP055350604, Retrieved from the Internet: URL:http://depts.washington.edu/cpac/NeSSI /40_IFPAC_09/Presentations/Job Elders NeSSI III reconfigurable Gas Analzyer IFPAC.pdf [retrieved on 2017-03-01]
- YANG M ET AL: "Development of a Palm Portable Mass Spectrometer", JOURNAL OF THE AMERICAN SOCIETY FOR MASS SPECTROMETRY, ELSEVIER SCIENCE INC, US, vol. 19, no. 10, 1 October 2008 (2008-10-01), pages 1442-1448, XP025480691, ISSN: 1044-0305, DOI: 10.1016/J.JASMS.2008.05.011 [retrieved on 2008-05-24]

## Description

### BACKGROUND OF THE INVENTION

### Field of the Invention

The present invention relates to a biological information measurement system, and more particularly to a biological information measurement system that measures physical condition of a test subject on the basis of defecation gas discharged in a bowl of a toilet installed in a toilet installation room.

### Description of the Related Art

In recent years, a mortality rate caused by cancer extremely decreases due to evolution of a diagnosis technique for serious illness, such as cancer, and of a technique of cancer treatment, with evolution of medical technology. However, presenting to a hospital at regular intervals for diagnosis to prevent cancer burdens a patient. In contrast, many patients actually present to a hospital after realizing wrong physical condition, and thus unfortunately still many people have cancer. In addition, no practical device for preventing cancer has been developed yet, so that it cannot be said that cancer prevention is sufficiently achieved.

In light of the circumstances, the present inventors have studied for a long time with a strong desire for manufacturing a device that is really required in the market, such as a device capable of more simply and easily diagnosing serious illness, such as cancer, at home without presenting to a hospital, to achieve prevention or early treatment of serious illness.

The present applicants have developed devices, such as: a device that is mounted in a seat of a Western-style toilet to collect defecation gas discharged into a bowl when a test subject defecates to acquire the amount of stool discharged on the basis of a concentration of carbon dioxide contained in the defecation gas as a biological information index (refer to Patent Literature 1: Japanese Patent No. 5131646); and a device in which a deodorizing device assembled in a seat of a flush toilet sucks defecation gas that is discharged together when a test subject defecates so that a carbon dioxide gas sensor measures a concentration of carbon dioxide of the gas sucked to allow intestinal conditions of a test subject to be estimated on the basis of the measured concentration of carbon dioxide (refer to Patent Literature 2: Japanese Patent No. 5019267). Unfortunately, these devices estimate only current intestinal conditions, so that it is impossible to achieve a purpose of the present inventors to enable serious illness, such as cancer, to be simply and easily diagnosed, as well as to enable a risk state of the serious illness to be simply and easily acquired. In addition, there is also known a fart detector in which gas sensor is arranged so as to be brought into contact with air near an excretory organ of a human to detect a fart on the basis of a peak value of output of the gas sensor (refer to Patent Literature 3: Japanese Patent Laid-Open No. 2003-90812). In the fart detector, a tube inserted into an excretory organ of a patient staying in bed in a diaper or underwear worn by the patient is drawn, and air is sucked through the tube by a suction pump to collect a fart of the patient. In addition, the fart detector only distinguishes a fart and urination on the basis of a half-value width of a peak value of output of the gas sensor so that a doctor checks whether a fart is discharged after an appendix operation, or time to replace a diaper is detected, whereby it is impossible to achieve the purpose of the present inventors. Meanwhile, Japanese Patent Laid-Open No. 2014-160049 (Patent Literature 4) discloses a portable type apparatus for measuring a risk of colorectal cancer that includes a sensor for measuring methyl mercaptan gas from components of a fart discharged by a test subject, a calculation unit for calculating a concentration of the methyl mercaptan gas measured by the sensor, and a display, to estimate a risk of acquiring colorectal cancer.

Japanese Patent Laid-Open No. 9-43182 (Patent Literature 5) describes a biological monitoring device. In the biological monitoring device, a fabric T-bandage to which gas sensor is attached is provided so that the gas sensor is arranged near an anus to detect a fart discharged from the anus. A signal from the gas sensor is transmitted to a processor to be stored in a memory. It is also known that data stored in a memory is compared with previous data, and that a warning is displayed in a display device if there is abnormality, such as a large difference.

Japanese Patent No. 3525157 (Patent Literature 6) describes a method of measuring components of flatus. In the method of measuring components of flatus, a sampling tube is arranged at a portion in a seat of a toilet. When a person to be measured turns on a main switch of a device, a suction pump is operated to suck gas near an anus. An index gas detector always measures a concentration of carbonic acid gas in the gas sucked, and a control/arithmetic processing unit recognizes that a flatus has been diffused if the concentration measured steeply increases. If a flatus is diffused, another suction pump starts operating to allow a part of gas sucked to be inserted into a sample measuring tube. An inserted sample is fed into a column so that gas components are separated to be ionized. It is also known that the amount of ionization is converted into an electric signal so that a concentration of gas components of a detection object in the flatus is measured.

Japanese Patent Laid-Open No. 2014-206945 (Patent Literature 7) describes a health information utilization system. In the health information utilization system, personal health information on health management, inputted from a terminal device, is individually stored in a database of each of a plurality of data centers, and an analysis server device reads out the personal health information to analyze it. A big data creation server device searches the personal health information under a specific condition to create big data and store it. The health information utilization system allows health content based on knowledge in a special field to be browsed at a terminal device, and stores the personal health information in the plurality of data centers to manage it, as well as allows a health determination result acquired by applying automatic determination processing to the personal health information, and a health determination result acquired by determination processing applied by an expert, to be browsed at a terminal. The system described above is also known.

In order to develop a device capable of diagnosing serious illness, such as cancer, in recent years, it has been known that there is a correlation between a disease of colorectal cancer and components of a flatus contained in a fart and a stool. Specifically, colorectal cancer patients have more methyl mercaptan gas containing a sulfur component, in components of flatus, as compared with healthy people.

Components of flatus are discharged along with a stool, as a fart and defecation gas, during defecation. Thus, the present inventors, as published in Nihon Keizai Shimbun issued January 5, 2015, have studied on the assumption that measuring a specific gas, such as methyl mercaptan gas, in a fart and defecation gas, discharged during defecation, enables colorectal cancer in the intestine to be found out, as with Patent Literature 4 above, and the like. However, a measuring device capable of accurately measuring only this specific gas, such as methyl mercaptan gas, is very expensive and large in size. In addition, methyl mercaptan gas is contained in minute amount in defecation gas, and is contained in less amount than the minute amount in a stage before getting cancer. As a result, it is very difficult to measure the methyl mercaptan gas, and thus the present inventors have been faced with a problem in which it is not realistic in cost and size that at least this kind of gas analyzer capable of accurate measurement is assembled in a household toilet device to be widely used as a consumer product.

The patent application WO 2004/008953 A1 discloses a method for determining the cause of a disease by the analysis of fingerprinting the volatile organic compounds evolved from a stool sample.

However, the present inventors continue to study by having strong feeling for necessity of providing a device that is capable of allowing general consumers to readily purchase it, and capable of simply and easily performing diagnosis at home, in order to reduce the number of people who have a serious illness, such as cancer, as far as possible, and then finally find out a technical solution for realizing the device.

It is an object of the present invention to provide a diagnosis system that is capable of allowing general consumers to readily purchase it, as well as capable of measuring defecation gas at home to prevent people from having a serious disease, such as a cancer, or urging people to present to a hospital to receive treatment under a moderate condition, the diagnosis system being really required in the market, having high practicality.

### SUMMARY OF THE INVENTION

In order to solve the problem described above, the present invention is a biological information measurement system that measures physical condition of a test subject on the basis of defecation gas discharged into a bowl of a flush toilet installed in a toilet installation space, and the biological information measurement system includes: a test subject identification device for identifying a test subject who uses the flush toilet; a suction device that sucks gas in the bowl into which the defecation gas was discharged by the test subject; a gas detector provided with a gas sensor that is sensitive to methyl mercaptan gas of odiferous gas, containing a sulfur component, as well as to odiferous gas other than the methyl mercaptan gas, included in the defecation gas sucked by the suction device; a control device that controls the suction device and the gas detector; a storage device that stores first detection data acquired by the gas detector for each test subject identified by the test subject identification device; a data analyzer that analyzes physical condition of a test subject on the basis of time-dependent change in a plurality of first detection data items that is detected in a defecation act performed multiple times in a predetermined period, and that is stored in the storage device; and an output device that outputs an analysis result acquired by the data analyzer.

Heretofore, there has been actually no effective device other than diagnosis at hospital for checking whether people have serious illness, such as cancer, or for checking people for prevention of serious illness. In contrast, according to the present invention, general consumers can simply and easily purchase the device to perform measurement at home. In addition, it is possible to allow a test subject to be prevented from having a serious disease, such as cancer, or to present to a hospital to receive treatment under a moderate condition, by only performing an excretory act as usual to measure defecation gas discharged during defecation without making an effort to perform additional measurement action. In this way, the present invention achieves an excellent effect of enabling a device that is really required in the market to be realized and a diagnosis system having high practicality to be provided.

Before advantageous effects of the present invention is specifically described, a technical idea of allowing a system to be widely used at standard home as a consumer product will be described. Key point of the idea are reverse thinking and effective simplified knowledge acquired by understanding characteristics of serious illness, such as cancer, and using the characteristics.

Specifically, one of key points of a system of the present invention is acquired by reverse thinking of a device installed at each home by which people are not diagnosed as having serious illness, such as cancer. That is, a test subject of general consumers really wants to know whether to be in a stage before having cancer (hereinafter this stage is referred to as ahead-disease), instead of whether to have cancer, to recognize an increasing a risk of cancer to improve a future life for preventing having cancer. Thus, it is thought that a device capable of allowing health people to accurately recognize a risk of cancer to improve physical condition for preventing having cancer is worth to a device required at standard home.

Another key point of the system of the present invention is acquired by a simplified idea that a device capable of diagnosing a specific kind of cancer, such as a rectal cancer, or diagnosing an increasing risk of a specific kind of cancer, is unnecessary. The idea is acquired from characteristics of a test subject who is anxious about any kind of cancer instead of about a specific kind of cancer, such as a rectal cancer. Thus, the inventors have simply thought that accuracy of measurement capable of identifying a kind of cancer is unnecessary, on the basis of an assumption that it is quite unnecessary to identify a kind of cancer instead of an assumption that device has a commercial value if diagnosing a specific kind of cancer.

Yet another key point of the system of the present invention is acquired by a simplified idea that extremely low diagnosis accuracy for each excretory act may be allowed. The idea is acquired on the basis of characteristics of cancer that develops for a long time, such as a few years, so that an occasion of diagnosis occurs for a long time by year. Thus, it is found that influence of even low diagnosis accuracy at one time does not substantially matter if a device is provided to allow healthy people to reduce their risk for having cancer, by themselves, whereby an effective simplified idea based on the matter found becomes one of the keys.

Specific effects of a system in accordance with the present invention configured on the basis of the knowledge and the effective simplified idea described above will be described below.

In the present invention, since defecation gas discharged into a bowl of a toilet is measured to analyze physical condition of a test subject, it is possible to perform diagnosis by allowing a test subject to only defecate as usual without requiring an effort to perform measurement action. Requiring no effort allows the test subject to have no burden, so that it is possible to continue measurement for a long time to reliably acquire information on a change in health condition, and on a state where a risk of cancer is increasing.

In addition, in the present invention, no sensor for measuring methyl mercaptan gas at a pinpoint is used, and a sensor that is widely sensitive also to odiferous gas other than the methyl mercaptan gas, in defecation gas, is used. If the sensor for measuring methyl mercaptan gas at a pinpoint is used, it is possible to reliably detect a colorectal cancer because there is a correlation between the amount of methyl mercaptan gas and a colorectal cancer, and also to reliably find that a risk of cancer is increasing from the amount thereof. However, it is found that it is impossible to determine that a risk of cancer is increasing unless a risk of cancer increases to some extent to increase the amount of methyl mercaptan gas, whereby the sensor is unsuitable for the present invention having an object to prevent people from having cancer.

In contrast, the sensor that is widely sensitive to odiferous gas is capable of detecting not only a state where a risk of cancer is increasing, but also a risk of cancer from wrong physical condition. Specifically, first if a risk of cancer increases, a very strong odiferous gas containing a sulfur component, such as methyl mercaptan gas or hydrogen sulfide, increases in amount. Then, the sensor that is widely sensitive to odiferous gas is capable of detecting increase of this kind of gas. As described later, although the amount of odiferous gas temporarily increases due to change of physical condition by day, a state of having an increased very strong odiferous gas containing a sulfur component, such as methyl mercaptan gas or hydrogen sulfide, continues for a long time if people have cancer. Thus, even if a sensor that is widely sensitive to odiferous gas other than methyl mercaptan gas in defecation gas is used, it is possible to determine that there is a high possibility of disease of cancer to cause a risk of cancer to increase if the amount of gas is high for a long time. Accordingly, the sensor that is widely sensitive also to odiferous gas serves also as a sensor for measuring methyl mercaptan gas at a pinpoint in this point.

The present invention uses a gas detector that is sensitive not only to methyl mercaptan gas but also to odiferous gas other than methyl mercaptan gas, in defecation gas, so that only the amount of odiferous gas in the defecation gas can be detected, but the amount of methyl mercaptan gas cannot be measured, whereby it is impossible to accurately identify a state of cancer. However, the present inventors find out that using gas detector that is sensitive not only to methyl mercaptan gas, but also to odiferous gas other than methyl mercaptan gas, in defecation gas, allows a device to effectively serve as a device for preventing a state where a risk of cancer increases in healthy people, and a risk, such as having cancer. Specifically, healthy people have a small total amount of methyl mercaptan gas and odiferous gas other than the methyl mercaptan gas. In contrast, a total amount of methyl mercaptan gas and odiferous gas other than the methyl mercaptan gas temporarily increases due to deterioration of intestinal environment other than having cancer. The deterioration of intestinal environment is specifically caused by the following, such as excessive obstipation, a kind of meal, lack of sleep, crapulence, excessive drinking, and excessive stress. It can be said that each of these causes is a bad living habit. The bad living habit will result in cancer, however, there is no means of recognizing a risk of cancer state even if the risk of cancer increases, and thus many people continue the bad living habit on the basis of a convenient assumption that the many people themselves survive.

In this way, performing the bad living habit as described above increases all or any one of odiferous gases in defecation gas, such as methyl mercaptan, hydrogen sulfide, acetic acid, trimethylamine, or ammonia. In contrast, the present invention analyzes physical condition on the basis of detection data acquired by gas detector that detects not only methyl mercaptan gas, but also odiferous gases other than methyl mercaptan gas, such as hydrogen sulfide, acetic acid, trimethylamine, or ammonia, in defecation gas. Thus, an analysis result based on a total amount of the odiferous gas in the defecation gas reflects a result caused by a wrong physical condition and a bad living habit, of a test subject, so that the analysis result is usable as an index based on objective data for improving a physical condition and a living habit in which this kind of risk of cancer may increase, or is usable as an effective index for maintaining a health condition to reduce a risk of having cancer, whereby it is found that the analysis result acts on the object of improving a living habit and reducing a risk of cancer in an extremely effective manner to achieve an excellent effect.

In this way, the present invention measures methyl mercaptan gas and odiferous gas other than the methyl mercaptan gas to enable measurement capable of notifying a state where a risk of cancer may increase, and a suitable warning of having cancer if this kind of state continues for a long time, to a test subject. The so-called reverse thinking allows knowledge suitable for the object of reducing people having cancer to be found out.

In addition, since the present invention uses a sensor that is widely sensitive not only to methyl mercaptan gas but also to odiferous gas other than the methyl mercaptan gas, a device can be manufactured at low cost, thereby enabling the device to be provided as a consumer product. Accordingly, it is possible to sufficiently satisfy a request of test subjects that diagnosis can be simply and easily performed at home to prevent having a serious disease, such as cancer, or they can be urged to present to a hospital to receive treatment under a moderate condition.

According to the present invention configured in this way, first detection data acquired by the gas sensor sensitive to methyl mercaptan gas of odiferous gas, containing a sulfur component, as well as to odiferous gas other than the methyl mercaptan gas, is stored for each test subject, and physical condition of a test subject is analyzed on the basis of time-dependent change in a plurality of first detection data items in a defecation act performed multiple times in a predetermined period. As a result, it is possible to chronologically grasp the amount of odiferous gas containing a sulfur component, in defecation gas, for a long period by using detection data acquired by the gas detector using the gas sensor sensitive to methyl mercaptan gas of odiferous gas, containing a sulfur component, as well as to odiferous gas other than the methyl mercaptan gas, whereby it is possible to notify a wrong physical condition to a test subject in a state of ahead-disease before having a serious disease, such as colorectal cancer. Then, it is possible to provide a biological information measurement system at a cost, allowing general consumers to readily purchase it. According to the biological information measurement system of the present invention, it is possible to prevent people from having a serious disease, such as a cancer by measuring defecation gas at home, or to urge people to present to a hospital to receive treatment under a moderate condition.

In the present invention, the gas detector is configured to react to healthy-state gas composed of at least one of hydrogen gas, carbon dioxide gas, and methane gas, contained in gas sucked by the suction device, to output second detection data, and that the data analyzer acquires a relationship between a first index related to odiferous gas containing a sulfur component, acquired on the basis of first detection data, and a second index related to healthy-state gas, acquired on the basis of second detection data in one defecation act, to analyze physical condition of a test subject on the basis of time-dependent change in the acquired relationship in a defecation act performed multiple times.

In the present invention configured in this way, the gas detector is configured to output second detection data related to healthy-state gas, and the data analyzer analyzes physical condition of a test subject on the basis of a relationship between the first index related to odiferous gas containing a sulfur component and the second index related to healthy-state gas.

In this way, in the present invention physical condition is analyzed on the basis of first detection data on odiferous gas containing a sulfur component and second detection data on healthy-state gas. The present invention is based on excellent technical knowledge acquired by the present inventors, in which analysis based on odiferous gas containing a sulfur component and healthy-state gas dramatically improves accuracy in analysis to enable securing accuracy in analysis of the order of a change in intestinal conditions and an increase in a risk of cancer even if simplified measurement is performed. Specifically, the amount of odiferous gas temporarily increases or decreased according to change in physical condition, and the amount of odiferous gas reliably continuously increases in a state where a risk of cancer increases due to environmental change in intestine. Then, the amount healthy-state gas reliably continuously decreases so as to be inversely proportional to the increase in the amount of odiferous gas. Thus, using a relationship above between the amount of odiferous gas and the amount of healthy-state gas enables performing accurate analysis of physical condition on the basis of the odiferous gas containing a sulfur component and the healthy-state gas, with even simplified analysis, without accurately grasping a total amount of odiferous gas by collecting all defecation gas of a test subject. Accordingly, it is possible to detect change in physical condition with simplified analysis based on a relationship between odiferous gas and healthy-state gas, in defecation gas collected in a short period, as well as to accurately analyze an increase in a risk of cancer. The present invention based on this knowledge enables useful analysis even if a device performing simplified analysis is used.

Meanwhile, since odiferous gas is in extremely trace amounts, analysis with only odiferous gas causes a problem in a measurement system and measurement accuracy if a measurement error is also considered. To secure sufficient reliability of measurement data, an expensive sensor for odiferous gas, with high sensitivity, is required, so that it is difficult to reduce an increase in price of a device. The present invention of the configuration described above uses a method of analysis, based on the technical knowledge described above found by the present inventors, to enable securing sufficient reliability of analysis by only adding an inexpensive sensor for healthy-state gas to a simple sensor for odiferous gas without using an expensive sensor, as well as enable dramatically increasing accuracy of analysis of physical condition.

In the present invention, it is preferable that there is further provided with a test-subject-information storage device that stores information on weight, age, and sex of a test subject, or information on elapsed time from a previous defecation act, and that the data analyzer analyzes physical condition of a test subject on the basis of first detection data, second detection data, and information on a test subject stored in the test-subject-information storage device.

Since the present invention configured in this way analyzes physical condition of a test subject on the basis of information on the test subject in addition to first detection data, and second detection data, it is possible to accurately measure the physical condition. That is, the present inventors find that the amount of odiferous gas containing a sulfur component, included in defecation gas, varies depending on age and sex of a test subject. Since the present invention configured as described above analyzes physical condition of a test subject in consideration of information on the test subject, a personal difference in the amount of odiferous gas containing a sulfur component can be absorbed to enable accurately measuring physical condition, as well as enable preventing an unnecessary mental burden from being applied to a test subject due to notification of a wrong result.

In the present invention, it is preferable that the output device displays a plurality of analysis results related to every defecation act analyzed by the data analyzer in a time-dependent manner.

Since the present invention configured in this way allows the output device to display a plurality of analysis results related to every defecation act in a time-dependent manner, a test subject can grasp his or her own physical condition as time-dependent change, whereby it is possible to urge the test subject to perform health management, such as improvement in a living habit.

In the present invention, it is preferable that the output device displays a plurality of analysis results in a time-dependent manner in a physical condition display table, provided with a first axis representing the first index acquired on the basis of first detection data, and a second axis representing the second index acquired on the basis of second detection data.

Since the present invention configured in this way displays a plurality of analysis results in a time-dependent manner in the physical condition display table, provided with the first axis related to odiferous gas containing a sulfur component, and the second axis related to healthy-state gas, a test subject can visually grasp his or her own change in physical condition, whereby it is possible to easily determine his or her own physical condition.

In the present invention, it is preferable that the physical condition display table is divided into a plurality of stage areas with respect to whether physical condition is good or bad, and a plurality of analysis results is plotted in a time-dependent manner in the physical condition display table divided into the areas.

Since the present invention configured in this way plots a plurality of analysis results in a time-dependent manner in the physical condition display table divided into the plurality of stage areas, with respect to whether physical condition is good or bad, a test subject can visually determine what level of physical condition corresponds to his or her own physical condition, whereby it is possible to make an effort for health management.

In the present invention, it is preferable that the suction device and the gas detector are configured to suck gas and detect first detection data, respectively, even before the test subject identification device identifies a test subject who uses the toilet, and that the storage device stores first detection data in association with the test subject identified by the test subject identification device after the first detection data is acquired.

The biological information measurement system of the present invention enables automatically measuring physical condition in a toilet installation room by only defecating as with usual defecation. However, if a test subject who defecates is not identified, the storage device cannot accumulate detection data. Meanwhile, since a test subject is required to perform operation for identifying a test subject for each defecation every day, it tends to be reluctant to perform the operation even if it is simple. Sometimes, a test subject wants to defecate immediately after entering a toilet installation room. If a test subject does not perform operation for identifying a test subject due to this kind of reason, there is a problem in which a biological information measurement system is not effectively used even if installed. Since the present invention configured as described above acquires detection data even before a test subject is identified, a test subject is released from reluctance to input specific information on a test subject before defecation. In addition, since a test subject can perform operation for identifying a test subject, even after entering a toilet installation room and siting on a seat to start defecation, or even after defecation is finished, it is possible to greatly reduce an operating burden when physical condition is measured. Accordingly, a test subject easily continues to measure physical condition without receiving an excessive operating burden.

In the present invention, it is preferable that if a test subject does not input information of identifying a test subject into the test subject identification device for a predetermined time after the gas detector acquires first detection data, the output device outputs notification of urging the test subject to input the information.

Since the present invention configured in this way notifies a test subject if the test subject does not input specific information on a test subject for a predetermined time, it is possible to prevent input of specific information on a test subject from being forgotten, whereby it is possible to easily continue to measure physical condition.

In the present invention, it is preferable that the control device is configured to control cleaning of the toilet, and that if a test subject does not input information of identifying a test subject into the test subject identification device after the gas detector acquires first detection data, the control device does not allow the cleaning of the toilet.

If it is expected that a measurement result may not be good, a test subject measuring his or her physical condition tends to be reluctant to positively measure the physical condition. In this kind of case, it is thought that a test subject may not intentionally perform operation for identifying a test subject when defecating to avoid measuring physical condition. Since the present invention configured as described above does not allow cleaning of the toilet if a test subject does not input specific information on a test subject, the test subject is partly urged to input the specific information on a test subject, and recognizes a result of measurement of physical condition. Accordingly, it is possible to reliably continue to measure physical condition as well as to strongly urge a test subject to manage his or her physical condition, whereby it is possible to prevent a continual wrong physical condition from developing into a serious disease.

In the present invention, it is preferable that the output device corrects an analysis result acquired by the data analyzer to plot the corrected analysis result in the physical condition display table.

Since the present invention configured in this way plots a corrected analysis result in the physical condition display table, it is possible to prevent a large variation of physical condition to be displayed due to a large error to be included in detection data, or a temporary wrong physical condition.

In the present invention, it is preferable that if a most recent analysis result is varied to a wrong physical condition side, the output device corrects an analysis result to be plotted in the physical condition display table to a good physical condition side to display the corrected analysis result.

Since the present invention configured in this way corrects an analysis result to be plotted in the physical condition display table to a good physical condition side, it is possible to prevent physical condition to be displayed from remarkably deteriorating to apply an excessive mental burden to a test subject, due to a large error to be included in detection data, or a temporary wrong physical condition.

In the present invention, it is preferable that if an analysis result showing a wrong physical condition continues predetermined times or more, the output device reduces an amount of correction with respect to the analysis result to display the corrected analysis result.

Since the present invention configured in this way reduces the amount of correction if an analysis result showing a wrong physical condition continues predetermined times or more, a result showing a wrong physical condition is displayed for a continual wrong physical condition and the wrong physical condition can be notified to a test subject before greatly deteriorating, whereby it is possible to urge the test subject to manage health, to present to a hospital, or the like.

In the present invention, it is preferable that the output device displays a message related to health management of a test subject on the basis of an analysis result acquired by the data analyzer.

Since the present invention configured in this way displays a message related to health management of a test subject, the test subject can take an appropriate action on the basis of his or her own physical condition displayed, whereby it is possible to early address improvement in physical condition.

In the present invention, it is preferable that the data analyzer calculates reliability of detected data, on the basis of first detection data and second detection data, and that the output device plots an analysis result corrected on the basis of the reliability of the data in the physical condition display table.

In the present invention using a sensor widely sensitive to odor components, first detection data may be affected by stink gas components, such as sweat and urine, and perfume, attached to a test subject, as well as stools attached to a toilet bowl, odor gas and an aromatic, remaining in a toilet space, an alcoholic disinfectant, and the like. In particular, as a stronger perfume or aromatic is used, or as a body and a toilet space becomes more unsanitary, measurement accuracy may decrease. In addition, there is a high possibility that if the sensor is used immediately after another person defecates, stink components, such as defecation gas and an attached odor, of the prior person, may remain in a toilet bowl or in a toilet space, whereby it is thought that measurement may be affected regardless of sanitary conditions. In contrast, since the present invention of the configuration described above corrects an analysis result on the basis of reliability, it is possible to prevent a plotted point to be displayed in the physical condition display table from greatly varying due to an analysis result with low reliability to prevent an unnecessary mental burden from being applied to a test subject.

In the present invention, it is preferable that if reliability of data, calculated by the data analyzer, is low, the output device increases the amount of correction for allowing an analysis result to be plotted in the physical condition display table to be closer to a previous plotted points side than a case where reliability of data is high.

Since the present invention configured in this way increases the amount of correction for an analysis result to be plotted in the physical condition display table to allow the analysis result to be close to the previous plotted points side if reliability of data is low, it is possible to prevent a plotted point from greatly varying to a wrong physical condition side due to data with low reliability to prevent an unnecessary mental burden from being applied to a test subject.

In the present invention, it is preferable that the data analyzer calculates reliability of data at a lower value as noise included in first detection data and second detection data increases.

Since the present invention configured in this way calculates reliability of data at a lower values as noise included in detection data increases, it is possible to prevent a plotted point from greatly varying to a wrong physical condition side on the basis of detection data with low accuracy, measured in an environment with many noise components, such as residual gas, to prevent an unnecessary mental burden from being applied to a test subject.

In the present invention, it is preferable that the data analyzer performs analysis by assigning a higher weight coefficient to first detection data and second detection data, acquired by detecting defecation gas discharged early in one defecation act of a test subject, than that to first detection data and second detection data acquired by detecting defecation gas discharged later in the one defecation act.

Although it has been thought that methyl mercaptan gas is created by a cancer cell to cause a large amount of the gas to be discharged at a timing corresponding to a position of the cancer cell during a defecation period, the present inventors find that a large amount of the gas tends to be discharged early in a period of a defecation act. It is thought that characteristics in which gas can be discharged easier than stool during defecation, and characteristics in which created gas gathers around an anus, may cause the finding. Since the present invention based on the finding assigns a higher weight coefficient to detection data acquired by detecting defecation gas discharged early than that to later detection data, it is possible to perform accurate measurement. In addition, measuring defecation gas discharged early enables presenting an analysis result to a test subject at an end of one defecation act, or immediately after the one defecation act, so that it is possible to present a measurement result of physical condition without allowing a test subject to excessively wait.

In the present invention, it is preferable that the data analyzer performs analysis by using only first detection data and second detection data, acquired by detecting defecation gas discharged first in one defecation act of a test subject.

Since the present invention configured in this way performs analysis by using only detection data on defecation gas discharged first, it is possible to perform useful measurement of physical condition, with high reliability, by analysis of a small amount of data.

In addition, a test subject measuring his or her physical condition tends to be reluctant to positively recognize a measurement result of physical condition if it is expected that the measurement result may not be good. In this kind of case, if a measurement result of physical condition is outputted after a test subject finishes one defecation act, it is thought that the test subject may leave a toilet installation room after finishing the defecation act without waiting for a measurement result of physical condition to be outputted. Since the present invention configured as described above uses only detection data on defecation gas discharged first to perform analysis, it is possible to output a measurement result of physical condition while a test subject sits on a seat before finishing a defecation act, whereby it is possible to reliably present a measurement result to even a test subject who is reluctant to positively recognize a measurement result of physical condition.

In the present invention, it is preferable that the physical condition display table is provided with the first axis representing the first index, and the second axis representing the second index, and in the physical condition display table, there are set a first region showing a predetermined physical condition level, and a second region showing wrong physical condition as compared with the first region, and at least a part of a boundary line between the first region and the second region is drawn so that a value of the first index increases with increase in a value of the second index, the second region showing wrong physical condition being distributed on a side across the boundary line, where a value of the first index is large.

Since a conventional health condition measuring device using defecation gas measures only carbon dioxide of healthy gas to measure physical condition, a measurement result of health condition is determined by only the amount of carbon dioxide. However, even if a large amount of carbon dioxide is created in a state of ahead-disease before having a serious disease, physical condition sometimes may start to deteriorate. According to the present invention configured as described above, a different evaluation of health condition is made depending on a value in the first axis representing the first index even if a value in the second axis representing the second index is identical, so that it is possible to perform more accurate measurement of physical condition.

In the present invention, it is preferable that the gas detector includes both a sensor for detecting hydrogen gas and a sensor for detecting carbon dioxide gas.

Since the present invention configured in this way includes a sensor for detecting hydrogen gas and a sensor for detecting carbon dioxide gas, it is possible to evaluate healthy gas indicating good physical condition on the basis of two kinds of gas, whereby it is possible to more accurately measure physical condition.

In the present invention, it is preferable that the gas detector is configured to be able to detect also vaporized short-chain fatty acid contained in defecation gas sucked by the suction device, and that the data analyzer analyzes the first index based on detection data on odiferous gas, the second index based on detection data on healthy-state gas, and a third index based on detection data on short-chain fatty acid, as physical condition of a test subject.

Since the present invention configured in this way analyzes physical condition on the basis of the first, second, and third indexes, it is possible to measure a wide range of physical condition from condition in which there is suspicion of disease to condition in which there is high immune strength against disease, as well as to perform more reliable measurement of physical condition.

In the present invention, it is preferable that the data analyzer is configured to analyze whether physical condition is good or bad on the basis of the first and second indexes to output an analysis result to the output device, and that if a value of the third index is large, the data analyzer outputs an analysis result, in which an analysis result based on the first and second indexes is greatly corrected to a good physical condition side as compared with a case where a value of the third index is small, to the output device.

In the first and second indexes, there are many error factors, such as condition of defecation, and noise of a measurement environment. In contrast, the present inventors find that short-chain fatty acid is a component created only under conditions where condition in intestine is good to have many good bacteria. Since the present invention configured as described above corrects an analysis result based on the first and second indexes to the good physical condition side if the third index based on short-chain fatty acid is large, it is possible to prevent an unnecessary mental burden from being applied to a test subject due to a measurement error, or the like, in the first and second indexes.

In the present invention, it is preferable that the gas detector is configured to be able to detect acetic acid or propionic acid of short-chain fatty acid, and that the data analyzer analyzes physical condition of a test subject on the basis of a time-dependent tendency of change in detection data on acetic acid or propionic acid.

The present inventors find that since acetic acid and propionic acid in short-chain fatty acid created by good bacteria in a good intestinal environment have high volatility, acetic acid and propionic acid volatize to be contained more in defecation gas than another short-chain fatty acid. Since the present invention configured as described above detects acetic acid or propionic acid as short-chain fatty acid to analyze physical condition of a test subject, it is possible to perform detection and analysis more easily than a case of performing analysis on the basis of another short-chain fatty acid, whereby it is possible to perform detection with an inexpensive sensor. In addition, a sensor for detecting short-chain fatty acid is not always required to be sensitive only to acetic acid or propionic acid, and thus sensors, such as a sensor capable of detecting both of them, and a sensor capable of detecting acetic acid or propionic acid, and another short-chain fatty acid, are also available.

In the present invention, it is preferable that there is further provided diarrhea determination means for detecting whether a test subject has diarrhea or not, and that if the diarrhea determination means determines that the test subject has diarrhea, detection data on short-chain fatty acid, acquired in the defecation act, is not used for analysis of physical condition, or weighting of the detection data is reduced.

The present inventors find that if an intestinal environment is good, short-chain fatty acid is contained in defecation gas. However, the present inventors also find that if a test subject has diarrhea, a large amount of short-chain fatty acid is contained in defecation gas. According to the present invention configured as described above, if it is determined that a test subject has diarrhea, detection data on short-chain fatty acid, acquired in the defecation act, is not used for analysis of physical condition, or weighting of the detection data is reduced, so that it is possible to prevent a risk in which a wrong physical condition is determined to be good due to diarrhea.

In the present invention, it is preferable that the data analyzer analyzes current health condition of a test subject as well as analyzes resistance to disease of the test subject on the basis of detection data on short-chain fatty acid.

Under a condition where there are many good bacteria in intestine, the good bacteria creating short-chain fatty acid to reduce pH in the intestine, bad bacteria that deteriorate an intestinal environment tend to be difficult to survive. In this way, if bad bacteria tend to be difficult to survive in intestine, it can be said that a condition in the intestine does not easily deteriorate and allows people to be less likely to have disease as compared with a condition where a large amount of healthy-state gas is only discharged. The present invention configured as described above can analyze not only current health condition of a test subject, but also immune strength of resistance to disease of a test subject on the basis of short-chain fatty acid.

The biological information measurement system of the present invention is capable of notifying wrong physical condition in a state ahead a disease to a test subject without applying an unnecessary mental burden to a test subject while enabling physical condition to be measured on a daily basis.

### BRIEF DESCRIPTION OF THE DRAWINGS

FIG. 1 shows a state in which a biological information measurement system in accordance with a first embodiment of the present invention is attached to a flush toilet installed in a toilet installation room;
FIG. 2 is a block diagram showing a configuration of the biological information measurement system of the first embodiment of the present invention;
FIG. 3 shows a configuration of a gas detector provided in the biological information measurement system of the first embodiment of the present invention;
FIG. 4 describes a flow of measurement of physical condition by the biological information measurement system of the first embodiment of the present invention;
FIG. 5 shows an example of a screen displayed in a display device of a remote control provided in the biological information measurement system of the first embodiment of the present invention;
FIG. 6 shows an example of a table of displaying physical condition displayed in the display device of the remote control provided in the biological information measurement system of the first embodiment of the present invention;
FIG. 7A shows an example of displacement of a plotted point of updated data by correction;
FIG. 7B shows limit processing with respect to the amount of displacement of a plotted point;
FIG. 8 shows an example of a diagnosis table displayed on a server of the biological information measurement system of the first embodiment of the present invention;
FIG. 9 is a graph schematically showing a detection signal of each of sensors provided in a biological information measurement system 1 in one defecation act of a test subject;
FIG. 10A is a graph showing estimation of the amount of discharge of odiferous gas in a case where a reference value of residual gas is not fixed;
FIG. 10B is a graph showing an example of detection values acquired by a semiconductor gas sensor for measuring odiferous gas in a case where a test subject uses an alcoholic toilet seat disinfectant;
FIG. 11 shows an example of update of the diagnosis table;
FIG. 12 is a graph for describing a method of determining showing reliability of measurement;
FIG. 13 shows a correction table for noise of stink gas attached to a test subject for determining influence of stink gas attached to a body or clothes of a test subject;
FIG. 14 shows a correction table for humidity for determining influence of humidity;
FIG. 15 shows a correction table for temperature for determining influence of temperature;
FIG. 16 shows a correction table for frequency of excretory acts for determining influence of frequency of excretory acts;
FIG. 17 shows a correction table showing a relationship between reliability recorded in a data analyzer and a correction rate of measurement values;
FIG. 18 shows a correction table for environmental noise;
FIG. 19 shows a correction table for stability of a reference value;
FIG. 20 shows a correction table for cleaning of disinfecting toilet seat;
FIG. 21 shows a correction value table for a total amount of defecation gas;
FIG. 22 shows a correction value table for a fart;
FIG. 23 shows a correction value table for the amount of stool;
FIG. 24 shows a correction value table for a kind of stool;
FIG. 25 shows a correction value table for an interval of defecation;
FIG. 26 shows a correction table for the amount of accumulated data;
FIG. 27 shows a correction value table for a flow rate of air;
FIG. 28 shows a correction table for CO₂;
FIG. 29 shows a correction table for methane gas;
FIG. 30 shows a correction table for hydrogen sulfide gas;
FIG. 31 shows a relationship between a discharge time and a discharge rate of defecation gas under each of conditions S1, S2, and S3, in which a total amount of discharged gas is identical, but a discharge time as well as a discharge rate per unit time (discharge concentration) is different;
FIG. 32 shows detection waveforms of a gas sensor in a case where a discharge time as well as a discharge rate per unit time is changed;
FIG. 33 shows the amount of gas calculated on the basis of the detection waveforms of the gas sensor;
FIG. 34 shows initial portions of the detection waveforms of the gas sensor shown in FIG. 32 by being enlarged in a time axis;
FIG. 35 is a graph showing a relationship between a discharge rate per unit time (discharge concentration) and an inclination of a rising edge of each of the waveforms of detection data acquired by the sensor;
FIG. 36 shows the amount of gas estimated on the basis of the product (gas sensor waveform area) of an inclination of a detection waveform acquired by a semiconductor gas sensor, and a reaching time to a peak, for each of the conditions S1, S2, and S3, in which a discharge time as well as a discharge rate per unit time (discharge concentration) is different;
FIG. 37A shows a state in which a device on a test subject side of a biological information measurement system in accordance with another embodiment is attached to a flush toilet installed in a toilet installation room;
FIG. 37B is a perspective view showing a measuring device of the device on a test subject side shown in FIG. 37A;
FIG. 38 shows a configuration of a suction device of another embodiment of the present invention;
FIG. 39 shows a configuration of a gas detector in accordance with another embodiment of the present invention, the gas detector being configured to vary a reaching time of each of hydrogen gas and odiferous gas to the odiferous gas sensor to separate influence of the hydrogen gas;
FIG. 40 shows a detection waveform acquired by a semiconductor gas sensor of a gas detector, shown in FIG. 39;
FIG. 41 shows an example of a physical condition display table displayed in the display device of the remote control provided in a biological information measurement system of another embodiment of the present invention;
FIG. 42A shows an example of correcting a plotted point to be displayed in the display device of the biological information measurement system in accordance with another embodiment of the present invention;
FIG. 42B shows a correction table based on the amount of acetic acid gas;
FIG. 43A shows an example of correcting a plotted point to be displayed in the display device in a variation of the embodiment shown in FIG. 42;
FIG. 43B shows a correction table based on the amount of acetic acid gas;
FIG. 44 shows a result of measurement of the amount of healthy-state gas and odiferous gas contained in defecation gas acquired from each of healthy people less than sixties, healthy people in sixties to seventies, patients having early cancer, and patients having advanced cancer;
FIG. 45 shows the amount of hydrogen sulfide gas contained in defecation gas, compared between healthy people and patients having colorectal cancer;
FIG. 46 shows the amount of methyl mercaptan gas contained in defecation gas, compared between healthy people and patients having colorectal cancer;
FIG. 47 shows the amount of hydrogen gas contained in defecation gas, compared between healthy people and patients having colorectal cancer;
FIG. 48 shows the amount of carbon dioxide gas contained in defecation gas, compared between healthy people and patients having colorectal cancer;
FIG. 49 shows the amount of propionic acid gas contained in defecation gas, compared between healthy people and patients having colorectal cancer;
FIG. 50 shows the amount of acetic acid gas contained in defecation gas, compared between healthy people and patients having colorectal cancer; and
FIG. 51 shows the amount of butyric acid gas contained in defecation gas, compared between healthy people and patients having colorectal cancer.

### DETAILED DESCRIPTION OF THE PREFERRED EMBODIMENTS

One embodiment of a biological information measurement system of the present invention will be described in detail below with reference to drawings.

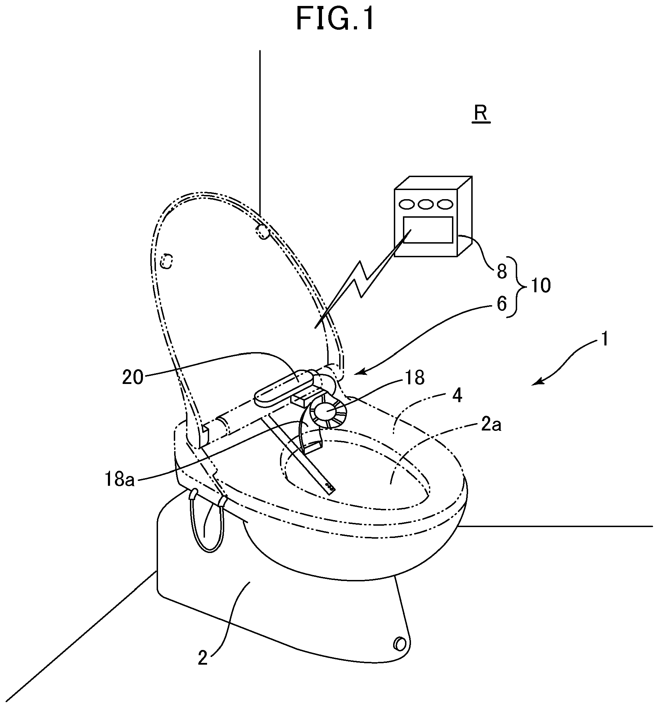

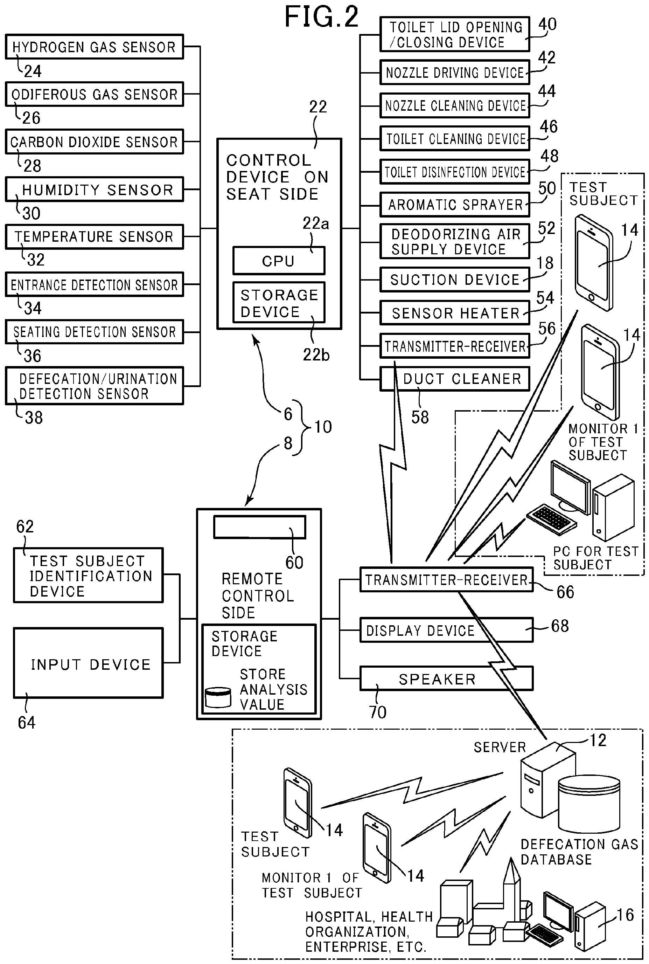

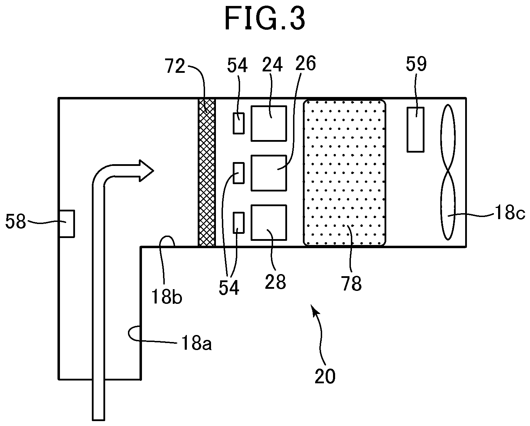

FIG. 1 shows a state in which a biological information measurement system in accordance with a first embodiment of the present invention is attached to a flush toilet installed in a toilet installation room. FIG. 2 is a block diagram showing a configuration of the biological information measurement system of the present embodiment. FIG. 3 shows a configuration of gas detector provided in the biological information measurement system of the present embodiment.

As shown in FIG. 1, the biological information measurement system 1 includes a measuring device 6 assembled inside a seat 4 mounted on a flush toilet 2 installed in a toilet installation room R, and a device 10 on a test subject side composed of a remote control 8 attached to a wall surface of the toilet installation room R. In addition, as shown in FIG. 2, the biological information measurement system 1 includes a server 12, a terminal 14 for a test subject, formed by installing dedicated software in a smartphone, and the like, and a medical facility terminal 16 installed in medical facilities, such as a hospital, to exchange data with the device 10 on a test subject side to serve as a part of the biological information measurement system 1. Further, measurement data transmitted from a large number of devices 10 on a test subject side is accumulated in the server 12 and the medical facility terminal 16, and then data analysis is performed.

The biological information measurement system 1 of the present embodiment analyzes physical condition including determination of cancer on the basis of odiferous gas containing a sulfur component, particularly a methyl mercaptan (CH₃SH) gas, in defecation gas discharged from a test subject during defecation. In addition, the biological information measurement system 1 of the present embodiment measures also healthy-state gas along with odiferous gas to improve analysis accuracy of physical condition on the basis of a correlation between the gases. The healthy-state gas originates from intestinal fermentation, and increases as an intestinal health degree increases. The healthy-state gas is specifically carbon dioxide, hydrogen, methane, short-chain fatty acid, and the like. In the present embodiment, a carbon dioxide gas and hydrogen gas, which are easy to be measured and are large in amount to enable reliability of measurement of a health index to be maintained at a high level, are measured as healthy-state gas. Each of the devices 10 on a test subject side is configured to display an analysis result during defecation of a test subject or immediately after the defecation. In contrast, the server 12 collects measurement results of a large number of test subjects to enable more detailed analysis by comparison with another test subject, and the like. In this way, in the biological information measurement system 1 of the present embodiment, the device 10 on a test subject side installed in the bathroom R performs a simple analysis, and the server 12 preforms a more detailed analysis.

Here, a measurement principle of physical condition in the biological information measurement system 1 of the present embodiment will be described. Documents and the like report that if people have cancer of digestive system, particularly colorectal cancer, odiferous gas containing a sulfur component, such as methyl mercaptan or hydrogen sulfide, are discharged from an affected portion simultaneously with defecation. The digestive system includes the esophagus, stomach, duodenum, small intestine, large intestine, liver, the pancreas, and gallbladder. Although the large intestine also can be classified into the appendix, caecum, rectal, and colon, hereinafter the four portions are collectively called the large intestine. Cancer changes little on a daily basis, and gradually develops. If the cancer develops, the amount of odiferous gas containing a sulfur component, particularly methyl mercaptan, increases. That is, if the amount of odiferous gas containing a sulfur component increases, it can be determined that the cancer develops. In recent years, a concept of "ahead-disease" has spread, so that there is spread a concept of preventing a disease by improving physical condition at the time when the physical condition is deteriorated before falling sick. Thus, it is required to detect cancer, particularly progressive cancer, such as colorectal cancer, before having cancer, to improve physical condition.

Here, defecation gas discharged during defecation includes nitrogen, oxygen, argon, water vapor, carbon dioxide, hydrogen, methane, acetic acid, trimethylamine, ammonia, propionic acid, methyl disulfide, methyl trisulfide, and the like, along with hydrogen sulfide and methyl mercaptan. Among them, it is required to measure odiferous gas containing a sulfur-based component, particularly methyl mercaptan to determine disease of cancer. Each of the propionic acid, methyl disulfide, and methyl trisulfide, contained in defecation gas, is a very trace amount as compared with the methyl mercaptan, so that each of them does not matter to analysis of physical condition, such as determination of cancer, whereby it is possible to ignore them. However, it cannot be said that each of other gas components is a negligible trace amount. In order to accurately determine cancer, it is generally thought to use a sensor capable of detecting only odiferous gas containing a sulfur component. Unfortunately, the sensor for detecting only odiferous gas containing a sulfur component is large in size and very expensive, so that it is difficult to be configured as an apparatus for household use.

In contrast, the present inventors have diligently studied to reach an idea that a gas sensor that detects not only methyl mercaptan in defecation gas, but also odiferous gas including another odiferous gas, is used to enable an apparatus for household use to be configured at low cost. Specifically, the present inventors determine to use a general semiconductor gas sensor or a solid electrolyte sensor, sensitive not only to a sulfur-containing gas containing a sulfur component, but also to another odiferous gas, as a sensor for detecting gas.

If a risk of cancer increases, a very strong odiferous gas containing a sulfur component, such as methyl mercaptan gas, increases in amount. Then, a sensor, such as a semiconductor gas sensor, and a solid electrolyte sensor, widely sensitive to odiferous gas, is capable of always detecting increase of this kind of gas. Unfortunately, as described later, a sensor, such as a semiconductor gas sensor, and a solid electrolyte sensor, widely sensitive to an odiferous gas, detects also another odiferous gas, such as hydrogen sulfide, methyl mercaptan, acetic acid, trimethylamine, or ammonia, which increases when people have wrong physical condition caused by a bad living habit. However, cancer is a disease developing for a long time, or a few years, so that a state of having an increased very strong odiferous gas containing a sulfur component, such as methyl mercaptan gas or hydrogen sulfide, continues for a long time if people have cancer. Thus, even if a general semiconductor gas sensor, or a solid electrolyte sensor, widely sensitive not only to sulfur-containing gas containing a sulfur component, but also to another odiferous gas, is used, it is possible to determine that there is a high possibility of disease of cancer to cause a risk of cancer to increase if the amount of gas is high for a long time.

In addition, a semiconductor sensor and a solid electrolyte sensor, using an oxidation-reduction reaction, detect not only methyl mercaptan gas, but also odiferous gas, such as acetic acid, trimethylamine, or ammonia, in defecation gas. However, the present inventors have discovered from experimental results that a mixed amount of odiferous gas, such as hydrogen sulfide, methyl mercaptan, acetic acid, trimethylamine, or ammonia, tends to increase if a bad living habit causes physical condition to be deteriorated, and tends to decrease if physical condition is good. Specifically, healthy people have a small total amount of methyl mercaptan gas and odiferous gas other than the methyl mercaptan gas. In contrast, a total amount of methyl mercaptan gas and odiferous gas other than the methyl mercaptan gas temporarily increases due to deterioration of intestinal environment caused by excessive obstipation, a kind of meal, lack of sleep, crapulence, excessive drinking, excessive stress, and the like.

Acetic acid in defecation gas tends to increase not only when physical condition is deteriorated due to diarrhea, and the like, but also when physical condition is good. That is, this tendency does not always agree with tendency of the amount of methyl mercaptan and another odiferous gas with change in physical condition described above. However, the amount of acetic acid contained in defecation gas is very small as compared with methyl mercaptan. Thus, even if the amount of acetic acid increases when physical condition is good, the amount of the increase is very small as compared with decrease in the amount of another odiferous gas. In addition, the amount of increase of acetic acid when physical condition is deteriorated due to diarrhea, and the like, is very large as compared with the amount of increase thereof when physical condition is good. Accordingly, the amount of odiferous gas contained in defecation gas tends to increase as a whole if physical condition is deteriorated due to a bad living habit, and tends to decrease if physical condition is good. Then, deterioration of intestinal environment due to this kind of bad living habit results in having cancer, so that the amount of odiferous gas contained in defecation gas is a suitable index to improve physical condition when people are still in a state before having cancer.

In the invention physical condition is analyzed on the basis of detection data acquired by a semiconductor sensor, or solid electrolyte sensor, sensitive not only to methyl mercaptan gas, but also to odiferous gas other than the methyl mercaptan gas, such as hydrogen sulfide, acetic acid, trimethylamine, ammonia, in defecation gas. Accordingly, it is possible to acquire an analysis result to which a result of a wrong physical condition and a bad living habit is reflected, and the analysis result is available as an index based on objective data for improving physical condition and a living habit that may increase a risk of cancer.

In addition, defecation gas contains not only odiferous gas, but also H₂ and methane, so that if a semiconductor gas sensor, or a solid electrolyte sensor, is used for a gas sensor, the gas sensor also reacts to H₂ and methane. Further, if a measuring device using a semiconductor gas sensor, or a solid electrolyte sensor, is set at each home, the sensor may react to an aromatic and a perfume.

In contrast, the present inventors, as described later in detail, achieve a method of removing influence of hydrogen and methane from detection data of a semiconductor gas sensor, or a solid electrolyte sensor, by using a hydrogen sensor, a methane sensor, and a column, and a method of removing influence of an aromatic and a perfume as noise by detecting defecation act. Accordingly, influence of hydrogen and methane, as well as influence of an aromatic and a perfume, is removed from data detected by the semiconductor gas sensor, or the solid electrolyte sensor, to enable the amount of only odiferous gas in defecation gas to be estimated.

The amount of methyl mercaptan and another odiferous gas contained in defecation gas is very small as compared with and methane. Accordingly, even if a semiconductor gas sensor, or a solid electrolyte sensor, is used, the amount of the mixed odiferous gas may not be accurately measured.

In contrast, the present inventors have paid attention to that healthy people have acidic intestinal environment, and that cancer patients have intestinal environment in which odiferous gas containing a sulfur component occurs to increase in amount, so that the intestinal environment becomes alkaline to reduce bifidobacteria, and the like, in amount, whereby the amount of healthy-state gas of ferment-base components, such as CO₂, H₂, or fatty acid, reliably and continuously decreases inversely with increase of the amount of odiferous gas.

Accordingly, the inventors have thought that even if measurement accuracy at each measurement is not always high, monitoring a correlation between the amount of odiferous gas, such as methyl mercaptan and the amount of healthy-state gas components, such as CO₂, or H₂ during defecation every day may enable occurrence of advanced cancer to be detected.

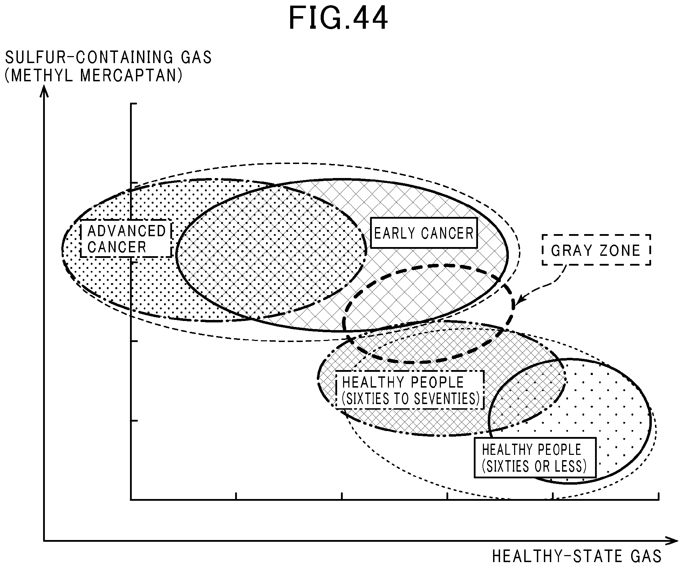

Then, the present inventors have measured the amount of healthy-state gas and odiferous gas contained in defecation gas acquired from each of healthy people less than sixties, healthy people in sixties to seventies, patients having early cancer, and patients having advanced cancer, and then a result shown in FIG. 44 has been acquired. That is, healthy people have defecation gas in which the amount of healthy-state gas is large, and the amount of odiferous gas is small. In contrast, cancer patients have defecation gas in which the amount of healthy-state gas is small, and the amount of odiferous gas is large. The amount of healthy-state gas contained in defecation gas in advanced cancer is less than that in early cancer. In addition, if the amount of healthy-state gas and the amount of odiferous gas is an intermediate amount between that of cancer patients and that of healthy people, the amount is within a gray zone, that is, it is thought that the gray zone is a state before having disease. Accordingly, the present inventors have thought on the basis of knowledge described above that if the amount of healthy-state gas of a test subject and the amount of odiferous gas, are measured, it is possible to improve determination accuracy of health condition on the basis of a correlation between the amounts.

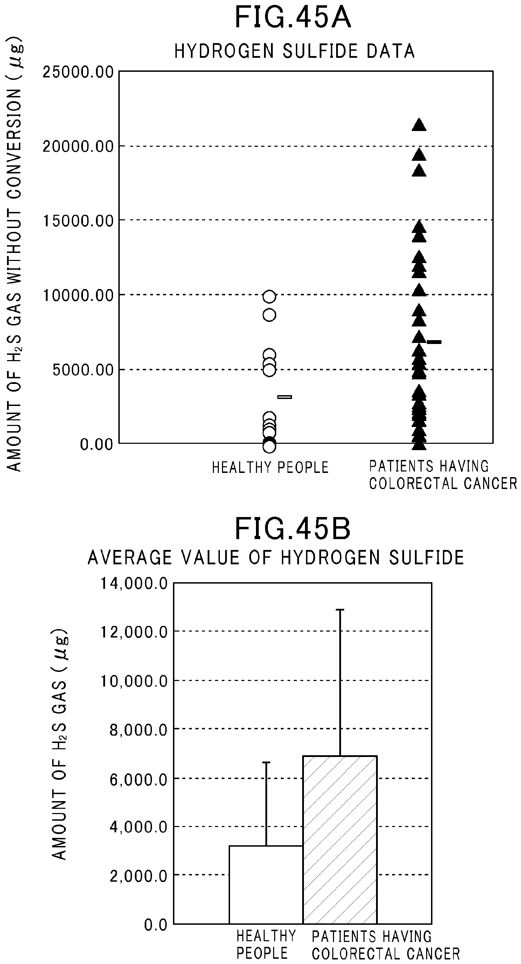

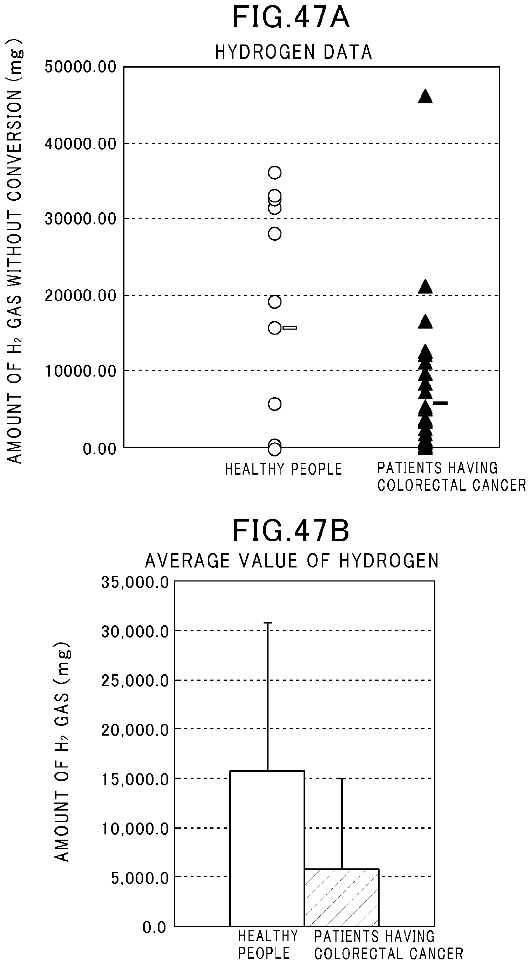

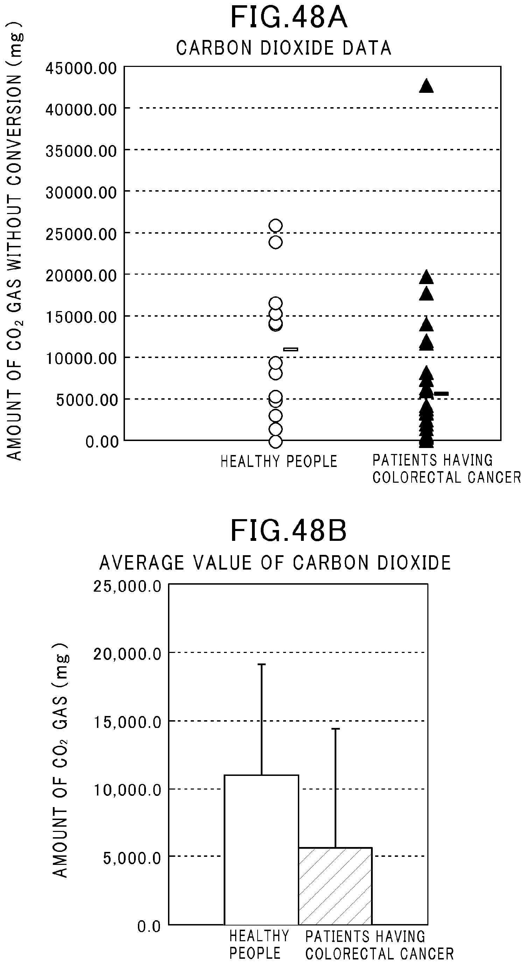

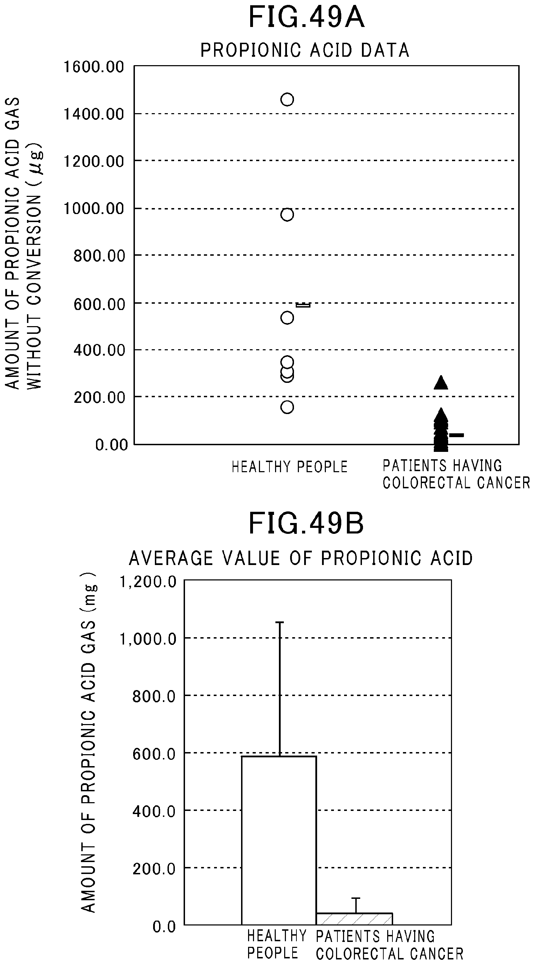

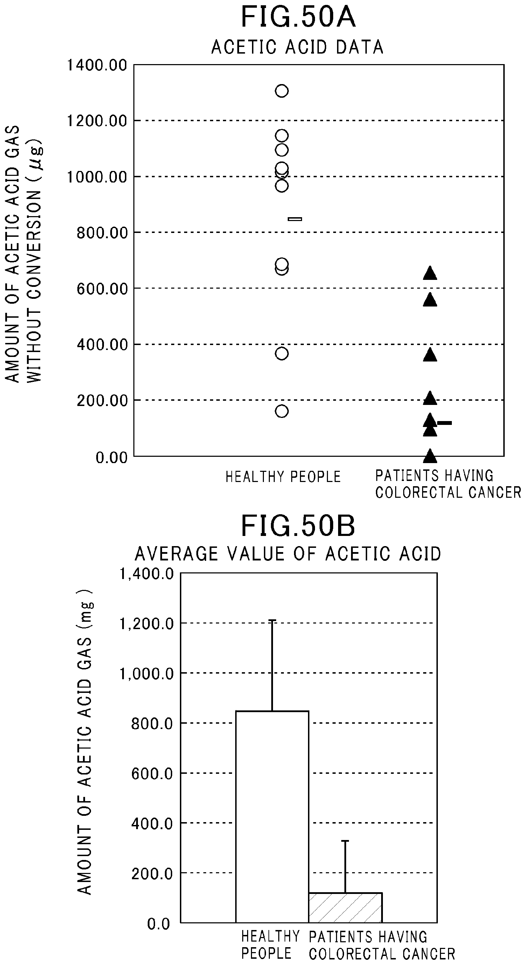

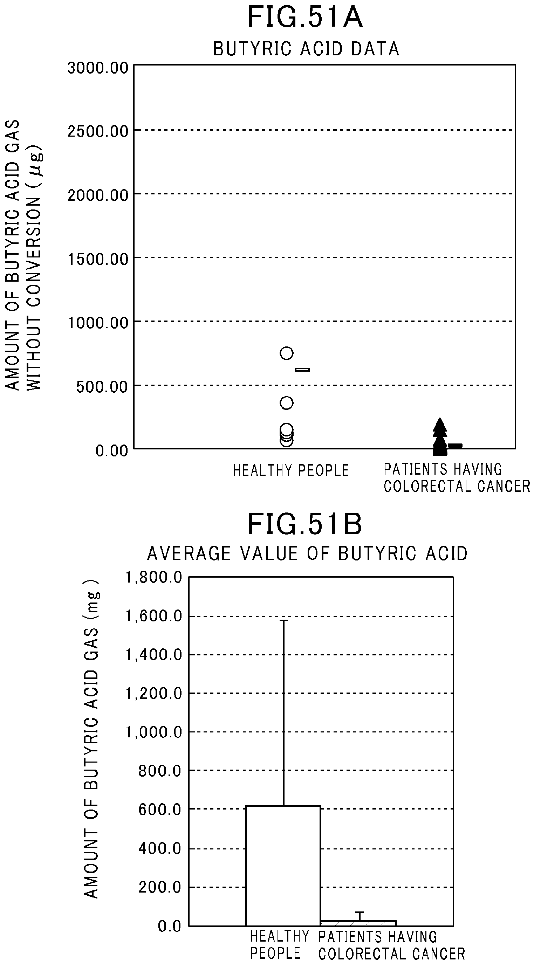

In addition, FIGS. 45 to 51 show measurement data on the amount of various kinds of gas contained in defecation gas, in which healthy people and colorectal cancer patients (including advanced cancer, and early cancer) are compared.

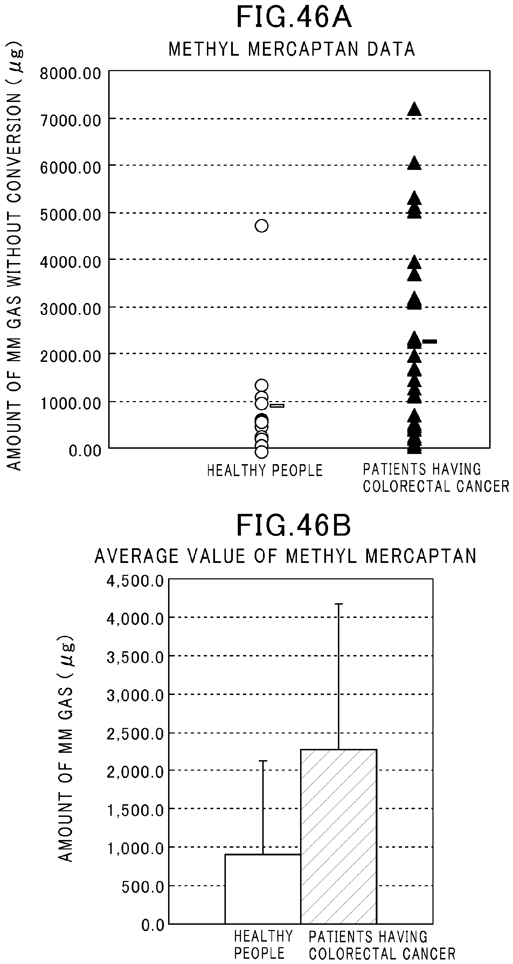

FIG. 45 shows the amount of hydrogen sulfide contained in defecation gas, in which healthy people and colorectal cancer patients are compared, and FIGS. 46 to 51 show the amount of methyl mercaptan gas, hydrogen gas, carbon dioxide gas, propionic acid gas, acetic acid gas, and butyric acid gas, respectively, in each of which healthy people and colorectal cancer patients are compared. In each of FIGS. 46 to 51, a portion (a) shows measurement data on the amount of each gas by plotting healthy people with a circular mark, and colorectal cancer patients with a triangular mark. In addition, each of portions (b) shows an average value of each measurement data with a bar graph, and standard deviation of each of the measurement data with a line segment.

As is evident from the measurement data shown in FIGS. 45 to 51, although the amount of various kinds of gas contained in defecation gas greatly varies in both healthy people and colorectal cancer patients, with respect to hydrogen sulfide gas and methyl mercaptan gas of odiferous gas, data indicating a large amount of gas is shown many times in the colorectal cancer patients, but there is little data indicating a large amount of gas in the healthy people. Meanwhile, with respect to hydrogen gas, and carbon dioxide gas, there is data indicating a large amount of gas in the healthy people, and there is little data indicating a large amount of gas in the colorectal cancer patients. In this way, while the amount of odiferous gas contained in defecation gas, indicating a risk of colorectal cancer, is large in the colorectal cancer patients, and small in the healthy people, the amount of hydrogen gas and carbon dioxide gas of healthy-state gas is large in the healthy people, and small in the colorectal cancer patient. Accordingly, magnitude relation between the amount of odiferous gas and the amount of healthy-state gas is reversed between the healthy people and the colorectal cancer patient. Although it is difficult to sufficiently measure physical condition of a test subject by using the measurement data acquired by one measurement of the amount of odiferous gas and healthy-state gas, the measurement data shows that if relation between odiferous gas and healthy-state gas is continuously measured multiple times for a predetermined period, it is possible to reliably measure physical condition of a test subject.

When measured defecation gas, the present inventors found that the amount of defecation gas discharged with the first excretory act was large, and a large amount of odiferous gas was also contained in a case where an excretory act was performed multiple times during one defecation (action of discharging a fart once or a stool once). Thus, in the present embodiment, health condition of a test subject is analyzed on the basis of defecation gas acquired first to accurately measure odiferous gas in trace amount. Accordingly, although measurement may be affected by a stool and a fart discharged by the first excretory act when the amount of gas discharged during the second excretory act or later is measured, this influence can be reduced.

The biological information measurement system 1 of the present embodiment is formed on the basis of the measurement principle described above. In the description below, odiferous gas includes methyl mercaptan gas of odiferous gas containing a sulfur component, and odiferous gas, such as hydrogen sulfide other than the methyl mercaptan, methyl mercaptan, acetic acid, trimethylamine, or ammonia.

Next, a specific configuration of the biological information measurement system 1 of the present embodiment will be described in detail.

As shown in FIG. 1, the device 10 on a test subject side in the biological information measurement system 1 is attached to the flush toilet 2 in the toilet installation room R, and a part thereof is assembled into a seat 4 with a function of cleaning anus. The seat 4 with a function of cleaning anus is provided with a suction device 18 that sucks gas in a bowl 2a of the flush toilet 2, as the measuring device 6, and a gas detector 20 that detects a specific component of the gas sucked. The suction device 18 shares a part of a function with a deodorizing device that is usually assembled in the seat 4 with a function of cleaning anus. Gas sucked by the suction device 18 is deodorized by the deodorizing device, and then is returned into the bowl 2a. Each of devices assembled in the seat 4, such as the suction device 18, and the gas detector 20, is controlled by a built-in control device 22 provided on a seat side (refer to FIG. 2).

As shown in FIG. 2, the device 10 on a test subject side is composed of the measuring device 6 assembled in the seat 4, and a data analyzer 60 built in the remote control 8.

The measuring device 6 includes a CPU 22a, and the control device 22 provided with a storage device 22b. The control device 22 is connected to a hydrogen gas sensor 24, an odiferous gas sensor 26, a carbon dioxide sensor 28, a humidity sensor 30, a temperature sensor 32, an entrance detection sensor 34, a seating detection sensor 36, a defecation/urination detection sensor 38, a toilet lid opening/closing device 40, a nozzle driving device 42, a nozzle cleaning device 44, a toilet cleaning device 46, a toilet disinfection device 48, an aromatic sprayer 50 of an aromatic injection device, a deodorizing air supply device 52, the suction device 18, a sensor heater 54, a transmitter-receiver 56, and a duct cleaner 58. As described later, the hydrogen gas sensor and the odiferous gas sensor may be formed into an integrated sensor.

The temperature sensor 32 measures temperature of a detecting portion of the odiferous gas sensor 26, and the like. The humidity sensor 30 measures humidity of gas sucked from the inside of the bowl 2a. Sensitivity of these sensors slightly varies depending on temperature of the detecting portion. Likewise, humidity change due to urination, and the like, affects sensitivity of the sensors. In the present embodiment, the amount of odiferous gas is very small in amount, so that the CPU 22a on a toilet side controls the sensor heater 54 described later, and a humidity adjuster 59 (refer to FIG. 3) to allow sensor temperature and suction humidity of the sensors 30 and 32 to be accurately maintained within a predetermined range, depending on temperature and humidity measured by the sensors 30 and 32, respectively. As a result, the sensor temperature and the suction humidity are adjusted to a predetermined temperature and humidity environment to enable gas in trace amount to be accurately and steady measured. These sensors and devices are not always required, and it is desirable to provide them to improve accuracy.

The entrance detection sensor 34 is an infrared ray sensor, for example, and detects entrance and leaving of a test subject into and from the toilet installation room R.

The seating detection sensor 36 is an infrared ray sensor, a pressure sensor, or the like, for example, and detects whether a test subject sits on the seat 4 or not.

In the present embodiment, the defecation/urination detection sensor 38 is composed of a microwave sensor, and is configured to detect a state of defecation, such as whether a test subject has discharged urine or a stool, whether a stool floats or sinks in seal water, and whether a stool is a diarrhea state or not. Alternatively, the defecation/urination detection sensor 38 may be composed of a CCD, and a water level sensor that measures transition of seal water.

The toilet lid opening/closing device 40 is provided to open and close a toilet lid on the basis of a detection signal of the entrance detection sensor 34, and the like, and according to a situation.

The nozzle driving device 42 is used to clean anus, and cleans anus of a test subject after defecation. The nozzle driving device 42 is configured to drive a nozzle to clean the flush toilet 2.

The nozzle cleaning device 44 cleans a nozzle of the nozzle driving device 42, and in the present embodiment, is configured to create hypochlorous acid from tap water to clean the nozzle with the hypochlorous acid created.

The toilet cleaning device 46 discharges water or tap water stored in a cleaning water tank (not shown) into a toilet to clean the inside of the bowl 2a of the flush toilet 2. Although the toilet cleaning device 46 is usually operated by a test subject while operating the remote control 8 to clean the inside of the bowl 2a, as described later, it is automatically operated by the control device 22 according to a situation.

The toilet disinfection device 48, for example, creates disinfecting water, such as hypochlorous acid water, from tap water, and sprays the disinfecting water created onto the bowl 2a of the flush toilet 2 to disinfect the bowl 2a.

The aromatic sprayer 50 sprays a predetermined aromatic into the toilet installation room R to prevent a test subject from spraying an arbitrary aromatic into the toilet installation room R to prevent an odor component that may be a disturbance with respect to measurement from being sprayed. Providing the aromatic sprayer 50 enables the predetermined aromatic in predetermined amount that does not affect measurement to be sprayed in a predetermined period according to a situation, and then the biological information measurement system 1 is able to recognize that the aromatic is sprayed. Accordingly, a disturbance with respect to measurement of physical condition is reduced to stabilize analysis results, so that the aromatic sprayer 50 serves as output result stabilizing means.

The suction device 18 is provided with a fan for sucking gas in the bowl 2a of the flush toilet 2, and the sucked gas is deodorized by a deodorant filter after flowing through a detecting portion of the odiferous gas sensor 26, and the like. Details of a configuration of the suction device 18 will be described later.

The deodorizing air supply device 52 discharges air that is deodorized after being sucked by suction device 18 into the bowl 2a.

The sensor heater 54 is provided to apply thermal activation to a detecting portion of the odiferous gas sensor 26, and the like. Maintaining a detecting portion at a predetermined temperature enables each sensor to accurately detect a predetermined gas component.

The duct cleaner 58 is provided to clean the inside of a duct 18a attached to the suction device 18 with hypochlorous acid acquired by electrolysis of tap water, or the like, for example.

In the present embodiment shown in FIG. 1, the suction device 18, the deodorizing air supply device 52, and the duct cleaner 58, are integrally formed into the deodorizing device. That is, the suction device 18 sucks gas in the bowl 2a into the duct 18a so that a deodorant filter 78 (refer to FIG. 3) applies deodorizing processing to the sucked gas, and then the gas to which the deodorizing processing is applied is discharged into the bowl 2a again. As a result, it is prevented that gas, to which the odiferous gas sensor 26 is sensitive, flows into the bowl 2a from the outside to change gas components in the bowl 2a during defecation of a test subject by a factor other than defecation gas discharged by the test subject. Thus, the deodorizing device provided with the deodorant filter 78, and the deodorizing air supply device 52, serve as output result stabilizing means. Alternatively, as a variation, the present invention may be configured to provide a gas supply device for measurement (not shown) that allows gas that is insensitive to each gas sensor to flow into the bowl 2a so as to allow gas for measurement with the same amount of gas sucked by the suction device 18 to flow into the bowl 2a. In this case, the gas supply device for measurement (not shown) serves as output result stabilizing means for stabilizing analysis results.

Next, as shown in FIG. 2, the remote control 8 is provided with the built-in data analyzer 60 to which a test subject identification device 62, an input device 64, a transmitter-receiver 66, a display device 68, and a speaker 70, are connected. In the present embodiment, the transmitter-receiver 66, the display device 68, and the speaker 70, serve as an output device that outputs analysis results by the data analyzer 60. The data analyzer 60 is composed of a CPU, a storage device, a program for operating the CPU and the storage device, and the like, and the storage device is provided with a database.

In the present embodiment, the input device 64 and the display device 68 are configured as a touch panel to accept various kinds of input, such as identification information on a test subject, including a name of the test subject, and the like, as well as to display a variety of information items, such as measurement results of physical condition.

The speaker 70 is configured to output various kinds of alarm, message, and the like, issued by the biological information measurement system 1.

In the test subject identification device 62, identification information on a test subject, including a name of the test subject, and the like, is previously registered. When a test subject uses the biological information measurement system 1, names of registered test subjects are displayed in the touch panel, and then the test subject selects his or her own name.

The transmitter-receiver 66 on a remote control 8 side is communicatively connected to the server 12 through a network. The terminal 14 for a test subject is composed of a device capable of displaying data received by a smartphone, a tablet PC, a PC, or the like, for example.

The server 12 includes a defecation gas database. The defecation gas database records measurement data including the amount of odiferous gas and healthy-state gas in each excretory act, and reliability data, along with a measurement date and time, by being associated with identification information on each test subject using the biological information measurement system 1. The server 12 also stores a diagnosis table, and includes a data analysis circuit.

In addition, the server 12 is connected to the medical facility terminal 16 installed in a hospital, a health organization, and the like, through a network. The medical facility terminal 16 is composed of a PC, for example, to enable data recorded in the database of the server 12 to be browsed.

Subsequently, with reference to FIG. 3, a configuration of the gas detector 20 built in the seat 4 will be described.

First, in the biological information measurement system 1 of the present embodiment, a semiconductor gas sensor is used in the gas detector 20 as a gas sensor to detect odiferous gas and hydrogen gas. In addition, a solid electrolyte type sensor is used in the gas detector 20 to detect carbon dioxide.

The semiconductor gas sensor includes a detecting portion composed of a metal oxide film containing tin oxide, and the like. If the detecting portion is exposed to reducing gas while being heated at a few hundreds degrees, oxidation-reduction reaction occurs between oxygen adsorbed in a surface of the detecting portion and the reducing gas. The semiconductor gas sensor electrically detects change in resistance of the detecting portion by the oxidation-reduction reaction to enable reducing gas to be detected. Reducing gas that a semiconductor gas sensor can detect includes hydrogen gas, and odiferous gas. In the present embodiment, although a semiconductor gas sensor is used for both a sensor for detecting odiferous gas, and a sensor for detecting hydrogen gas, material of each of detecting portions of the respective sensors is adjusted so that a detecting portion used in the odiferous gas sensor reacts strongly to odiferous gas, and a detecting portion used in the hydrogen gas sensor reacts strongly to hydrogen gas.

In this way, although the present embodiment uses a "semiconductor gas sensor" as an "odiferous gas sensor", as described above, the "semiconductor gas sensor" is a general type that is sensitive not only to methyl mercaptan gas of a detection object, but also widely to odiferous gas other than that. In addition, as described later, although a solid electrolyte sensor is available for an "odiferous gas sensor", as with a semiconductor gas sensor, a general type of a solid electrolyte sensor, sensitive to methyl mercaptan gas as well as widely to another odiferous gas other than the methyl mercaptan, may be used. That is, it is very difficult to manufacture a gas sensor that is sensitive only to methyl mercaptan gas, and even if the gas sensor can be manufactured, the gas sensor becomes very large in size and expensive. If this kind of large and expensive gas sensor is used, the gas sensor is feasible for a medical device used in advanced clinical examination, but it is impossible to manufacture a biological information measurement system at a cost enabling the system to be sold as a consumer product. The biological information measurement system of the present embodiment uses a simple and general gas sensor that is sensitive also to another odiferous gas other than methyl mercaptan gas of a detection object, as the "odiferous gas sensor", to be feasible as a consumer product. As described above, although the gas sensor used in the present embodiment is sensitive to methyl mercaptan gas, as well as to odiferous gas other than the methyl mercaptan gas, the gas sensor is referred to as an "odiferous gas sensor" in the present specification, for convenience. The "odiferous gas sensor" used in the present embodiment is sensitive to odiferous gas that representatively includes methyl mercaptan gas, hydrogen sulfide gas, ammonia gas, and alcoholic gas.

Although the "odiferous gas sensor" used in the biological information measurement system 1 of the present embodiment is sensitive to methyl mercaptan gas of an object, as well as to odiferous gas other than that, a variety of devices described later enable even this kind of gas sensor to be used for measurement with necessary and sufficient accuracy as a consumer product. Specifically, the devices include a device to improve a measurement environment in a space of a toilet installation room where a variety of odiferous gases exist, a device for data processing of extracting data on defecation gas by assuming defecation act of a test subject from a detection signal provided by a gas sensor, a device to prevent an excessive mental burden from being applied to a test subject even if detection data with a large error is acquired, and the like. Each of the devices will be described later in detail.

Although the present embodiment describes a case where a semiconductor gas sensor is used for a sensor for detecting odiferous gas and hydrogen gas, a solid electrolyte sensor is also available instead of the semiconductor gas sensor. The solid electrolyte sensor, for example, detects gas on the basis of the amount of ions that penetrates its solid electrolyte, such as stabilized zirconia, while the solid electrolyte is heated. Gas which can be detected by the solid electrolyte sensor includes hydrogen gas, and odiferous gas. In the present embodiment, a solid electrolyte sensor is used as a sensor for detecting carbon dioxide. A carbon dioxide sensor is not limited to the sensor above, and an infrared sensor or the like may be available. The sensor for detecting carbon dioxide may be eliminated.

As shown in FIG. 3, in the present embodiment, the gas detector 20 is arranged inside the suction device 18.

The suction device 18 includes the duct 18a directed downward, an air intake passage 18b directed substantially in a horizontal direction, and a suction fan 18c arranged downstream of the air intake passage 18b. In the duct 18a, the duct cleaner 58, and the humidity adjuster 59, are provided.

The gas detector 20 includes a filter 72 arranged inside the air intake passage 18b, the odiferous gas sensor 26, the hydrogen gas sensor 24, and the carbon dioxide sensor 28. As shown in FIG. 3, the filter 72 is arranged so as to traverse the air intake passage 18b, and the odiferous gas sensor 26, the hydrogen gas sensor 24, and the carbon dioxide sensor 28, are juxtaposed downstream of the filter 72.

In addition, the deodorant filter 78 is provided downstream of the odiferous gas sensor 26, so that the suction device 18 also serves as a deodorizing device by allowing the deodorant filter 78 to deodorize sucked gas.

Further, the humidity adjuster 59 is provided downstream of the deodorant filter 78. The humidity adjuster 59 is filled with a desiccant, and if it is required to reduce humidity in the bowl 2a, moisture is removed from air circulating in the bowl 2a by switching a flow channel so that the air passing through the deodorant filter 78 passes through the filled desiccant. Accordingly, the humidity in the bowl 2a is maintained at a proper value to maintain detection sensitivity of each gas sensor at an almost constant level. Thus, the humidity adjuster 59 serves as output result stabilizing means for preventing humidity change in the bowl 2a.

The suction fan 18c sucks stink gas containing odiferous gas, and the like, in the bowl 2a of the flush toilet 2, at a constant speed to deodorize the stink gas, and then returns the gas into the bowl 2a. The duct 18a for deodorization opens in the bowl 2a while its suction port is directed downward to prevent a splash of urine or the like from entering the inside of the duct 18a. Molecular weight of odiferous gas, such as methyl mercaptan, and of hydrogen gas, is small enough to allow the gases to rise immediately after defecation. In contrast, in the present embodiment, odiferous gas and hydrogen gas discharged is sucked by suction fan 18c through an inlet of the duct 18a, opening in the bowl 2a, so that it is possible to reliably guide the gases into the gas detector 20. In this way, the suction device 18 is operated before a test subject starts defecation, and brings gas at a constant flow velocity into contact with each gas sensor during defecation of the test subject. Accordingly, it is possible to acquire a steady measurement value. Thus, the suction device 18, and the control device 22 that allows the suction device 18 to operate, serve as output result stabilizing means.

The filter 72 does not have a deodorizing function, and is configured so as to allow odiferous gas, hydrogen, and carbon dioxide to pass therethrough, as well as to prevent foreign material, such as urine, and a cleaner from passing therethrough. For this kind of filter 72, a member for mechanically collecting the foreign material without using chemical reaction, such as a fine net-like member, is available. Accordingly, it is possible to prevent the odiferous gas sensor 26, the hydrogen gas sensor 24, and the carbon dioxide sensor 28, from being contaminated by a urinary calculus, or the like.

The sensor heater 54 is provided upstream of each gas sensor, and downstream of the filter 72. As described above, the odiferous gas sensor 26 and the hydrogen gas sensor 24, each of which is a semiconductor gas sensor, are capable of detecting hydrogen and odiferous gases while each of their detecting portions is heated to a predetermined temperature. The sensor heater 54 is provided to heat the detecting portions of the odiferous gas sensor 26 and the hydrogen gas sensor 24. The carbon dioxide sensor 28 is also required to heat its solid electrolyte to a predetermined temperature, so that the sensor heater 54 is provided. The sensor heater 54 also serves as a stink removing device for thermally removing stink gas components attached to each of the sensors. Even if a solid electrolyte sensor is used as the odiferous gas sensor, and the hydrogen gas sensor, it is required to provide a sensor heater for heating a detecting portion.

The sensor heater 54 also serves as means for removing a deposit attached to each sensor. Although foreign material is removed from gas passing through the filter 72, the sucked gas contains various stink gas components. Such stink gas components are attached to each gas sensor, and may cause noise when odiferous gas in trace amount is measured. In contrast, the sensor heater 54 heats a detecting portion of a sensor to enable stink gas attached to the sensor to be thermally removed without providing an additional device. The control device 22 controls the sensor heater 54 before a test subject starts defecation act so as to allow temperature of each gas sensor to be constant. That is, the control device 22 controls the sensor heater 54 so as to prevent temperature of each gas sensor from decreasing due to contact of an air flow. Accordingly, it is possible to maintain sensitivity of each gas sensor at a predetermined value during defecation of a test subject to enable a measurement error of each gas sensor to be reduced. Thus, the control device 22 and the sensor heater 54 serve as output result stabilizing means for stabilizing analysis results to be outputted.

The deodorant filter 78 is a catalytic filter that adsorbs stink gas, such as odiferous gas. The deodorant filter 78 removes gas, such as odiferous gas, from air, and the air is returned to the bowl 2a. Then, if odiferous gas or the like is contained in the gas returned into the bowl 2a, the odiferous gas or the like flows into the bowl 2a may be sucked through the duct 18a again to be detected by the odiferous gas sensor 26 again. Thus, in the present embodiment, the deodorant filter 78 is arranged downstream of the odiferous gas sensor 26 to reliably remove odor components, such as odiferous gas, from gas returned into the bowl 2a.

If a test subject sits on the seat 4, a portion above the bowl 2a is closed by his or her underwear, or the like. If the inside of the bowl 2a is placed under negative pressure, stink gas components attached to a body, clothes, and the like, of the test subject, may be sucked into the bowl 2a. In the biological information measurement system 1 of the present embodiment, sensitivity of the odiferous gas sensor 26 is set very high to detect only a trace amount of odiferous gas contained in defecation gas, so that even stink gas components attached to a body, clothes, and the like, of a test subject, may be a disturbance with respect to measurement. In contrast, in the present embodiment, gas after deodorized is returned into the bowl 2a, so that the inside of the bowl 2a is not placed under negative pressure to enable gas components attached to a body, clothes, and the like, of a test subject, to be prevented from being sucked into the bowl 2a.

Here, the semiconductor gas sensor used as the odiferous gas sensor 26 detects not only odiferous gas but also hydrogen. Thus, it is required to separate influence of hydrogen gas from detection data acquired by the semiconductor gas sensor. In the present embodiment, as a hydrogen separation mechanism for separating this kind of influence of hydrogen gas, in the gas detector 20, a detection value of hydrogen gas detected by the hydrogen gas sensor 24 is subtracted from a detection value of odiferous gas detected by the semiconductor gas sensor to separate influence of hydrogen gas so that the calculated value is outputted as a detection value of the odiferous gas sensor 26. A configuration that is composed of this kind of hydrogen separation mechanism, the semiconductor gas sensor, and the hydrogen gas sensor 24, to output a detection value corresponding to the amount of odiferous gas and hydrogen gas, is referred to as a detection value output mechanism. Calculation processing of subtracting a detection value of hydrogen gas detected by the hydrogen gas sensor 24 from a detection value of odiferous gas detected by the semiconductor gas sensor described above may be performed in the data analyzer 60, or the like. Although the present embodiment describes the hydrogen separation mechanism for separating influence of hydrogen gas from detection data acquired by the semiconductor gas sensor, it is also possible to separate influence of methane from detection data acquired by the semiconductor gas sensor by providing a methane sensor for detecting methane. A semiconductor gas sensor with a detecting portion formed of material adjusted so as to strongly react to methane may be used as the methane gas sensor.

Many people have no methane producer that produces methane in their intestine, or have very low amount thereof if existing, so that many people have a very low amount of methane contained in defecation gas. Thus, in the present embodiment, the hydrogen sensor 24 and the carbon dioxide sensor 26 are provided as a healthy-state gas sensor. However, a few people have a very large amount of methane producer in their intestines. Defecation gas of people having a very large amount of intestinal methane producer as described above contains a large amount of produced methane, but contains a low amount of produced hydrogen. Thus, if only the hydrogen sensor 24 and the carbon dioxide sensor 26 are provided, defecation gas of people having a very large amount of intestinal methane producer is unfavorably determined that there is a small amount of discharged healthy-state gas. In the present embodiment, although the hydrogen sensor 24 and the carbon dioxide sensor 26 are provided as a healthy-state gas sensor to fit with many people, a methane gas sensor instead of the hydrogen sensor 24 may be provided to fit with people having a large amount of methane gas. In addition, it is more preferable to provide the methane gas sensor in addition to the hydrogen sensor 24 and the carbon dioxide sensor 26 in advance to be able to correspond to any test subject.

As described above, defecation gas contains a large amount of hydrogen, and the semiconductor gas sensor detects not only odiferous gas but also hydrogen. For that, influence of hydrogen can be separated by subtracting the amount of hydrogen gas detected by the hydrogen gas sensor 24 from the amount of gas detected by the odiferous gas sensor 26 of a semiconductor gas sensor, so that it is possible to accurately measure the amount of odiferous gas.

In addition, hydrogen gas contained in defecation gas has very small molecular weight as compared with air to be easily released from the bowl 2a. For that, in the present embodiment, defecation gas is sucked by the fan 18c of the suction device 18 to enable defecation gas containing hydrogen gas to be reliably collected.

If sucked defecation gas is returned into the bowl 2a as it is, measurement accuracy by the odiferous gas sensor 26 decreases. In contrast, in the present embodiment, sucked defecation gas is deodorized by the deodorant filter 78 to be returned into the bowl to enable the amount of odiferous gas and hydrogen to be accurately measured. In addition, although the deodorant filter 78 as above is required to be arranged downstream of each sensor, if the deodorant filter 78 as above is provided downstream of each sensor, the sensor may be directly contaminated by foreign material. In contrast, in the present embodiment, the filter 72 without a deodorizing function is provided upstream of a sensor to enable contamination of the sensor by foreign material to be reduced without affecting measurement of odor components.

If gas is sucked into the bowl 2a, pressure in the bowl 2a decreases, and thus stink gas components attached to a body and clothes of a test subject may flow into the bowl 2a. In contrast, in the present embodiment, air after odor components have been deodorized is returned into the bowl 2a, so that stink gas components attached to a body and clothes of a test subject are prevented from flowing into the bowl 2a to enable accurate measurement.

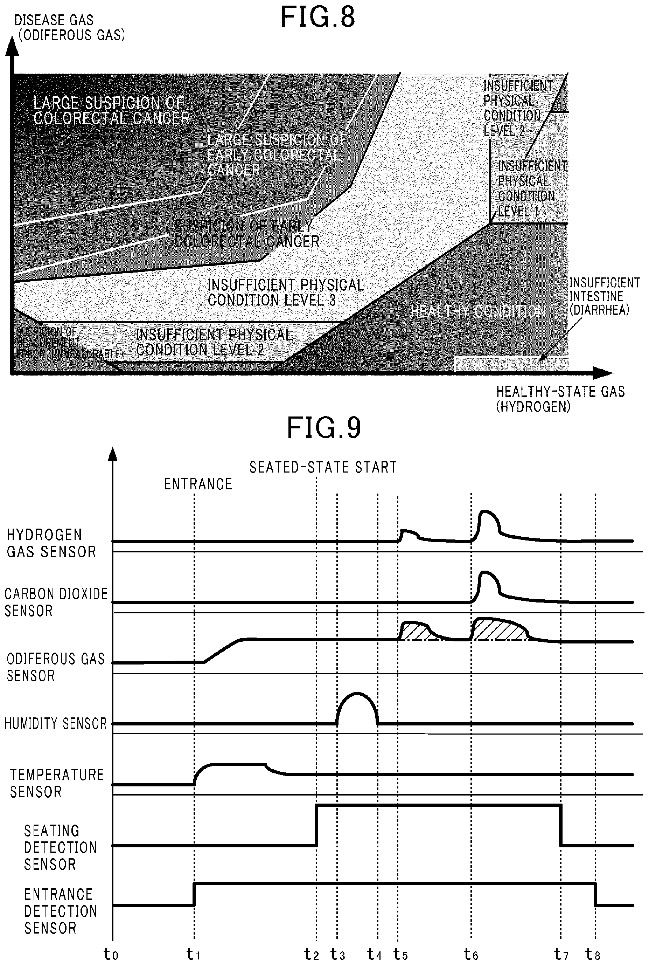

A configuration in which air after being deodorized to remove odor components is returned into the bowl 2a is not essential. If the configuration in which air after being deodorized to remove odor components is returned into the bowl 2a as above is not used, stink gas components attached to a body and clothes of a test subject may flow into the bowl 2a. However, as described later with reference to FIG. 9, when a reference value of residual gas is set, the reference value of residual gas is set by including influence of the stink gas components attached to a body and clothes of the test subject. Thus, it is possible to estimate the amount of gas without returning air after being deodorized to remove odor components into the bowl 2a.

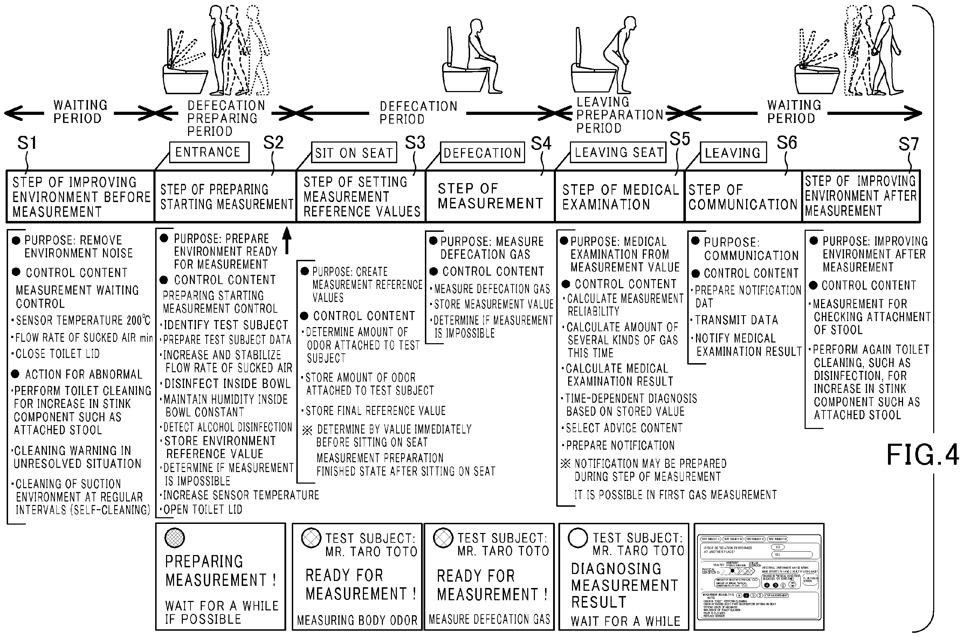

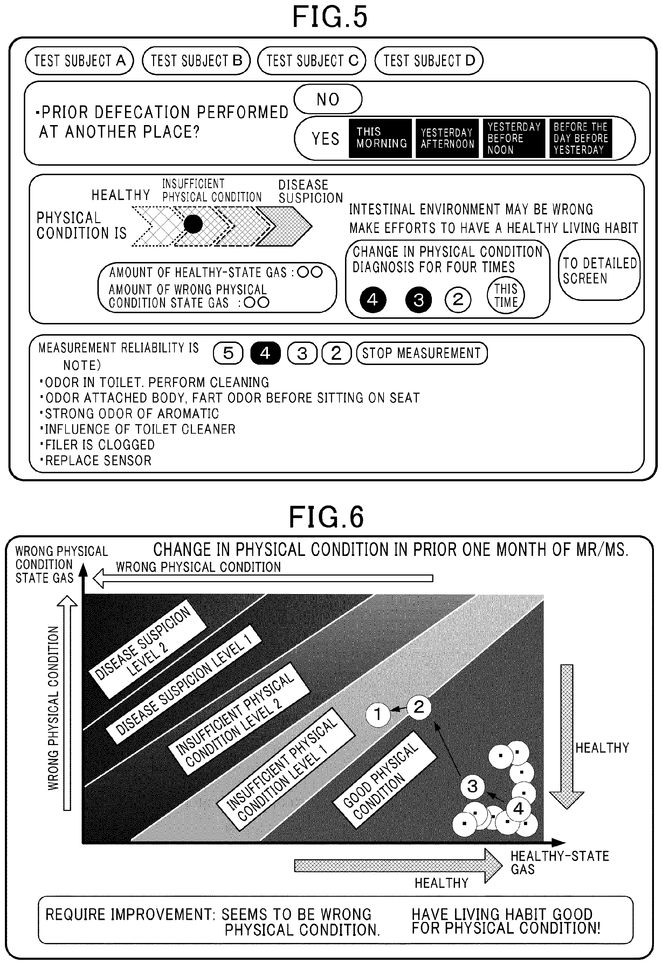

Next, with reference to FIGS. 4 and 5, a flow of measurement of physical condition by the biological information measurement system 1 in accordance with the first embodiment of the present invention will be described.

FIG. 4 describes a flow of measurement of physical condition, and an upper section shows each step of the measurement of physical condition, as well as a lower section shows an example of screens to be displayed in a display device of a remote control in each step. FIG. 5 shows an example of the screens to be displayed in the display device of the remote control.

The biological information measurement system 1 of the present embodiment analyzes physical condition including determination of cancer on the basis of a correlation between odiferous gas and healthy-state gas, in defecation gas discharged by a test subject during defecation. In each device on a test subject side, it is preferable that an analysis result is displayed during defecation, or in a short time until leaving a toilet installation room after one defecation period has been finished. However, if analysis is performed in a short time, analysis accuracy may decrease. It is difficult that the suction device 18 sucks the whole of defecation gas discharged by a test subject, and a condition where the inside of a toilet or a toilet installation room is very unsanitary, or a measurement environment with a strong aromatic, becomes a disturbance that affects measurement accuracy so that it may decrease. Thus, when physical condition including whether there is a disease or not is notified to a test subject in each device on a test subject side, in consideration of a mental burden of the test subject, it is devised that not only an absolute amount of odiferous gas having a strong relationship with cancer, but also change in physical condition of a test subject, or change in intestinal conditions, is strongly notified to the test subject, on the basis of time-dependent results acquired by measurement performed during defecation act performed many times for a long time. In addition, also in consideration of a measurement error during each defecation act, in the present embodiment, it is devised that physical condition is notified to a test subject on the basis of measurement results during one defecation act so that the physical condition to be notified to the test subject does not largely changes. The device is based on using characteristics of disease of cancer that develops for a long time, because if the amount of odiferous gas having a strong relationship with cancer is largely changed for a short time, it is not caused by a strong relationship with cancer, but largely caused by a result of a bad living habit or influence of noise, whereby a large change in physical condition may apply unnecessary mental anxiety to the test subject.

In the light of the above matter, in the present embodiment, the device 10 on a test subject side simply analyzes health condition on the basis of measurement results of defecation gas discharged first in one defecation act, or defecation gas discharged during the first excretory act to display an analysis result of the health condition. In contrast, the server 12 is capable of a detailed analysis on the basis of a total amount of gas discharged during one defecation act by comparing it with that of other test subjects, and the like. Then, in the biological information measurement system 1 of the present embodiment, the device 10 on a test subject side installed in the toilet installation room R performs a simple analysis, and the server 12 performs a more detailed analysis.

As shown in FIG. 4, in measurement during one defecation act by the biological information measurement system 1 of the present embodiment, the following steps is performed: step S1 of improving environment before measurement; step S2 of preparing starting measurement; step S3 of setting measurement reference values; step S4 of measurement; step S5 of medical examination; step S6 of communication; and step S7 of improving environment after measurement.

Step S1 of improving environment before measurement is performed before a test subject enters the toilet installation room R. The entrance detection sensor 34 (refer to FIG. 2) detects whether a test subject enters the toilet installation room R, or not.

In step S1 of improving environment before measurement, the control device 22 on a seat side allows the sensor heater 54, the suction device 18, and the toilet lid opening/closing device 40, to switch to a measurement waiting mode to control them. The sensor heater 54 is controlled in the measurement waiting mode on the basis of temperature measured by the temperature sensor 32 so that temperature of a detecting portion of the odiferous gas sensor 26 becomes waiting temperature (such as 200°C) lower than temperature when measurement is performed. The suction device 18 is controlled in the measurement waiting mode so that a flow rate of sucked air becomes minimum. The toilet lid opening/closing device 40 is controlled in the measurement waiting mode so that a toilet lid is closed.

In step S1 of improving environment before measurement, although the detecting portion of the odiferous gas sensor 26 is at a temperature lower than an optimum temperature because the sensor heater 54 is in the measurement waiting mode, it is possible to measure concentration of odiferous gas. If there is an occurrence source of stink gas in the bowl 2a, such as a case where there is a stool attached to the flush toilet 2, or the like, concentration of gas measured by the odiferous gas sensor 26 becomes a predetermined value or more. The control device 22 allows toilet cleaning to be performed if the concentration of gas measured by the odiferous gas sensor 26 exceeds a predetermined value in step S1 of improving environment before measurement. Specifically, the control device 22 performs as follows: allows the nozzle driving device 42 to discharge cleaning water through a nozzle to clean the bowl 2a; allows the toilet cleaning device 46 to discharge water stored in a cleaning water tank into the bowl 2a to clean the inside of the bowl 2a; or allows the toilet disinfection device 48 to create disinfecting water, such as hypochlorous acid water, from tap water, or the like to spray disinfecting water created onto the bowl 2a to disinfect the bowl 2a.

If the concentration of gas measured by the odiferous gas sensor 26 is a predetermined value or more, the control device 22 also enables the suction device 18 to discharge gas in the bowl 2a to reduce concentration of gas. Gas sucked by the suction device 18 is deodorized by the deodorant filter 78, so that the suction device 18 and the deodorant filter 78 serve as a deodorizing device. The suction device 18 sucks gas while the toilet lid is opened to enable not only the inside of the bowl 2a but also the inside of the toilet installation room R to be deodorized, so that the suction device 18 and the deodorant filter 78 can also serve as a toilet installation room deodorizing device. Preferably, if the suction device 18 and the deodorant filter 78 serve as a deodorizing device, the amount of gas to be sucked by the suction device 18 is increased as compared with when measurement of physical condition is performed during defecation of a test subject.

Alternatively, the control device 22 may be configured so as to be able to control a ventilator (not shown) provided in the toilet installation room R to allow the ventilator to operate to reduce concentration of gas. In this way, concentration of odiferous gas remaining in the bowl 2a is reduced to reduce influence of residual gas noise caused by the gas remaining. Thus, cleaning or disinfection of the bowl 2a by the nozzle driving device 42, and the toilet cleaning device 46 or the toilet disinfection device 48, as well as ventilation and deodorizing inside the bowl 2a or the toilet installation room R, performed in step S1 of improving environment before measurement, serves as noise-responding means for reducing influence of residual gas noise, and residual gas removal means for reducing concentration of residual odiferous gas. The noise-responding means performed when a test subject does not enter the toilet installation room R, or in a period other than during defecation of a test subject, serves as first noise-responding means, as well as the residual gas removal means.

In step S1 of improving environment before measurement, if the amount of gas measured by the odiferous gas sensor 26 is not less than a predetermined value even if the toilet cleaning described above is performed, the control device 22 allows the transmitter-receiver 56 to transmit a cleaning warning command signal. When the transmitter-receiver 66 on the remote control 8 side receives the cleaning warning command signal, the display device 68 or the speaker 70 notifies a test subject that toilet cleaning should be performed.

In addition, in step S1 of improving environment before measurement, the control device 22 allows cleaning of suction environment to be performed at regular intervals. Specifically, the control device 22 allows the duct cleaner 58 to operate to spray cleaning water into the duct 18a of the suction device 18 to clean the duct 18a, and the like. Further, the sensor heater 54 heats each of detectors of the hydrogen gas sensor 24, the odiferous gas sensor 26, and the carbon dioxide sensor 28, to a high temperature of a cleaning temperature, to perform sensor cleaning of burning stink gas components attached to a surface of each of the detector of the gas sensors 24, 26, and 28.

Next, when the entrance detection sensor 34 detects entrance of a test subject, the control device 22 transmits a signal of starting step S2 of preparing starting measurement to the transmitter-receiver 66 on the remote control 8 side through the transmitter-receiver 56, and then step S2 of preparing starting measurement is performed in synchronization with the remote control side.

In step S2 of preparing starting measurement, first, the test subject identification device 62 built in the remote control 8 identifies a test subject. Specifically, in the biological information measurement system 1, a resident of a house in which the system is installed is registered, and a registered resident is displayed as a candidate of the test subject. That is, as shown in FIG. 5, buttons of respective candidates, such as a "test subject A", a "test subject B", and a "test subject C", are displayed in an upper portion of the display device 68 of the remote control 8, and then a test subject entering the toilet installation room R presses a button corresponding to oneself to identify the test subject. In addition, the data analyzer 60 built in the remote control 8, with reference to data in a storage device, acquires previous measurement data on personal identification information received by the test subject identification device 62, and a physical condition display table as reference data to be a basis of analysis.

In addition, in step S2 of preparing starting measurement, the data analyzer 60, as shown in FIG. 5, allows a display device to display a message in a second section of its screen, such as: a question about whether previous defecation was performed in the toilet installation room in which this device is installed, such as "Was previous defecation performed in another place?"; and options of answers to the question, such as "Yes (This morning)", "Yes (Yesterday afternoon)", "Yes (Yesterday before noon)", "Before the day before yesterday", and "No". Once a test subject answers these questions, the input device 64 of the data analyzer 60 receives defecation history information on the test subject. This kind of defecation history information on elapsed time from previous defecation act of a test subject is stored in a storage device (test subject information storage device) built in the remote control 8, and the test subject information storage device also stores information on a test subject previously registered, such as weight, age, and sex. The defecation history information is transmitted to the server 12 to be recorded in a database of the server 12.

In step S2 of preparing starting measurement, the control device 22 on a toilet side allows the sensor heater 54, the suction device 18, and the toilet lid opening/closing device 40 to switch to a measurement mode. The sensor heater 54 is controlled in the measurement mode on the basis of temperature measured by the temperature sensor 32 so that temperature of a detecting portion of the odiferous gas sensor 26 becomes temperature (such as 350°C) suitable for measurement. The suction device 18 is controlled in the measurement mode so that a flow rate of sucked air is increased to the extent that defecation gas does not leak to the outside of the bowl 2a to be constantly maintained at the extent so as not to vary. The toilet lid opening/closing device 40 is controlled in the measurement mode so that a toilet lid is opened.

If concentration of odiferous gas detected by the odiferous gas sensor 26 is high in step S2 of preparing starting measurement, the control device 22 allows the toilet disinfection device 48 to disinfect the inside of the bowl 2a.

In step S2 of preparing starting measurement, if humidity measured by the humidity sensor 30 is unsuitable for measurement of defecation gas by the odiferous gas sensor 26, the control device 22 transmits a signal to the humidity adjuster 59 to control it so that humidity in the bowl becomes a proper value.

In the step of preparing starting measurement, when the seat 4 is cleaned with a sheet or spraying, by using alcoholic disinfectant, the odiferous gas sensor 26 reacts to alcohol to suddenly increase concentration of gas. In this way, if concentration of gas measured by the odiferous gas sensor 26 suddenly increases, the data analyzer 60 allows the display device 68 to display a warning.

The data analyzer 60 stores a measurement value measured by the odiferous gas sensor 26, as an environment reference value of a noise level to be a basis of measurement of defecation gas. The data analyzer 60 then determines whether the measurement of defecation gas is possible or not on the basis of the environment reference value. If the data analyzer 60 determines that measurement of a noise level being performed, or the measurement of defecation gas is impossible, the display device 68 is allowed to display a message, such as "During measurement preparation. Wait for a while if possible", as shown in a lower section of FIG. 4, to urge a test subject to wait for defecation.

In this way, in step S2 of preparing starting measurement, a level of noise composed of noise caused by odiferous gas remaining before a test subject enters the toilet installation room, noise of a test subject caused by odiferous components attached to the test subject who enters the toilet installation room, and the like, is determined before the test subject sits on a seat so that the level is stored as a reference value of noise caused by an environment and a test subject, as well as possibility of measurement is determined.

Next, when the seating detection sensor 36 detects that a test subject sits on a seat, the control device 22 transmits a signal of starting step S3 of setting measurement reference values to the data analyzer 60 through the transmitter-receiver 56, and then step S3 of setting measurement reference values is performed in synchronization with the data analyzer 60. If the seating detection sensor 36 repeats detection and non-detection predetermined times, this state is caused by influence of cleaning of the seat by the test subject, whereby it is desirable to return to S1 in this kind of state.

In step S3 of setting measurement reference values, the data analyzer 60 determines noise of stink gas attached to a test subject, or a level of noise caused by a test subject, on the basis of a measurement value measured by the odiferous gas sensor 26. That is, if a measurement value measured by the odiferous gas sensor 26 is insufficiently reduced and is unstable, it is determined that there is a possibility that disinfection is performed by using alcoholic disinfectant or the like to continue the display, "During measurement preparation. Wait for a while if possible", shown in the lower section of FIG. 4. Alternatively, if a level of noise caused by a test subject is a predetermined value or more, the data analyzer 60 transmits a signal to the nozzle driving device 42 of a local cleaning device to allow the nozzle driving device 42 to operate to clean the anus of a test subject, or the data analyzer 60 allows the display device 68 to notify a test subject that anus cleaning should be performed. In this way, indication of performing anus cleaning and notification encouraging the anus cleaning, as well as notification of a large noise to a test subject, by the data analyzer 60, serves as second noise-responding means for reducing noise of a test subject by action different from that of the first noise-responding means. While the first noise-responding means described above is performed when no test subject enters the toilet installation room R, the second noise-responding means is performed when a test subject is in the toilet installation room R. On the other hand, if a measurement value measured by the odiferous gas sensor 26 is sufficiently reduced, this display is erased. In addition, if a measurement value measured by the odiferous gas sensor 26 is insufficiently reduced even if a predetermined time has elapsed, the data analyzer 60 stops measurement of physical condition and allows the display device 68 to display the stop to notify a test subject. In this way, if the data analyzer 60 determines that gas components in the bowl 2a before a period during defecation of a test subject is unsuitable for measurement, the data analyzer 60 stops the measurement of physical condition of a test subject to serve as output result stabilizing means.

In addition, in step S3 of setting measurement reference values, the data analyzer 60, as described later, sets a reference value for estimating the amount of gas, on the basis of concentration of gas measured by the odiferous gas sensor 26.

Next, the data analyzer 60, as described later, determines that a test subject performs an excretory act if a measurement value measured by the odiferous gas sensor 26 largely rises from the reference value. The data analyzer 60 performs step S4 of measurement from when determining that the test subject performs an excretory act until when the seating detection sensor 36 detects that the test subject leaves the seat.

In step S4 of measurement, the control device 22 allows a storage device to store detection data for each test subject identified by test subject identification device 62, the detection data being measured by the hydrogen gas sensor 24, the odiferous gas sensor 26, the carbon dioxide sensor 28, the humidity sensor 30, the temperature sensor 32, the entrance detection sensor 34, the seating detection sensor 36, and the defecation/urination detection sensor 38. The control device 22 transmits these measurement values stored in the storage device to the data analyzer 60 through the transmitter-receiver 56, after step S4 of measurement is finished. In the present embodiment, although the measurement values are transmitted to the data analyzer 60 from the control device 22 after step S4 of measurement is finished, besides this, the measurement values may be transmitted in real time in parallel with measurement.

The control device 22 starts measurement of defecation gas even if a test subject inputs no information identifying the test subject into the test subject identification device 62. After then, if the test subject inputs information on the test subject during one defecation, detection data detected before the information is inputted is stored in the storage device in association with the inputted information on the test subject. This is a practical device corresponding to characteristics of defecation, in which a test subject is first allowed to perform no various kinds of input in an urgent situation of defecation, and to perform the input after calming down. In addition, if the test subject inputs no information on the test subject even if a predetermined time has elapsed after measurement has been started, the display device 68 and the speaker 70 output a message for urging the test subject to perform the input to notify the test subject. Accordingly, it is possible to prevent a test subject from omitting input.

At the same time, as with step S3 of setting measurement reference values, the data analyzer 60 determines whether measurement is possible or not. If the data analyzer 60 determines that the measurement is possible, the data analyzer 60 allows the display device 68 to display a message that the measurement being performed to the test subject, such as "Subject: Mr. Taro Toto (identification information on a test subject)", and "Measurement is ready. Measurement being performed", as shown in the lower section of FIG. 4.

Next, when the seating detection sensor 36 detects that a test subject leaves the seat, the control device 22 transmits a signal of starting step S5 of medical examination to the data analyzer 60 through the transmitter-receiver 56. When receiving the signal, the data analyzer 60 starts step S5 of medical examination.

The data analyzer 60 first calculates reliability of measurement that is described later, on the basis of a measurement value measured by each sensor.

On the other hand, if no information identifying a test subject is inputted after the test subject has left the seat, the control device 22 prohibits cleaning of the flush toilet 2. That is, if no information for identifying a test subject is inputted, the control device 22 does not allow the flush toilet 2 to discharge cleaning water and allows a message urging the test subject to perform input to be displayed even if the test subject operates a cleaning button (not shown) of the remote control 8. Accordingly, it is possible to strongly urge a test subject to input information for identifying a test subject.

The data analyzer 60, as described later in detail, also estimates the amount of odiferous gas and hydrogen gas (healthy-state gas).

In step S5 of medical examination, the data analyzer 60 performs calculation of results of a medical examination to analyze physical condition of a test subject on the basis of time-dependent change in a plurality of detection data items that is detected in defecation performed multiple times in a predetermined period and that is stored in a storage device, as well as performs time-dependent diagnosis based on stored values, and then selects advice contents based on the time-dependent diagnosis. The data analyzer 60, as shown in a third section from the top of FIG. 5, allows the display device 68 to display advice contents selected as a message related to health management. In an example shown in FIG. 5, present physical condition of a test subject that corresponds to "insufficient physical condition" is displayed as a result of a medical examination is displayed, as well as "Intestinal environment may be wrong. Make efforts to have a healthy living habit" is displayed as an advice.

In a portion below that of the result of a medical examination, there is displayed the amount of healthy-state gas, such as hydrogen gas, or carbon dioxide gas, as well as the amount of wrong physical condition state gas, such as odiferous gas, in the measurement in this time. In a portion below that of the advice, measurement results of previous four times measurements are displayed together. If a test subject presses a button of "detailed screen" in a display screen, there is displayed a table showing change in physical condition of a test subject for the last one month. This display will be described later. In this way, analysis results displayed in the display device 68 of the remote control 8 include only a state of physical condition, an advice, and change in physical condition (history of measurement data), and include no notification related to a determination result of disease of cancer, such as displayed in the medical facility terminal 16. These analysis results may be notified in the terminal 14 for a test subject.

As shown in a lowermost section of FIG. 5, reliability of measurement data in this time is displayed in a lower portion of a screen of the display device 68. In the example shown in FIG. 5, the reliability is displayed as "4" that is relatively high. If the reliability is low, a cause of decrease in reliability as well as an advice for improving the decrease is displayed in a portion below that of display of the reliability. For example, if residual gas noise caused by gas remaining in a bowl, or test subject noise caused by a test subject, is large, a test subject is notified that the noise reduces the reliability to affect measurement results. Thus, the display of reliability by the display device 68 serves as noise-responding means. Calculation of the reliability will be described later.

Next, when the entrance detection sensor 34 detects that a test subject leaves the toilet installation room R, the control device 22 transmits a signal of transmitting data to the data analyzer 60 through the transmitter-receiver 56. When receiving the signal, the data analyzer 60 performs step S6 of communication.

In step S6 of communication, the data analyzer 60 transmits the following to the server 12 through a network: information for distinguishing a test subject identified by the test subject identification device 62; data measured by various sensors; calculated reliability; information on a measurement date and time; stool condition information on at least one of the amount of stool and a state of the stool acquired by the defecation/urination detection sensor 38; and notifying data including defecation history information. The server 12 records the information received in a database.

The control device 22 also performs step S7 of improving environment after measurement after the entrance detection sensor 34 has detected that a test subject has left the toilet installation room R.

The control device 22 allows the odiferous gas sensor 26 to measure concentration of gas in step S7 of improving environment after measurement. If concentration of gas measured by the odiferous gas sensor 26 is larger than a predetermined value even if a predetermined time has elapsed after a defecation period has been finished, the control device 22 determines that there is a stool attached to the bowl 2a of the flush toilet 2 to allow the toilet cleaning device 46 to discharge cleaning water stored in a cleaning water tank into the bowl 2a to clean the inside of the bowl 2a, or to allow the toilet disinfection device 48 to create disinfecting water, such as hypochlorous acid water, from tap water, or the like to spray disinfecting water created onto the bowl 2a to disinfect the bowl 2a.

The additional toilet cleaning by the toilet cleaning device 46, as well as the disinfection of the bowl 2a by the toilet disinfection device 48, serves as residual gas removal means for reducing concentration of remaining odiferous gas. Preferably, toilet cleaning performed automatically by the residual gas removal means is set so that its cleaning capability is higher than that of usual toilet cleaning performed by allowing a test subject to operate a cleaning switch (not shown) of the remote control 8. Specifically, it is preferable that the toilet cleaning performed by the residual gas removal means is set to have a high frequency of discharge of cleaning water into the bowl 2a, or flow velocity of the cleaning water is set high. The disinfection of the bowl 2a performed by the residual gas removal means is set so that its disinfection capability is higher than that of usual disinfection of the bowl performed by allowing a test subject to operate a disinfection switch (not shown) of the remote control 8. Specifically, the disinfection of the bowl performed by the residual gas removal means is set so that water for disinfection of higher concentration as compared with usual disinfection is sprayed, or a large amount of water for disinfection is sprayed.

If concentration of gas measured by the odiferous gas sensor 26 is more than a predetermined value even if a predetermined time has elapsed after a defecation period has been finished, the residual gas removal means determines that there is a contamination in the duct 18a to allow the duct cleaner 58 to operate. The duct cleaner 58 cleans the inside of a duct 18a attached to the suction device 18 with hypochlorous acid acquired by electrolysis of tap water, or the like.

If concentration of gas measured by the odiferous gas sensor 26 does not decrease sufficiently and is still more than the predetermined value even if the cleaning and the disinfection processing, described above, are performed, the residual gas removal means allows the display device 68 to display a message of encouraging cleaning of the flush toilet 2.

Then, in step S7 of improving environment after measurement, the control device 22 allows the sensor heater 54, the suction device 18, and the toilet lid opening/closing device 40 to switch to the measurement waiting mode to finish one measurement.

Next, with reference to FIG. 6, the physical condition display table will be described. The physical condition display table is to be displayed by pressing the button of "detailed screen" in the display screen shown in FIG. 5. A storage device on the remote control 8 side stores the physical condition display table, defecation dates and times of a test subject in association with identification information on the test subject, and previous measurement data, for each test subject. Although the previous measurement data stored in the storage device on the remote control 8 side may be data throughout a defecation period, measurement data on defecation gas discharged by the first excretory act in the defecation period (the first measurement data during the excretory act) is preferable due to capacity of the storage device.

As shown in FIG. 6, the physical condition display table is determined on the basis of an experiment performed by the present inventors, described above, and is a graph in which the vertical axis represents an index related to the amount of odiferous gas (referred to as wrong physical condition state gas in the display), referred to as a first index, and the horizontal axis represents an index related to the amount of healthy-state gas, referred to as a second index. The first index relates to the amount of odiferous gas based on first detection data detected by the gas detector 20, and the second index relates to the amount of hydrogen gas of healthy-state gas based on second detection data detected by the gas detector 20. The display device 68 of the remote control 8 displays the physical condition display table with the vertical axis and the horizontal axis as above, in which a measurement result of defecation gas of a test subject is plotted in a time-dependent manner. That is, as shown in FIG. 6, a plotted point representing the latest measurement result of the same test subject is referred to as "1", that representing the last result is referred to as "2", that representing the last but one result is referred to as "3", and the like, and then each of plotted points of the last thirty times is displayed with a numeral. Accordingly, a test subject can recognize time-dependent change in his or her own physical condition. Although the present embodiment displays plotted points of thirty times, those of a few weeks and a few months may be available, or those in units of year may be also available because cancer develops in years. It is more desirable to enable a test subject to change a display range according to a situation. Further, it is needless to say that if a display range is wide, it is more preferable to change a display method in consideration of viewability so that monthly averages of plotted points for one year, or two years, are used.

The physical condition display table sets regions of a plurality of stages corresponding to whether physical condition is good or wrong, in accordance with a relationship between the index related to healthy-state gas and the index related to odiferous gas, such as: a "disease suspicion level 2", a "disease suspicion level 1", an "insufficient physical condition level 2", an "insufficient physical condition level 1", and a "good physical condition". As shown in FIG. 6, the "disease suspicion level 2" corresponding to the worst state of physical condition is set in a upper-left region in the physical condition display table, where the amount of odiferous gas is maximum and the amount of healthy-state gas is minimum. On the other hand, the "good physical condition" corresponding to the best state of physical condition is a lower-right region in the physical condition display table, where the amount of odiferous gas is minimum and the amount of healthy-state gas is maximum. The "disease suspicion level 1", "insufficient physical condition level 2", and "insufficient physical condition level 1", showing physical condition levels between the worst and best conditions, are set in the order from the upper-left in the physical condition display table as belt-like regions rising diagonally up and to the right. This kind of physical condition display table is preset in accordance with weight, age, sex, and the like of a test subject, and displaying plotted points based on the first and second indexes in the table enables analysis based on detection data and test subject information to be performed.

As above, in the present embodiment, two indexes of the index related to the amount of odiferous gas and the index related to the amount of healthy-state gas are used, so that it is possible to evaluate physical condition of a test subject and change in physical condition thereof in more detail. For example, even in a case where the amount of healthy-state gas showing a good physical condition is large, if the amount of odiferous gas is also large, evaluation is not the level of the best physical condition (the upper-right region in the physical condition display table). Conversely, even in a case where the amount of healthy-state gas showing a good physical condition is very low, if the amount of odiferous gas is low, evaluation is not the level of the worst physical condition (the lower-left region in the physical condition display table).

For example, a boundary line between the "insufficient physical condition level 1" and the "insufficient physical condition level 2" showing a worse state than that of the level 1 is drawn rising diagonally up and to the right so that as the amount of the index related to healthy-state gas in the horizontal axis increases, the index related to the amount of odiferous gas in the vertical axis also increases, and the "insufficient physical condition level 2" showing a state where physical condition is wrong is distributed on a side of the boundary line where the index related to the amount of odiferous gas is large. The boundary line is set in this way, so that in the present embodiment, even if the amount of the index related to healthy-state gas in the horizontal axis is the same value, evaluation of physical condition varies depending on a value of the index related to the amount of odiferous gas in the vertical axis. In order to acquire the same evaluation, it is required that as a value of the amount of odiferous gas in the vertical axis increases, a value of the amount of healthy-state gas in the horizontal axis also increases.

The storage device on the remote control 8 side stores advices corresponding to the states of physical condition. Specifically, there are stored advices, such as: "Present to a hospital" corresponding to a state of physical condition, the "disease suspicion level 2"; "Recommend presenting to a hospital" corresponding to a state of physical condition, the "disease suspicion level 1"; "Concern for disease increases. Reduce stress and improve a living habit immediately" corresponding to a state of physical condition, the "insufficient physical condition level 2"; "Intestinal environment is wrong. Make an effort to have a healthy living" corresponding to a state of physical condition, the "insufficient physical condition level 1"; and "Physical condition is good" corresponding to a state of physical condition, the "good physical condition". In the physical condition display table, plotted points showing physical condition of a test subject, as well as an advice corresponding to a region where the latest plotted point is positioned is displayed.

However, the display device 68 of the remote control 8 does not plot each of analysis results acquired by the data analyzer 60 as it is in the physical condition display table, and plots each of the analysis results at a position to which each of them is displaced after predetermined correction has been applied to each of them. It is assumed that the biological information measurement system 1 of the present embodiment detects disease, such as colorectal cancer, and this kind of disease does not steeply develop in a few days. Meanwhile, the biological information measurement system 1 of the present embodiment sucks defecation gas from the bowl 2a of the flush toilet 2 installed in the toilet installation room R to analyze the sucked gas, and it is impossible to collect all of the defecation gas. In addition, there is a possibility that various factors, such as that a test subject wears perfume, and that gas to which the odiferous gas sensor 26 is sensitive, such as odiferous gas, remains in the toilet installation room R, may cause an error in measurement results of physical condition.

Thus, if physical condition displayed on the basis of one measurement result of a test subject greatly inclines toward wrong physical condition, an unnecessary mental burden is applied to a test subject. In addition, if a measurement result of physical condition greatly varies for each measurement, it results in losing confidence of a test subject in a measurement result of physical condition. Thus, the biological information measurement system 1 of the present embodiment allows the data analyzer 60 to apply correction to an analysis result to prevent a measurement result to be displayed from greatly varying for each measurement. However, detection data stored in the storage device of the remote control 8 and detection data transmitted to the server 12 to be stored, to which no correction is applied, are stored along with reliability of the detection data. It is preferable that the storage device of the remote control 8 stores a coordinate of a display after correction in consideration of a next display. All of detection data acquired by the biological information measurement system 1 of the present embodiment in this way does not have high reliability. However, if data on daily defecation act is continuously acquired for a long period to be accumulated in the storage device of the remote control 8 and the server 12, it is possible to detect change in physical condition of a test subject for a long period. As a result, it is possible to call attention to a test subject before physical condition of the test subject is greatly deteriorated, to prevent the test subject from having a serious disease, such as colorectal cancer.

Correction applied to detection data in this way serves as output result stabilizing means for preventing an index of physical condition of a test subject to be outputted to the display device 68 from varying toward a wrong physical condition due to a detection error, and the like.

In the present embodiment, it is not always required to apply correction to detection data to be stored in the storage device of the remote control 8, and also detection data after the correction may be stored.

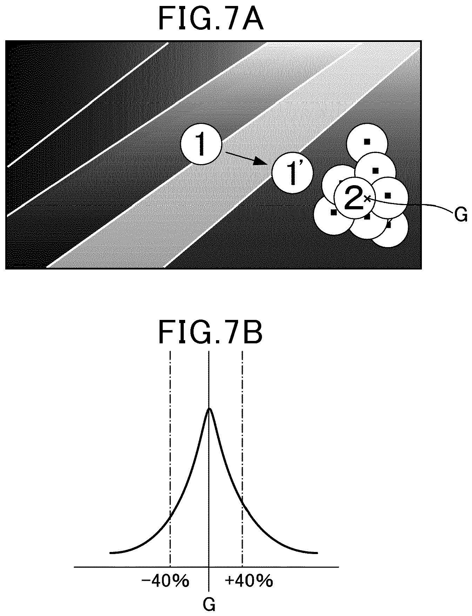

Next, with reference to FIG. 7, correction of plotted points will be described.

FIG. 7A shows an example of displacement of a plotted point of updated data by correction, and FIG. 7B shows limit processing with respect to the amount of displacement of a plotted point.

First, as shown in FIG. 7A, a plotted point calculated by the data analyzer 60 on the basis of the latest measurement is represented as "1", and the point is greatly displaced from the center G of an area of plotted points of measurement data of the last thirty times. In this way, if the plotted point "1" that is greatly displaced from distribution of measurement data up to the previous measurement is displayed, an excessive mental burden may be applied to a test subject. Since a risk of cancer does not increase in a day, it is highly possible that this kind of large change in measurement data does not show an increase in a risk of cancer, but a result of a bad living habit in the previous day, or influence of noise. In the present embodiment, correction is performed in a manner that gives due consideration for applying no excessive mental burden to a test subject. Thus, if the latest analysis result varies toward a wrong physical condition side (in an upper-left direction), the data analyzer 60 displaces a position at which the plotted point "1" is displayed in the physical condition display table toward the center G of an area by a predetermined distance on the basis of reliability of measurement data in this time to allow the plotted point "1" to be displayed. That is, in an example shown in FIG. 7A, the latest measurement data is displayed at a position of a plotted point "1"' acquired by correcting the plotted point "1" so that the plotted point "1" is displaced toward the center G of an area (on a good physical condition side), and the plotted point "1" is not actually displayed. A displacement distance of the plotted point "1" toward the center G of an area direction increases, as reliability of the latest measurement data decreases. In this way, displacing the latest plotted point on a side showing good physical condition enables a mental burden to a test subject to be reduced. Calculation of reliability of measurement data will be described later. However, if displacement of the latest plotted point toward the wrong physical condition side continues predetermined times or more, the data analyzer 60 reduce the amount of correction (the amount of correction of displacement). Accordingly, a test subject can recognize that his or her own physical condition is deteriorated, and can be encouraged to make an effort to improve the physical condition.

If a very large noise is applied to the latest measurement of physical condition to very greatly shift the latest plotted point, it is thought that physical condition displayed may be greatly displaced toward the wrong physical condition side even if the correction described in FIG. 7A is applied. Thus, as shown in FIG. 7B, there is a predetermined limit of a displacement distance of the latest data from the center G of an area. That is, displacement of the latest data from the center G of an area is limited to a range of ±40% of a coordinate value of the center G, and even if the latest data is displaced by 40% or more from the coordinate of the center G of an area, the latest data is plotted at a position displaced by 40%. For example, in a case where a coordinate value of the center G of an area is represented as (x, y), a range of coordinate values at which the latest data can be plotted is represented as (0.6x to 1.4x, 0.6y to 1.4y), and the latest data is not plotted at a position out of the range.

In addition, if displacement of the latest data exceeding this kind of 40% continues twice, a range in which the latest data can be displaced is eased to 60%. Accordingly, for example, if the coordinate value of the center G of an area is represented as (x, y), a range of coordinate values at which the latest data can be plotted is changed to that represented as (0.4x to 1.6x, 0.4y to 1.6y). Because it is thought that if a large displacement of the latest data as above occurs at high frequency, it is not a mere measurement error, but a reflection of some sort of change in physical condition of a test subject.

Next, with reference to FIG. 8, a diagnosis table on a server side will be described. Processing in the server below is performed by a data analysis circuit provided in the server 12.

FIG. 8 shows an example of a diagnosis table displayed on the server side. As described above, in the biological information measurement system 1 of the present embodiment, measurement data for all defecation periods analyzed by the data analyzer 60 is sequentially transmitted to the server 12 through the Internet to be stored in a database on the server side. This accumulated measurement data can be displayed in the medical facility terminal 16 installed in a medical facility registered by a test subject. For example, when a test subject has a medical examination in the medical facility after receiving the message, "Recommend presenting to a hospital" displayed in the display device 68 of the remote control 8, the medical facility terminal 16 enables a diagnosis table for a server to be displayed. In the diagnosis table, its vertical axis and horizontal axis represent the same indexes as those of the physical condition display table to be displayed in the display device 68 of the remote control 8, and a state of physical condition assigned to each region is more specific. A doctor refers to measurement data on a test subject stored in a database on a server 12 side in the medical facility terminal 16 to be able to refer to time-dependent physical condition of the test subject, and thus the data can be useful for inspection and treatment in the medical facility. Alternatively, it is also possible to configure the present invention so that if measurement data transmitted to the server 12 shows excessive wrong physical condition, a medical facilities registered by a test subject notifies the terminal 14 for a test subject, corresponding the test subject, of encouraging the test subject to have a medical examination.

The diagnosis table displayed in the medical facility terminal 16 is different from the physical condition display table displayed in the display device 68 of a test subject as described above. As shown in FIG. 8, the diagnosis table on the server 12 side is determined on the basis of an experiment performed by the present inventors, and in the diagnosis table, a disease state is associated corresponding to a relationship between the amount of healthy-state gas and the amount of odiferous gas. Specifically, in the diagnosis table, the following regions are set corresponding to a relationship between the amount of healthy-state gas and the amount of odiferous gas: "Large suspicion of colorectal cancer", "Large suspicion of early colorectal cancer", "Suspicion of early colorectal cancer ", "Insufficient physical condition level 3", "Insufficient physical condition level 2", "Insufficient physical condition level 1", "Healthy condition", " Insufficient intestine (diarrhea)", and "Suspicion of measurement error".

In a diagnosis table on the server side, set in this way, previous measurement data on a test subject is plotted in a time-dependent manner on the basis of a position of a plotted point to perform determination of disease of cancer, such as: "Large suspicion of colorectal cancer", "Large suspicion of early colorectal cancer", and "Suspicion of early colorectal cancer". No correction as well as no limit is applied to a plotted point displayed in the diagnosis table on the server side, so that a doctor checks data displayed for diagnosis along with its reliability in a comprehensive manner. Since a diagnosis table and a determination result displayed in the medical facility terminal 16 are set based on the premise that a doctor refers to them, a name of disease, development thereof, and the like, are more specifically displayed. If plotted points are positioned, for example, in regions related disease of cancer, such as the "Large suspicion of colorectal cancer", "Large suspicion of early colorectal cancer ", and "Suspicion of early colorectal cancer", for a long time, a message of a high possibility of disease is displayed. A doctor is able to check plotted points shown, reliability of measurement, and the like, for diagnosis in a comprehensive manner to notify a test subject of a state of the physical condition. The medical facility terminal 16 is configured to be capable of also displaying reliability calculated by referring to a database, data measured by various sensors, information on stool condition related to at least one of the amount of stool and condition of stool, and defecation history information, along with a diagnosis table in which previous measurement data is plotted in a time-dependent manner.

A large number of devices 10 on a test subject side are connected to the server 12, a large number of measurement data items of test subjects are accumulated in the server 12. In addition, a database on the server 12 side also accumulates data on disease condition acquired from a result of detailed examination of a test subject, performed in a medical facility, after the test subject has had a medical examination in the medical facility on the basis of certain measurement data. Thus, it is possible to accumulate data acquired by associating data measured by the biological information measurement system 1 of the present embodiment with actual disease condition, on the server 12 side. The diagnosis table on the server side is sequentially updated on the basis of measurement data on a large number of test subjects accumulated in this way, so that it is possible to perform diagnosis with higher accuracy on the basis of the updated diagnosis table. It is also possible to update the physical condition display table on the basis of the data accumulated on the server side. The physical condition display table updated on the basis of the data on the server side is downloaded into each of the devices 10 on a test subject side through the Internet to be displayed in the display device 68 of the remote control 8. Even if the physical condition display table is updated, a message to be shown to a test subject is corrected to an appropriate content in the physical condition display table that is to be directly presented to the test subject.

Next, with reference to FIG. 9, data detected by each of sensors provided in the biological information measurement system 1 of the present embodiment, and estimation of the amount of gas based on the data, will be described.

FIG. 9 is a graph schematically showing a detection signal of each of the sensors provided in the biological information measurement system 1 in one excretory act of a test subject. FIG. 9 shows a waveform of a detection signal of each of the sensors, such as the hydrogen gas sensor 24, the carbon dioxide sensor 28, the odiferous gas sensor 26, the humidity sensor 30, the temperature sensor 32, the seating detection sensor 36, and the entrance detection sensor 34, in the order from an upper section.

Estimation of the amount of gas based on a detection signal of each of the sensors is performed by the data analyzer 60 serving as physical condition state discrimination means for discriminating a physical condition state, that is, by a CPU built in the remote control 8 and a storage device, or by a CPU of the server 12 and a storage device. In the data analyzer 60, there are preset a starting threshold value of a rate of change in the amount of gas for determining starting time of an excretory act, read out from storage means of the remote control 8, and a stability threshold value with respect to the amount of gas, capable of allowing stable measurement to be performed. The term, an excretory act, here includes a fart.

First, at time t1 of FIG. 9, the entrance detection sensor 34 detects entrance of the test subject. The data analyzer 60 allows the odiferous gas sensor 26 to measure the amount of odiferous gas even in a state before the entrance detection sensor 34 detects entrance of the test subject into the toilet installation room R (time to to t₁). Even in this case, the odiferous gas sensor 26 reacts due to influence of aromatic, and remaining stool attached to the bowl 2a of the flush toilet 2 to output a certain level of a detection signal. In this way, a measurement value of the odiferous gas sensor 26 before entrance of the test subject is set as an environment reference value of the amount of gas that is residual gas noise. In a state before the entrance detection sensor 34 detects entrance of the test subject, the odiferous gas sensor 26 and the suction device 18 are in a power saving state. Accordingly, temperature of the sensor heater 54 for heating a detecting portion of the odiferous gas sensor 26 is set lower, and a rotation speed of the suction fan 18c is also reduced to reduce a flow rate of passing air.

When the entrance detection sensor 34 detects entrance of the test subject at the time t₁, the odiferous gas sensor 26 and the suction device 18 are in a startup state. Accordingly, temperature of the sensor heater 54 of the odiferous gas sensor 26 increases, as well as a rotation speed of the fan of the suction device 18 increases to suck gas at a predetermined flow rate. As a result, a detection value by the temperature sensor 32 temporarily greatly increases, and then converges to a proper temperature (after the time t₁ of FIG. 9). In the present specification, a period in which the entrance detection sensor 34 detects entrance of the test subject into the toilet installation room R (time t₁ to t₈ of FIG. 9) is referred to as one "defecation act". When the test subject enters the toilet installation room R, a detection signal detected by the odiferous gas sensor 26 increases, because the odiferous gas sensor 26 reacts to a body odor of the test subject, perfume and hair liquid used by the test subject, and the like. That is, an increment from residual gas noise before the test subject enters the toilet installation room R is test subject noise caused by the test subject. A noise measurement circuit built in the data analyzer detects residual gas noise caused by gas remaining in the bowl 2a, and test subject noise caused by the test subject. The odiferous gas sensor 26 is set at a very high sensitivity to detect a very trace amount of odiferous gas contained in the order of ppb in defecation gas discharged into a toilet to react even to the order of odor to which a human's sense of smell is insensitive.

Next, when the seating detection sensor 36 detects that the test subject sits on the seat 4 at time t₂ of FIG. 9, this time point is set as a starting point of one defecation period of the test subject. In the present specification, a period in which the seating detection sensor 36 detects whether the test subject sits on the seat 4 (time t₂ to t₇ of FIG. 9) is referred to as one "defecation period". Then, a detection value detected by the odiferous gas sensor 26 in a period after a starting point (time t₂) of the defecation period, and immediately before a start of the first excretory act described later (time t₅ of FIG. 9), is set as a reference value of residual gas.

In an example shown in FIG. 9, a detection value of the humidity sensor 30 increases in a period between the time t₃ and the time t₄ after the test subject has sat on the seat 4 at the time t₂, because urination of the test subject is detected. Then, since there is little change in a detection value of odiferous gas sensor 26, the data analyzer 60 determines that an excretory act is not performed. Subsequently, a detection value of each of the hydrogen gas sensor 24 and the odiferous gas sensor 26 steeply rises at the time t₅. In this way, if a detection value of the odiferous gas sensor 26 steeply rises in a defecation period after the test subject has sat on the seat 4, the data analyzer 60 determines that an excretory act is performed.

When the excretory act is performed, the data analyzer 60 estimates the amount of odiferous gas discharged from the test subject on the basis of a fluctuation range of an increment of a detection value of the odiferous gas sensor 26 from the reference value of residual gas (a hatched area in a graph of detection values of the odiferous gas sensor 26). That is, the data analyzer 60 sets a value of detection data at the starting point of the defecation period of the test subject as the reference value of a noise level caused by the test subject to estimate the amount of odiferous gas by the first excretory act by integrating with time a difference between a detection value acquired by the odiferous gas sensor and the reference value from a starting point to a finishing point. In this way, since the data analyzer 60 estimates the amount of odiferous gas on the basis of a difference from a reference value, it is possible to reduce influence of noise caused by a test subject. Thus, a circuit that is built in data analyzer 60 to perform this calculation serves as a noise reduction circuit, as well as serves as second noise-responding means for reducing influence of test subject noise. If a noise level caused by the test subject is a predetermined value or more, the data analyzer 60 allows the display device 68 to notify the fact. Detailed estimation of the amount of odiferous gas will be described later. Likewise, the data analyzer 60 estimates the amount of hydrogen gas discharged from the test subject on the basis of an increment of a detection value of the hydrogen gas sensor 24 from a reference value of residual gas. After an excretory act of the test subject has been performed (after the time t₅ of FIG. 9), a detection value of each of the odiferous gas sensor 26 and the hydrogen gas sensor 24 returns to the reference value of residual gas. Subsequently, when the second excretory act of the test subject is performed at the time t₆, a detection value of each of the odiferous gas sensor 26, the carbon dioxide sensor 28, and the hydrogen gas sensor 24, steeply rises again. For the second excretory act, as with the first excretory act, the amount of odiferous gas and the amount of hydrogen gas, discharged from the test subject, are also estimated on the basis of an increment from the reference value of residual gas. When the amount of odiferous gas and the amount of hydrogen gas of the second excretory act or later are estimated, the reference value may be changed for each excretory act in consideration of influence of floating stool in seal water in the bowl, and the like.

In this way, if the test subject performs excretory acts multiple times after entering the toilet installation room, or if the amount of gas of a predetermined threshold value or more is detected multiple times, the amount of defecation gas by an excretory act of each time is estimated in like manner. When the amount of defecation gas of the second excretory act or later are calculate, the reference value may be changed for each excretory act in consideration of influence of floating stool in seal water in the bowl, and the like.

Subsequently, the seating detection sensor 36 detects that the test subject leaves the seat at the time t₇ of FIG. 9 to finish the one defecation period, and then the entrance detection sensor 34 detects that the test subject leaves the toilet installation room at the time t₈ to finish the one defecation act. The data analyzer 60 estimates the amount of defecation gas by excretory act of each time until the entrance detection sensor 34 detects that the test subject leaves the toilet installation room.

Each of the remote control 8 and the server 12 determines physical condition of the test subject on the basis of the amount of defecation gas measured in this way. In this case, it is desirable to enable measurements of physical condition to be displayed on the remote control 8 side during a defecation period, or immediately after the defecation period has been finished. Then, if excretory acts are performed multiple times, stools accumulate in the bowl 2a to reduce accuracy of measurement of the amount of defecation gas, based on odiferous gas. Meanwhile, in the first excretory act, defecation gas reaching the most downstream portion of the large intestine is discharged, so that it is possible to acquire most useful information for measurement of physical condition to increase reliability of the measurement. Based on the fact, on the remote control 8 side, when the amount of defecation gas (the amount of odiferous gas and hydrogen gas) by the first excretory act is estimated, physical condition of a test subject is measured on the basis of only the amount of defecation gas by the first excretory act to be displayed in the display device 68 of the remote control 8. Alternatively, it is also possible to measure a state of physical condition by allowing a weighting of a measurement value based on detection data on an initial excretory act in one defecation act to be higher than a weighting for a later excretory act.

In contrast, on the server 12 side, it is desirable to accurately perform determination by using a total amount of defecation gas by excretory acts of multiple times. Thus, on the server 12 side, a state of physical condition of a test subject is determined on the basis of a total amount of defecation gas by excretory acts of multiple times (a total amount of odiferous gas and hydrogen gas), or more preferably, on the basis of a total amount of defecation gas by every excretory act included in one defecation period from sitting on a seat to leaving the seat. Although determination of a state of physical condition of a test subject on the server 12 side does not always require a total amount of defecation gas by every excretory act included in one defecation period, it is preferable that the determination is based on a total amount of defecation gas by every excretory act included in defecation periods of multiple times.

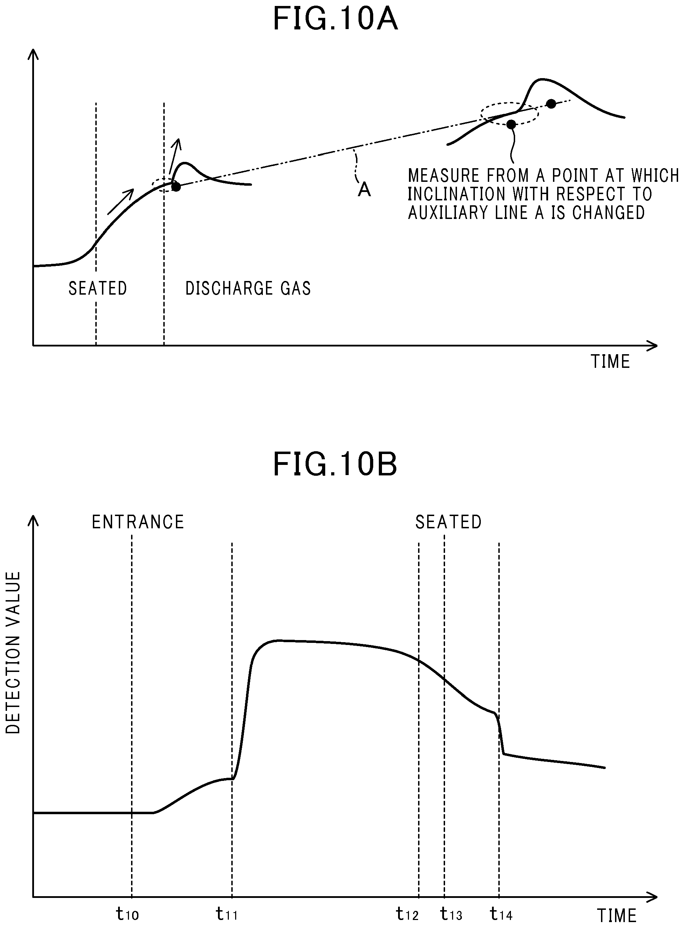

In the example shown in FIG. 9, although the reference value of residual gas is constant, it is possible to estimate the amount of discharge of odiferous gas even if the reference value is not constant. For example, if a detection value detected by the odiferous gas sensor 26 tends to increase, as shown in FIG. 10A, a reference value is indicated as an auxiliary line A that is drawn on the assumption that a rate of change in an increase of a detection value detected by the odiferous gas sensor 26 before an excretory act is started continues before and after the excretory act. Accordingly, it is possible to estimate the amount of odiferous gas by determining that one excretory act is started at the time when an inclination of detection values of the odiferous gas sensor 26 from the auxiliary line A greatly varies.

The amount of odiferous gas is estimated on the basis of a difference from a reference value that is set by using the amount of residual gas before an excretory act, so that it is desirable that there is no large change in the reference value. Thus, if a rate of change of detection values detected by the odiferous gas sensor 26 before a starting point of an excretory act (or a rate of change of a reference value of an inclination of the auxiliary line A) is a predetermined threshold value or less, the data analyzer 60 allows notification means composed of the display device 68 of the remote control 8 or the speaker 70 to notify the fact that estimation of the amount of defecation gas has high accuracy.

Meanwhile, if a spray aromatic is sprayed immediately before an excretory act, or a disinfecting sheet of an alcoholic toilet seat disinfectant or a disinfect spray is used, a detection value detected by the odiferous gas sensor 26 before the excretory act greatly varies. If a value in this kind of state is set as a reference value, it is impossible to estimate an accurate amount of odiferous gas. Thus, if a reference value of a noise level caused by a test subject is a predetermined value or more, or a rate of change of the reference value is a predetermined threshold value or more, the data analyzer 60 allows the notification means composed of the display device 68 of the remote control 8 or the speaker 70 to notify the fact that estimation of the amount of defecation gas has low accuracy. If an excretory act is performed even if this kind of notification is performed, no measurement for analysis of physical condition is performed, or reliability of measurement is reduced.

Next, with reference to FIG. 10B, detection of use of an alcoholic toilet seat disinfectant will be described. FIG. 10B is a graph showing an example of detection values of the odiferous gas sensor 26 in a case where a test subject uses an alcoholic toilet seat disinfectant.

First, after the entrance detection sensor 34 has detected entrance of a test subject at time t₁₀ of FIG. 10B, a detection value of the odiferous gas sensor 26 gradually rises because the odiferous gas sensor 26 reacts to a body odor and the like of the test subject. Next, when the test subject takes out a seat disinfecting sheet using alcoholic disinfectant at time t₁₁, the odiferous gas sensor 26 reacts to a smell of alcohol so that its detection value steeply rises. When the test subject finishes disinfecting the seat 4 at time t₁₂, and throws away the disinfecting sheet into the bowl 2a, a detection value of the odiferous gas sensor 26 immediately starts to decrease because alcoholic has high volatility. The present inventors find out that the detection value steeply increased due to the alcoholic disinfectant decreases by waiting for a while to enable measurement because characteristics of the alcoholic disinfectant described above is different from those of remaining stink gas components. However, in a case of disinfect with an alcoholic disinfecting sheet, the sheet may float in seal water when thrown away. In this case, the alcohol continues to vaporize so that the decrease of the detection value steeply increased tends to be delayed. Thus, it is desirable to discharge the sheet as described below.

Subsequently, after the seating detection sensor 36 has detected that a test subject has sat on the seat at time t₁₃, if the test subject operates the cleaning switch (not shown) of the remote control 8 to perform cleaning of the flush toilet 2, a disinfecting sheet floating in seal water in the bowl 2a is discharged to allow a detection value of the odiferous gas sensor 26 to steeply decrease. If an alcoholic disinfectant is used, the odiferous gas sensor 26 generally operates as above.

If a detection value of the odiferous gas sensor 26 steeply increases to a predetermined value or more, in a period after the entrance detection sensor 34 has detected entrance of a test subject, and before the seating detection sensor 36 detects that the test subject sits on the seat, a seat disinfection detection circuit built in the data analyzer 60 determines that the test subject disinfects the seat 4, or the like, by using an alcoholic disinfectant. The present inventors find out that it is possible to detect an act of disinfecting the seat 4 of a specific act performed by a test subject in the toilet installation room R from a detection signal of each of the entrance detection sensor 34, the seating detection sensor 36, and the odiferous gas sensor 26.

If no cleaning of the flush toilet 2 is performed for a predetermined time after the seat disinfection detection circuit has detected use of an alcoholic disinfectant and a test subject has sat on the seat, a disinfect noise-responding circuit built in the data analyzer 60 transmits a signal to the toilet cleaning device 46 to automatically perform toilet cleaning. In addition, if the seat disinfection detection circuit detects use of an alcoholic disinfectant, the disinfect noise-responding circuit allows the suction fan 18c to increase its rotation speed. Accordingly, the amount of gas sucked by the suction device 18 increases to allow alcohol components volatilized while the seat is disinfected to be actively deodorized by the deodorant filter 78, thereby enabling a detection value of the odiferous gas sensor 26 to be reduced. That is, if the seat disinfection detection circuit detects a disinfectant, the disinfect noise-responding circuit allows a deodorizing device to operate to reduce influence of noise caused by an alcoholic disinfectant.

In a state where the seat disinfection detection circuit detects use of an alcoholic disinfectant, and a detection value of the odiferous gas sensor 26 increases, the disinfect noise-responding circuit stops measurement of physical condition, and allows the display device 68 to display a message of waiting for defecation to notify a test subject of the message. Then, the disinfect noise-responding circuit allows the display device 68 to display a message of waiting for defecation until the measurement of physical condition becomes possible, to notify the test subject of the message. Accordingly, influence of noise caused by the alcoholic disinfectant is reduced. Meanwhile, a detection value of the odiferous gas sensor 26, which steeply increases by use of the alcoholic disinfectant, starts decreasing when the test subject finishes disinfection.

If a noise level detected by the odiferous gas sensor 26 is reversed to a downward tendency, the disinfect noise-responding circuit allows the display device 68 to delete the message of waiting for defecation displayed therein to notify the fact that the measurement becomes possible. That is, in a state where a noise level caused by an alcoholic disinfectant is in a downward tendency, it is possible to detect a rising edge of a detection value of the odiferous gas sensor 26, in the downward tendency. The data analyzer 60 detects a time point when a detection value of the odiferous gas sensor 26 in the downward tendency rises, as discharge of defecation gas by a test subject. In a state where the noise level detected by the odiferous gas sensor 26 decreases at a predetermined rate of change or more, the disinfect noise-responding circuit stops the measurement of physical condition to continue to display the message of waiting for defecation, because in a state where the noise level steeply decreases, a rise of a detection value by discharge of defecation gas is masked so that it is impossible to accurately detect discharge of defecation gas. In addition, it is desirable to stop the measurement in a state where a reference value greatly decreases, because a calculation error also may increase.

If a noise level is a predetermined value or more due to use of an alcoholic disinfectant, the disinfect noise-responding circuit stops measurement of physical condition, or reduces reliability of measurement. As described above, if the reliability of measurement is reduced, a plotted point in the physical condition display table described in FIG. 7A is corrected to be more greatly displaced toward a region showing good physical condition. That is, if disinfection for the seat is detected, the disinfect noise-responding circuit corrects determination of physical condition to be outputted by the display device 68 toward the region showing good physical condition.

Meanwhile, if many stools are attached to the flush toilet 2, or a large amount of aromatics are used, an absolute value of the amount of gas detected by the odiferous gas sensor 26 increases, so that a detection value of the sensor may be saturated in some cases, or measurement accuracy may be out of a high measurement accuracy band. In this kind of state, it is difficult to accurately estimate a trace amount of odiferous gas. Thus, the data analyzer 60 performs no measurement of physical condition, or reduces reliability of measurement also in a case where an absolute amount of a reference value is a predetermined threshold value or more.

In the database of the server 12, as described above, measurement data on the amount of odiferous gas and the amount of healthy-state gas of an additional test subject is sequentially accumulated. In addition, in the database of the server 12, a medical examination result for cancer acquired when a test subject has a medical examination at a medical facility is stored from the medical facility terminal 16 by being associated with identification information on the test subject. The server 12 updates a stored diagnosis table on the basis of this kind of medical examination result for cancer, and change in history of change in the amount of odiferous gas and healthy-state gas.

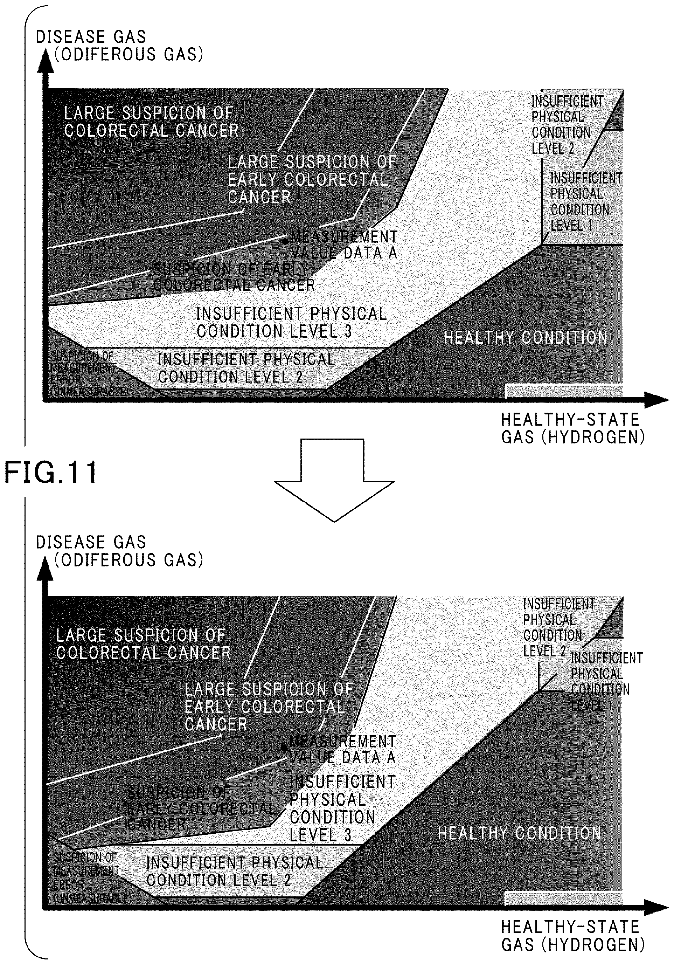

FIG. 11 shows an example of update of the diagnosis table. For example, it is assumed that analysis performed by plotting measurement data A on odiferous gas and healthy-state gas of a test subject in an old diagnosis table results in determination of the "suspicion of early colorectal cancer" is determined, and the test subject is diagnosed as early colorectal cancer by medical examination. In this kind of case, as shown in FIG. 11, the respective regions, "large suspicion of colorectal cancer", "large suspicion of early colorectal cancer", and "suspicion of early colorectal cancer", are enlarged so as to include a potion corresponding to the measurement data A on the test subject diagnosed as early colorectal cancer, and the region, "insufficient physical condition level" is narrowed. Conversely, for example, in a case where there are many test subjects diagnosed as no suspicion of cancer by results of medical examination even if it is determined that the test subjects are in the region, "suspicion of early colorectal cancer" in an old diagnosis table from a correlation between the amount of odiferous gas and that of healthy-state gas, the region, "insufficient physical condition level" is enlarged, and the respective regions, "large suspicion of colorectal cancer", "large suspicion of early colorectal cancer", and "suspicion of early colorectal cancer" are narrowed. If the diagnosis table is updated, each of the regions in the display table is also changed.

The server 12 also stores attribute information on a test subject, such as weight, age, and sex, and a plurality of physical condition display tables classified according to a tendency of history of change in measurement data on odiferous gas and healthy-state gas.

If more detailed analysis of physical condition is requested in the device 10 on a test subject side, identification information on a test subject as well as attribute information on the test subject, such as weight, age, and sex, is registered in the server 12. When measurement data on a test subject requesting such detailed analysis is accumulated in the server 12, the server 12 selects a physical condition display table of conditions close to attribute information on the test subject, and history of change in measurement data. The server 12 then transmits the selected physical condition display table to the device 10 on a test subject side through a network. When receiving an additional physical condition display table from the server 12, the device 10 on a test subject side changes a physical condition display table that is already stored to the received physical condition display table. Accordingly, it is possible to perform accurate analysis of physical condition in accordance with the attribute of the test subject and the history of measurement data in the device 10 on a test subject side.

Although the embodiment described above is configured to store history of measurement data also in the device 10 on a test subject side, besides this, the measurement data may be stored in only the database of the server 12 so that the device 10 on a test subject side reads out history of previous measurement data from the database of the server 12 to perform calculation of results of medical examination and time-dependent diagnosis in step S5 of medical examination.

Here, a method of calculating reliability in step S5 of medical examination in FIG. 4 will be described in detail. A semiconductor gas sensor used as the odiferous gas sensor 26 has a feature of detecting not only odiferous gas, but also peripheral stink gas, such as an aromatic, and a disinfecting sheet, and stink gas attached to a body and clothes of a test subject. In addition, a detection value of odiferous gas detected by the semiconductor gas sensor is also changed depending on a stool state (such as a diarrhea state or not) and the amount of stool. Thus, it is required to evaluate influence of stink gas noise and a stool state in order to determine a disease for cancer. In the present embodiment, a reliability determination circuit provided in the data analyzer 60 of the device 10 on a test subject side installed in a toilet installation room evaluates events that affect accuracy of measurement, such as influence of this kind of stink gas noise of defecation gas, a stool state, and the like, to determine reliability of measurement as an index indicating accuracy of gas detection by the gas detector 20.

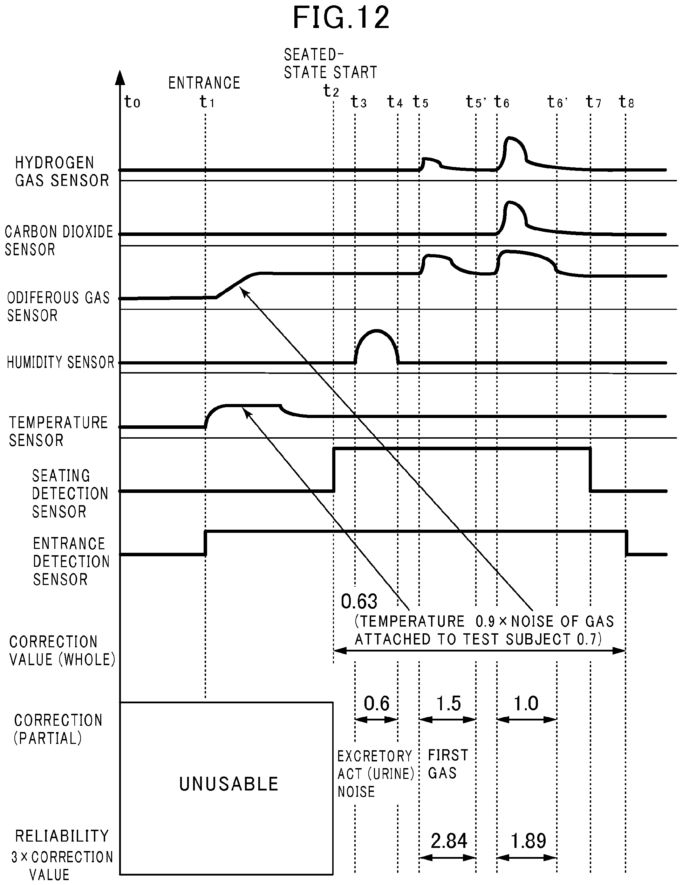

FIG. 12 is a graph for describing a method of determining measurement reliability. In description below, correction for influence of each of stink gas attached to a body and clothes of a test subject, humidity, temperature, and frequency of discharge of defecation gas, in the method will be described, for example. Determination of reliability of measurement below is performed by using the reliability determination circuit for determining reliability of detection of odiferous gas, provided in the data analyzer 60 of the remote control 8.

Output of each of the hydrogen gas sensor 24, the odiferous gas sensor 26, the carbon dioxide sensor 28, the humidity sensor 30, the temperature sensor 32, the entrance detection sensor 34, the seating detection sensor 36, and the defecation/urination detection sensor 38, provided in the measuring device 6, is transmitted to the data analyzer 60 of the remote control 8. FIG. 12 shows an example of the output from these sensors.

The data analyzer 60 of the remote control 8 previously stores a plurality of reliability correction tables for calculating the reliability.

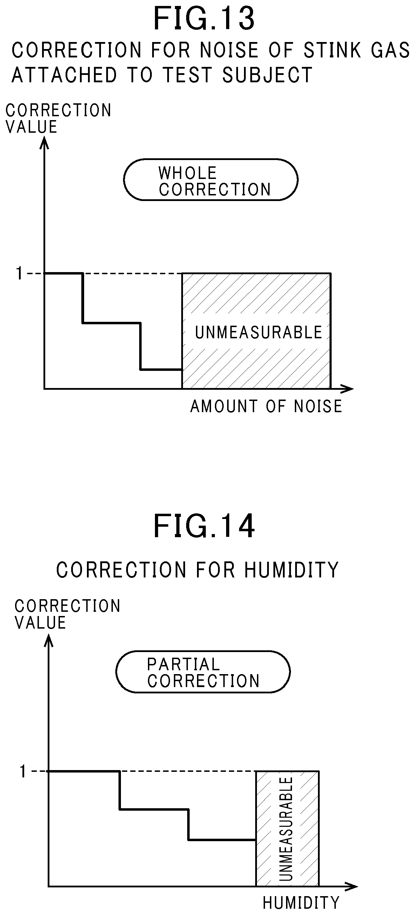

FIGS. 13 to 16 show, respectively, a correction table for noise of stink gas attached to a test subject for determining influence of stink gas attached to a body and clothes of a test subject, a correction table for humidity for determining influence of humidity, a correction table for temperature for determining influence of temperature, and a correction table for frequency of excretory acts for determining influence of frequency of excretory acts.

The semiconductor gas sensor used as the odiferous gas sensor 26 detects even stink noise (environmental noise) other than defecation gas attached to a test subject. If the amount of stink gas components attached to a test subject (the amount of noise) is large, it can be said that reliability of measurement is low. Thus, as shown in FIG. 13, a correction value is determined for the amount of noise of attached stink gas in the correction table for noise of stink gas attached to a test subject. Specifically, if the amount of stink gas components attached to a test subject is less than a predetermined value, the correction value is set at "1" at which no correction is performed. If the amount of the stink gas components attached to the test subject is the predetermined amount or more, as the amount of the stink gas components increases, the amount of correction is negatively increased from "1" to gradually reduce a reliability, and if the amount of noise of the stink gas components attached to the test subject is overly more than the predetermined amount, it is determined that measurement is impossible (the correction value is set at "0"). The amount of noise of attached stink gas is determined on the basis of detection data detected by the odiferous gas sensor 26 in a non-defecation period before the seating detection sensor 36 detects that the test subject sits on the seat. Since the stink gas components attached to the test subject affect measurement not only in a part of a defecation period but also in all of the defecation period, reliability is corrected throughout the defecation period. Hereinafter, correction of reliability throughout a defecation period in this way is referred to as "whole correction".

When a test subject urinates, humidity in the bowl 2a rises to increase humidity of gas reaching a detecting portion of the odiferous gas sensor 26. If humidity of gas reaching the odiferous gas sensor 26 increases, resistance of the odiferous gas sensor 26 changes to reduce its sensor sensitivity. In addition, if urine splashes on stool attached to the inside of the bowl 2a, the stool attached softens from a dry state so that much defecation gas may be temporarily discharged again from the stool attached while the urine splashes into the bowl 2a. The defecation gas discharged from the stool attached may be detected by the odiferous gas sensor as noise when defecation gas discharged from a test subject is measured. Thus, as shown in FIG. 14, if humidity measured by the humidity sensor 30 is less than a predetermined value, a correction value is set at "1" in the correction table for humidity. If the humidity is a predetermined value or more, reliability decreases as humidity increases, and if the humidity measurement is more than a limit value, it is determined that measurement is impossible (the correction value is set at "0"). Since urination is a temporary act, the correction table for humidity is used for "partial correction" that is applied to only a period in which change in humidity measured by the humidity sensor 30 is found. Hereinafter, correction of reliability in only a specific period in a defecation period in this way, or correction of reliability of all of the defecation period, including different correction for each period in the defecation period, is referred to as the "partial correction".

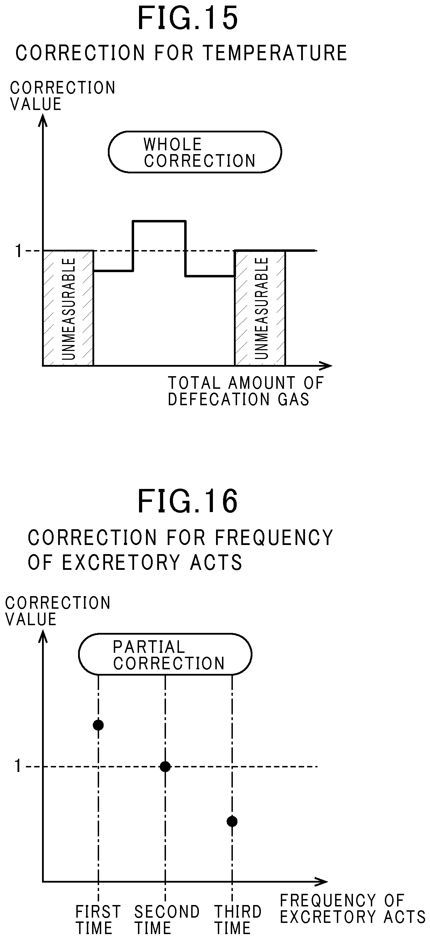

The semiconductor gas sensor used as the odiferous gas sensor 26 detects odiferous gas while its detecting portion formed of tin oxide is heated, on the basis of an oxidation-reduction reaction between oxygen adsorbed in a surface of the detecting portion and reduction gas. Thus, if temperature of the detecting portion is higher or lower than a predetermined temperature range, sensor sensitivity decreases. For this reason, as shown in FIG. 15, in the correction table for temperature, a correction value is determined depending on temperature detected by the temperature sensor 32. Specifically, if temperature detected by the temperature sensor 32 is within a suitable temperature range of measurement by the detecting portion of the odiferous gas sensor 26, a correction value is set at a value more than "1" to increase reliability, and if the temperature detected by the temperature sensor 32 is in a slightly higher or lower range than the suitable temperature range, the reliability is set at a value less than "1" to reduce the reliability. In addition, if the temperature detected by the temperature sensor 32 is higher than an upper limit value in a measurable temperature range, or lower than a lower limit value in the measurable temperature range, it is determined that measurement is impossible (the correction value is set at "0"). Since temperature correction does not greatly vary in a defecation period, the temperature correction is used for whole correction to be applied to all of the defecation period.

As described above, if an excretory act is performed multiple times during one defecation period, the amount of defecation gas itself is large at the first excretory act (the amount of odiferous gas also increases), whereby accuracy of analysis in an early excretory act is higher than that in a later excretory act in the defecation period. Thus, as shown in FIG. 16, in the correction table for frequency of excretory acts, a correction value of the first defecation gas is set at a value more than "1" to increase reliability. Then, that of the second defecation gas is set at "1", and that of the third defecation gas or later is set at a value less than "1", so that the correction values gradually decreases as the number of times increases. In this way, it is devised that the first defecation gas is preferentially to be a diagnosis object. The correction table for frequency of excretory acts is used for correction in only a period in which defecation gas is detected, and thus is used for the partial correction.

As shown in FIG. 12, when the entrance detection sensor 34 detects entrance of a test subject at the time t₁, processing shifts to the step of preparing starting measurement from the step of improving environment before measurement in a standby state so that the control device 22 of the measuring device 6 allows the sensor heater 54 and the suction device 18 to operate. Accordingly, temperature detected by the temperature sensor 32 rises to converge to a proper temperature. Then, the data analyzer 60 of the remote control 8 acquires a correction value corresponding to the convergence temperature measured by the temperature sensor 32 in a non-defecation period before the seating detection sensor 36 detects that a test subject sits on the seat, with reference to the correction table for temperature. In an example shown in FIG. 12, a temperature correction value is set at 0.9.

When the test subject enters the toilet installation room at the time t₁, detection data detected by the odiferous gas sensor 26 increases due to stink noise attached to the test subject, and then converges to a constant value. Subsequently, the seating detection sensor 36 detects that the test subject sits on the seat, at the time t₂. The data analyzer 60 of the remote control 8 acquires a correction value corresponding to detection data measured by the odiferous gas sensor 26 in a non-defecation period before the seating detection sensor 36 detects that the test subject sits on the seat. In the present embodiment, a correction value of noise of stink gas attached to a test subject is 0.7.

Next, if the test subject urinates in a defecation period after the seating detection sensor 36 has detected that the test subject has sat on the seat, at the time t₃, a detection value by the humidity sensor 30 rises. It is preferable that detection of the rise in humidity by the humidity sensor 30 may be performed based on, for example, humidity before a defecation period, or before the seating detection sensor 36 detects that the test subject sits on the seat. If the humidity sensor 30 detects the rise of detection data in this way, the data analyzer 60 acquires a correction value corresponding to the detection data that rises, for a period in which the detection data rises, with reference to the correction table for humidity. In the present embodiment, a partial correction value in a period in which detection data by the humidity sensor 30 rises (or from the time t₃ to the time t₄) is 0.6.

Subsequently, if a test subject performs an excretory act at the time t₅, and the time t₆, to cause a rate of change in difference between detection data detected by the odiferous gas sensor 26 and a reference value to be a predetermined value or more, the data analyzer 60 calculates the amount of gas with the excretory act. Accordingly, the data analyzer 60 acquires the following correction values according to a frequency of excretory acts in the defecation period with reference to a correction table for frequency of excretory acts: a correction value in a period corresponding to the first excretory act (or from time t₅ to time t₅') is 1.5; and a correction value in a period corresponding to the second excretory act (or time t₆ to time t₆') is 1.0.

The data analyzer 60 calculates reliability of measurement of gas detection with each excretory act on the basis of the whole correction value and the partial correction value, estimated in this way. In the present embodiment, reliability is based on 3, and reliability of measurement for each excretory act is calculated as the product of multiplying all corresponding partial correction values by the product of three times all whole correction values. Specifically, reliability of measurement of the first excretory act is acquired as follows: 3 (reference value) x 0.9 (temperature correction value) x 0.7 (test subject attached noise correction value x 1.5 (frequency correction value) = 2.84. Reliability of measurement of the second excretory act is acquired as follows: 3 (reference value) x 0.9 (temperature correction value) x 0.7 (test subject attached noise correction value x 1.0 (frequency correction value) = 1.89.

The reliability calculated in this way is then displayed in the display device 68 of the remote control 8 as described with reference to FIG. 5. In addition, the calculated reliability is transmitted to the server 12 from the device on a test subject side along with detection data of the odiferous gas sensor 26 and detection data of the hydrogen gas sensor 24 to be stored in a defecation gas database in the server 12. At this time, in the defecation gas database in the server 12, raw data, to which no correction by reliability described later is applied, of the detection data of the odiferous gas sensor and the detection data of the hydrogen gas sensor is stored. When measurement data is browsed by the medical facility terminal 16 connected to the server 12, the reliability of measurement is displayed along with the detection data of the odiferous gas sensor 26 and the detection data of the hydrogen gas sensor 24. A doctor at a medical facility performs diagnosis with reference to the reliability of measurement displayed in the medical facility terminal 16 along with the detection data on odiferous gas and hydrogen gas. Accordingly, when the doctor, or the like, performs diagnosis of physical condition of a test subject on the basis of the measurement data, it is possible to perform more accurate diagnosis by using data with high reliability of measurement. The doctor may perform diagnosis without using data with low reliability of measurement, or without attaching importance to it. If reliability of measurement data on a part of a period or all of the period is 1 or less, measurement accuracy is very low. Thus, it may be determined that measurement is impossible, and no measurement data may be transmitted to the server 12.

It is also possible to correct detection data of the odiferous gas sensor 26 and the hydrogen gas sensor 24 on the basis of the reliability of measurement calculated in this way. Specifically, if the reliability of measurement is high, actual detection value is used, however, if the reliability of measurement is low, a detection value is corrected so as to be a value close to a previous detection value. For example, there is description below of a case where a detection value detected additionally is corrected so as to be close to previous measurement data stored in the storage device of the remote control 8 when physical condition is analyzed on the basis of detection data on defecation gas with the first excretory act, in the device 10 on a test subject side. As described above, it is calculated that the reliability with the first excretory act is 2.84.

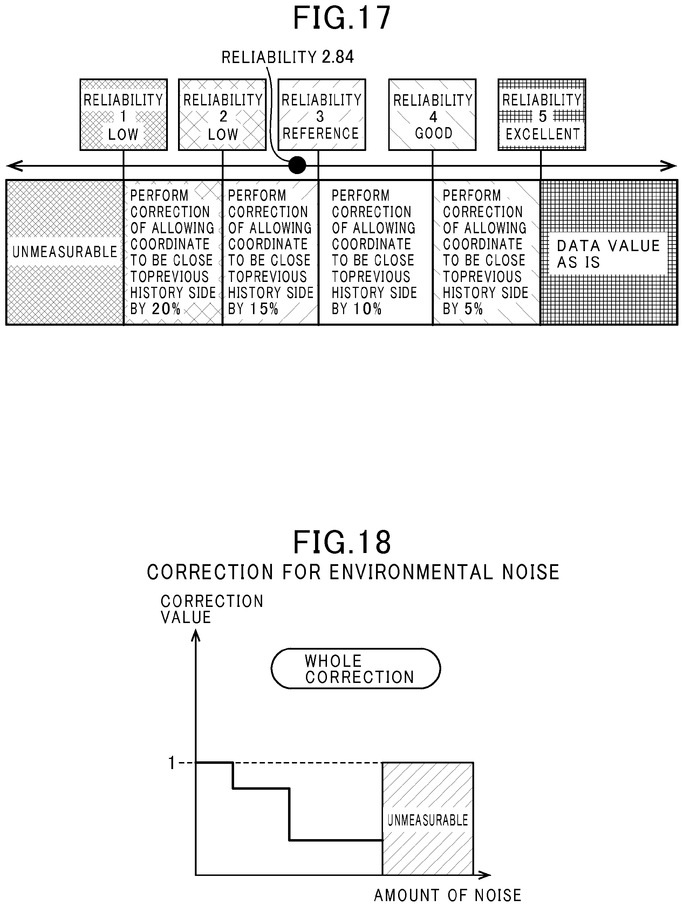

The data analyzer 60 determines the amount of correction of a measurement value on the basis of the reliability calculated in this way. FIG. 17 shows a correction table showing a relationship between reliability recorded in a data analyzer and a correction rate of measurement values. As shown in FIG. 17, for example, in the present embodiment, if reliability is 1 or less, reliability of detection data is too low to use a measurement value. That is, analysis of physical condition based on detection data acquired in a period in which reliability is a predetermined value or less is not performed, and the analysis is performed on the basis of only detection data with reliability more than the predetermined value so that a result of the analysis is displayed in the display device 68. If the reliability is more than 1 and is not more than 2, correction of allowing a measurement value to be close to a previous history side by 20% is performed. If the reliability is more than 2 and is not more than 3, correction of allowing a measurement value to be close to the previous history side by 15% is performed. If the reliability is more than 3 and is not more than 4, correction of allowing a measurement value to be close to the previous history side by 10% is performed. If the reliability is more than 4 and is not more than 5, correction of allowing a measurement value to be close to the previous history side by 5% is performed. In addition, if the reliability is more than 5, a measurement value is used without correction.

In the example described above, the reliability of measurement of the first excretory act is 2.84. Thus, as described with reference to FIG. 7A, correction is performed so that a plotted point of the latest data is close to a previous measurement value by 15% to be displayed along with previous data.

Correction based on this kind of reliability may be performed on the server 12 side. If analysis of physical condition is performed on the server 12 side, for example, a detection value of odiferous gas and a detection value of hydrogen gas in an excretory act in which the reliability is a predetermined value or more in one defecation period is totaled so that analysis of physical condition may be performed on the basis of the totaled data. In addition, it is not always required to apply correction based on reliability of measurement to detection data to be stored in the storage device of the remote control 8, and also detection data after the correction may be stored.

The correction table is not limited to the correction table for noise of stink gas attached to a test subject, the correction table for temperature, and the correction table for humidity, described above. Each of FIGS. 18 to FIG. 29 shows an example of a correction table.

For example, if there is stink noise (environmental noise) other than defecation gas, such as an aromatic, in the toilet installation room, the odiferous gas sensor 26 may detect the stink noise to cause accuracy of measurement to be reduced. Then, the data analyzer 60 corrects reliability to evaluate influence of environmental noise. The amount of this kind of environmental noise can be evaluated on the basis of detection data detected by the odiferous gas sensor 26 before the entrance detection sensor 34 detects entrance of a test subject, for example. FIG. 18 shows a correction table for environmental noise. As shown in FIG. 18, if the amount of environmental noise is less than a predetermined value, a correction value of environmental noise is 1, and as the amount of environmental noise increases more than the predetermined value, the correction value is also reduced to reduce reliability. If the amount of environmental noise is an upper limit value in a measurable noise range or more, it is determined that measurement is impossible. Since the correction value of environmental noise affects throughout a defecation period, the correction value thereof may be used for the whole correction.

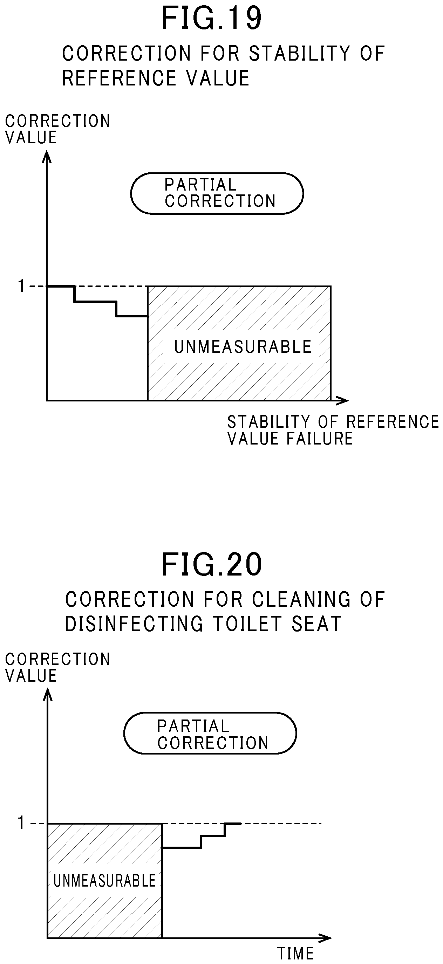

In a case where detection data of the odiferous gas sensor 26 greatly varies when a reference value is set, such as a case where a spray aromatic is used, for example, and in a case where an inclination of a reference value set when the amount of gas is estimated is large, accuracy of the amount of gas estimated decreases. Then, the data analyzer 60 corrects reliability with reference to a correction table for stabilizing a reference value to evaluate influence of this kind of failure condition of stability of a reference value (referred to as stability failure of a reference value). The stability of a reference value can be evaluated on the basis of an inclination with respect to a time axis of the reference value in a non-defecation period, and a fluctuation of a detection value of the odiferous gas sensor 26 when the reference value is set, for example. FIG. 19 shows a correction table for stability of a reference value. As shown in FIG. 19, a correction value of stability noise of a reference value is 1 if stability failure of a reference value is small, and decreases as the stability failure of a reference value increases. If the stability failure of a reference value is a predetermined value or more, it is determined that measurement is impossible. Since the amount of gas is estimated by setting a reference value for each excretory act, the correction value of stability noise of a reference value is used for a correction value of only a period corresponding to each excretory act, or the partial correction.

In a case where the seat is cleaned with a disinfecting sheet, for example, the odiferous gas sensor 26 detects even components, such as alcohol, contained in the disinfecting sheet. Although influence of the components, such as alcohol, contained in the disinfecting sheet, causes the odiferous gas sensor 26 to measure a large value immediately after the disinfecting sheet has been used, a value measured by the odiferous gas sensor 26 decreases for a short time because alcoholic has high volatility. Then, the data analyzer 60 corrects reliability depending on influence of seat disinfection, with reference to a correction table for cleaning of disinfecting toilet seat. Using of a disinfecting sheet can be detected by detecting, for example, a great variation of detection data of the odiferous gas sensor 26 from a predetermined value after the entrance detection sensor 34 has detected entrance of a test subject, and before the seating detection sensor 36 detects that the test subject sits on the seat. FIG. 20 shows a correction table for cleaning of disinfecting toilet seat. If using of a disinfecting sheet is detected in this way, it is determined that measurement is impossible in a predetermined period after detection of the disinfecting sheet (a correction value is set at 0), and a correction value in a period after the predetermined period increases from a value less than 1 to 1, as time elapses. Since influence of a disinfecting sheet changes as time elapses, as described above, the correction value is used for the partial correction.

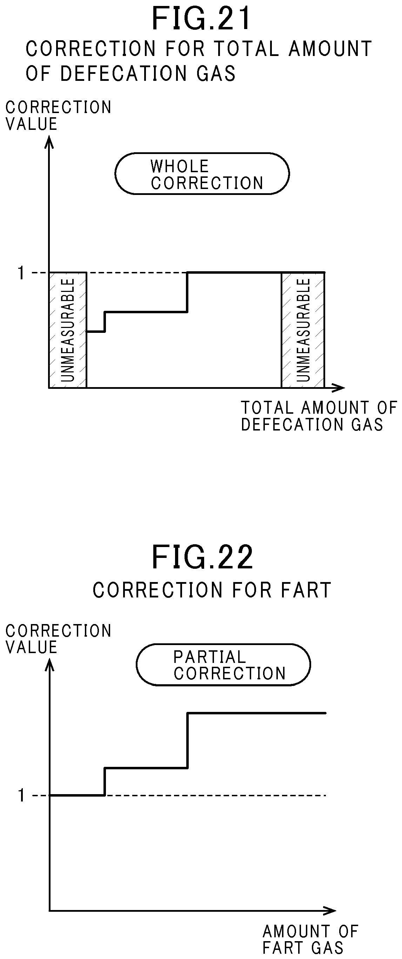

Since a trace amount of odiferous gas is contained in defecation gas, analysis of physical condition can be more accurately performed with increase of odiferous gas discharged in a defecation period. Then, the data analyzer 60 corrects reliability on the basis of a total amount of odiferous gas, with reference to a correction value table for a total amount of defecation gas. The total amount of defecation gas can be evaluated from a total of the amount of gas estimated on the basis of detection data of the odiferous gas sensor in a defecation period. FIG. 21 shows a correction value table for a total amount of defecation gas. As shown in FIG. 21, if a total amount of defecation gas is a predetermined value or more, it is determined that measurement is impossible because some kind of problem, such as that an aromatic is sprayed during measurement, occurs, so that a correction value of a total amount of defecation gas is set at 0, and if the total amount of defecation gas is a predetermined value or less, it is determined that measurement is impossible because the amount of defecation gas is too low to perform accurate measurement, so that the correction value of a total amount of defecation gas is set at 0. In a range in which it is not determined that measurement is impossible (the correction value is 0), if a total amount of defecation gas is large, the correction value is set at 1, and as the total amount of defecation gas decreases, the correction value decreases. Since a correction value is set on the basis of a total amount of defecation gas throughout a defecation period in correction of a total amount of defecation gas, the correction is used for the whole correction.

When a fart occurs, a large amount of defecation gas is discharged into a bowl as compared with that during defecation, so that defecation gas by a fart is suitable for analysis of physical condition. Thus, if a fart from a test subject is detected, the data analyzer 60 corrects reliability during the fart on the basis of the amount of defecation gas contained in the fart, with reference to a correction value table for a fart. With respect to a fart act, it is possible to determined that a fart act is performed when it is detected that a difference between a detection value of the odiferous gas sensor 26 and a reference value steeply rises at a rate of change of a predetermined value or more after the seating detection sensor 36 has detected that a test subject has sat on the seat. In addition, a period from a time point, from which the difference described above steeply rises, until a detection value of the gas sensor 26 returns to the reference value again, may be set as a fart period. In order to more accurately detect that a fart act is performed, it is required to detect that detection data of the odiferous gas sensor 26 steeply rises at a rate of change of the predetermined value or more, and to allow a seal-water-amount sensor, or the like, to detect that no stool is discharged into the bowl. FIG. 22 shows the correction value table for a fart. As shown in FIG. 22, in the correction value table for a fart, if the amount of fart gas (the amount of defecation gas detected by the odiferous gas sensor) is small, a correction value may be set at 1, and may be set so as to increase with increase of the amount of fart gas.

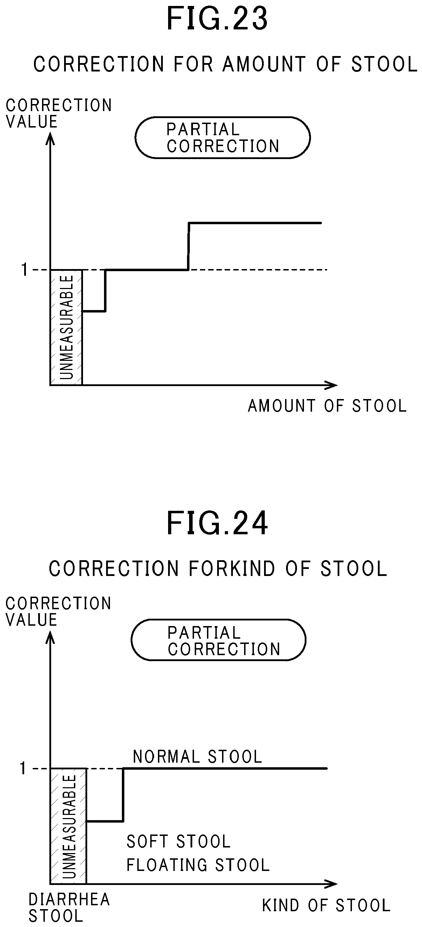

If there are a large amount of stool in each excretory act, the amount of defecation gas increases to enable analysis of physical condition to be more accurately performed, however, if there a little amount of stool in the each excretory act, the amount of defecation gas decreases to reduce accuracy of the analysis of physical condition. Thus, the data analyzer 60 corrects reliability on the basis of the amount of stool during the each excretory act, with reference to a correction value table for the amount of stool. The amount of stool can be evaluated by a seal-water-amount sensor (device of measuring the amount of stool) for detecting change in the amount of seal water, in the defecation/urination detection sensor 38, for example. FIG. 23 shows the correction value table for the amount of stool. As shown in FIG. 23, if the amount of stool is a predetermined value or less, it is determined that measurement is impossible, because the amount of defecation gas as well as the amount of stool is very low so that it is impossible to perform accurate analysis. If the amount of stool exceeds the predetermined value, as the amount of stool increases, a correction value increases stepwise from a value less than 1 to a value more than 1. Since the amount of stool is determined for each excretory act, a correction value of the amount of stool is used for the partial correction.

For example, if stool is a diarrhea state, discharge time is too short to allow a sensor to sufficiently detect defecation gas. In addition, if stool after defecation floats in seal water, defecation gas is discharged from the stool floating in the seal water to deteriorate detection accuracy of defecation gas. In addition, if stool is a diarrhea state, a large amount of acetic acid gas, which is impossible in normal stool, is discharged as defecation gas. As a result, it is impossible to perform measurement of physical condition based on the amount of short-chain fatty acid gas. Thus, diarrhea determination means (circuit) of a program installed in the data analyzer 60 corrects reliability depending on a kind of stool of each excretory act, with reference to a correction table of a kind of stool. The kind of stool can be detected on the basis of detection results acquired by using a CCD, a microwave sensor, or the like, of the defecation/urination detection sensor 38, as a stool state detector. Alternatively, it is also possible to determine diarrhea on the basis of detection data on acetic acid gas because a very large amount of acetic acid gas is discharged at the time of diarrhea. In addition, providing a CCD, a microwave sensor, or the like, in the bowl, as a floating detector, enables floating of stool to be detected. FIG. 24 shows a correction value table for a kind of stool. As shown in FIG. 24, if there is diarrhea stool, the diarrhea determination means determines that measurement is impossible (a correction value is set at 0). If floating stool is detected, a correction value in the following excretory act is set less than 1, and if normal stool is detected, the correction value is set at 1. Since a kind of stool is determined for each excretory act, a correction value of a kind of stool is used for the partial correction. In this way, if the diarrhea determination means determines diarrhea of a test subject, detection data on the diarrhea is not used for analysis of physical condition (measurement is impossible), or weighting of the detection data is reduced (a correction value is set less than 1).

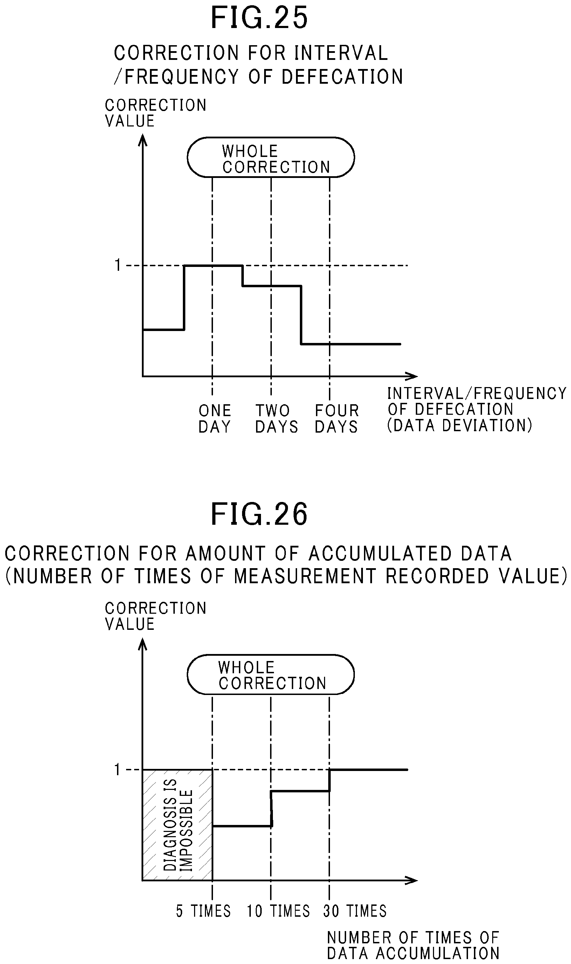

Usually, healthy people have defecation about once every day. In contrast, if gastrointestinal condition becomes worse due to food poisoning, or the like, defecation may be performed several times in a day. In this case, even if defecation is performed, the amount of defecation gas discharged during the defecation is also small. In addition, if frequency of defecation is low due to obstipation, or the like, the amount of defecation gas increases due to increase in creation time of odor components, or increase in the amount of stool. If an interval of defecation increases too much, accuracy of analysis of physical condition is deteriorated. Then, the data analyzer 60 corrects reliability on the basis of an interval of defecation, with reference to a correction table for an interval of defecation. The interval of defecation can be determined on the basis of a date and time of the previous defecation stored in the data analyzer 60, and the defecation history information inputted in step S2 of preparing starting measurement. FIG. 25 shows a correction value table for an interval of defecation. As shown in FIG. 25, a correction value is set as follows: if an interval of defecation is too short, a correction value is set greatly less than 1; if the interval of defecation is about a day, the correction value is set at 1; if the interval of defecation is about two days, the correction value is set less than 1; and if the interval of defecation is four days or more, the correction value is set greatly less than 1. The correction value of an interval of defecation is used for the whole correction.

In determination of physical condition based on defecation gas, if gastrointestinal condition becomes worse due to crapulence of the previous day, or the like, for example, a state of physical condition is determined to be worse than a state of actual physical condition. Thus, a result of analysis of physical condition varies depending on daily living. Accordingly, for example, if a day with bad physical condition due to crapulence, or the like, expectedly continues when analysis of physical condition by the biological information measurement system of the present embodiment is started, only an analysis result of bad physical condition is displayed even if history of the physical condition is displayed. As a result, there is a possibility that a medical facility, or the like, cannot perform accurate determination of disease. Then, the data analyzer 60 corrects reliability depending on the number of previous measurement data items stored in the device on a test subject side, with reference to a correction table for the amount of accumulated data. FIG. 26 shows the correction table for the amount of accumulated data. As shown in FIG. 26, a correction value is set as follows: if the number of data accumulation times is less than five, it is determined that diagnosis is impossible (a correction value is set at 0); if the number of data accumulation times is five or more and less than ten, the correction value is set greatly less than 1; if the number of data accumulation times is ten or more and less than thirty, the correction value is set less than 1; and if the number of data accumulation times is thirty or more, the correction value is set at 1. The device on a test subject side of the present embodiment is not a device for diagnosing cancer, but a device that intends to allow a test subject to recognize that a risk of cancer increases with change in physical condition, and to allow the test subject to improve his or her living. Thus, the present device does not have high accuracy of one measurement, but has value in history of change in measurement, whereby it is desirable to perform this kind of correction to prevent an unnecessary mental burden.

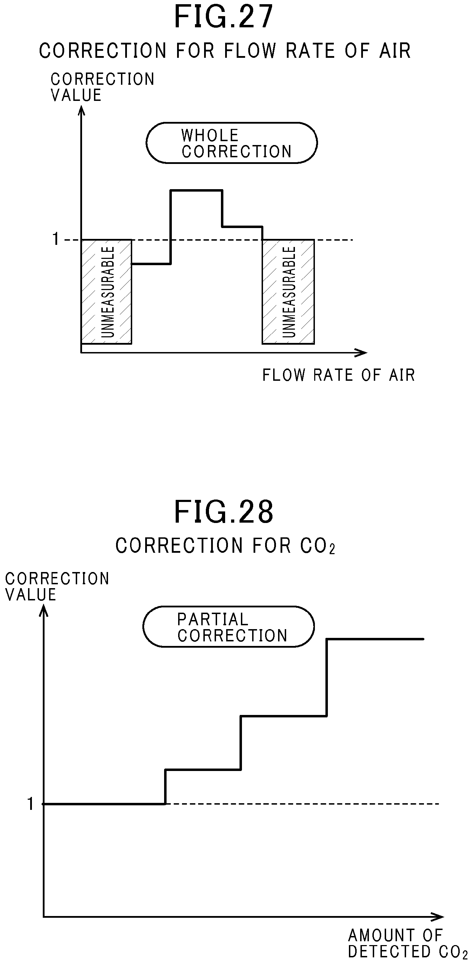

If the filter 72 provided in the duct 18a is clogged, a flow rate of air sucked into the duct 18a is reduced. For this reason, if a flow rate of gas to be fed to the odiferous gas sensor 26 and the hydrogen gas sensor 24 varies, detection data of the odiferous gas sensor 26 and the hydrogen gas sensor 24 may vary depending on the flow rate. In addition, if velocity of gas to be fed to the odiferous gas sensor 26 and the hydrogen gas sensor 24 is high, a period in which the gas is in contact with the sensors is so short that a detecting portion of each of the sensors does not sufficiently react to the gas. Thus, it is desirable that a flow rate of air fed to the odiferous gas sensor 26 and the hydrogen gas sensor 24 is constant. Then, the data analyzer 60 corrects reliability depending on a flow rate of gas (velocity of gas) to be fed to the odiferous gas sensor 26 and the hydrogen gas sensor 24, with reference to a correction value table for a flow rate of air. The flow rate of gas can be estimated on the basis of electric current and voltage, applied to the suction fan 18c provided in a deodorizing device, for example. FIG. 27 shows a correction value table for a flow rate of air. As shown in FIG. 27, in the correction value table for a flow rate of air, a correction value is set as follows: if a flow rate of air is less than a lower limit value in a measurable range of a flow rate of air and an upper limit value therein or more, it is determined measurement is impossible (a correction value is set at 0): if the flow rate of air is within an optimum range, the correction value is set more than 1; and if the flow rate of air is within the measurable range other than the optimum range, the correction value is set at a value close to 1. In the present embodiment, influence of decrease in a flow rate of air caused by clogging on detection sensitivity of a sensor is more than influence of a case where a flow rate of air is high thereon, so that a correction value within a range higher than the optimum range within the measurable range is set higher than a correction value within a range lower than the optimum range. Since the flow rate of air does not greatly vary during measurement, the correction value is used for the whole correction.

Defecation gas contains CO₂ gas as well as hydrogen gas, as healthy-state gas. Thus, if a CO₂ gas sensor detects a large amount of CO₂, it means that a sensor device reliably detects defecation gas. Then, the data analyzer 60 corrects reliability on the basis of detection data on CO₂ detected by the carbon dioxide sensor 28, with reference to a correction table for CO₂. FIG. 28 shows a correction table for CO₂. As shown in FIG. 28, in the correction table for CO₂, if the amount of detected CO₂ is less than a predetermined value, a correction value is set at 1, and if the amount of detected CO₂ is the predetermined value or more, the correction value increases with increase in the amount of detected CO₂. Since a correction value of CO₂ can be calculated for each excretory act, the correction value of CO₂ is used for the partial correction. In this way, detected hydrogen gas is corrected on the basis of the amount of CO₂ gas in the present embodiment, so that healthy-state gas is evaluated by using hydrogen gas and CO₂ gas.

In a case where analysis of physical condition is performed by using detection data of the hydrogen gas sensor as detection data on healthy-state gas, a correction table for H₂ that is set so that a correction value increases with increase in a detection value detected by the hydrogen gas sensor 24 may be used instead of the correction table for CO₂.

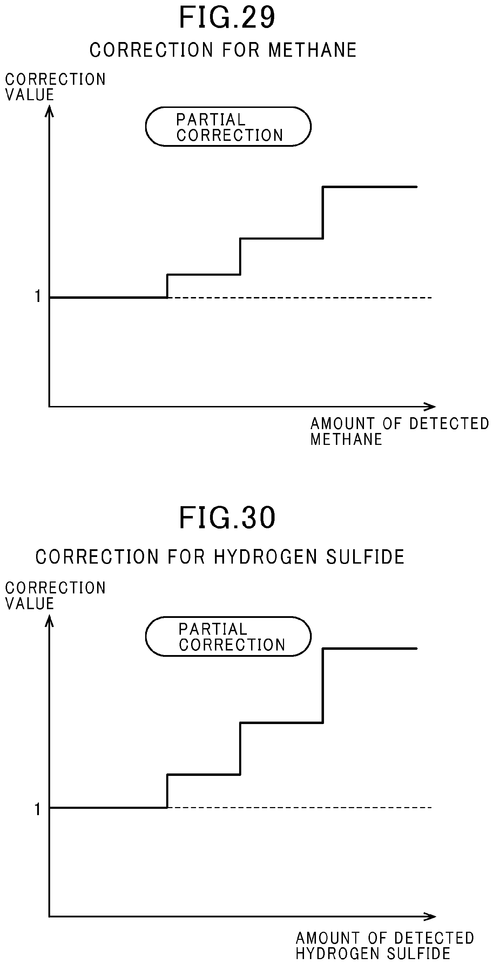

Defecation gas contains methane as well as hydrogen gas, as healthy-state gas. Thus, a methane gas sensor that is strongly sensitive to methane gas is provided in the duct 18a of the deodorizing device, for example, and if the methane gas sensor detects a large amount of methane, it means that a large amount of defecation gas is discharged. Then, the data analyzer 60 corrects reliability on the basis of the amount of methane gas detected by the methane gas sensor, with reference to a correction table for methane gas. FIG. 29 shows a correction table for methane gas. As shown in FIG. 29, in the correction table for methane gas, if the amount of detected methane gas is less than a predetermined value, a correction value is set at 1, and if the amount of detected methane gas is the predetermined value or more, the correction value increases with increase in the amount of detected methane gas. Since a correction value of methane gas can be calculated for each excretory act, the correction value of methane gas is used for the partial correction.

In the present embodiment, although reliability is corrected to be set high if a detection value of each of CO₂ and methane is high, besides this, it is also possible to perform correction so that a detection value of hydrogen gas increases if a detection value of each of CO₂ and methane is high.

If there is cancer in the intestines, hydrogen sulfide gas as well as odiferous gas is contained in defecation gas. Thus, a hydrogen sulfide gas sensor that is strongly sensitive to hydrogen sulfide gas is provided in the duct 18a of the deodorizing device, for example, and reliability is corrected on the basis of detection data on hydrogen sulfide gas detected by the hydrogen sulfide gas sensor. FIG. 30 shows a correction table for hydrogen sulfide gas. As shown in FIG. 30, in the correction table for hydrogen sulfide gas, if the amount of detected hydrogen sulfide gas is less than a predetermined value, a correction value is set at 1, and if the amount of detected hydrogen sulfide gas is the predetermined value or more, the correction value increases with increase in the amount of detected hydrogen sulfide gas. Since a correction value of hydrogen sulfide gas can be calculated for each excretory act, the correction value of hydrogen sulfide gas is used for the partial correction. Reliability is calculated by using a part or all of the correction tables described above.

Next, since detailed description related to a method of estimating the amount of gas is omitted in the example described with reference to FIG. 9, it is described here.

A semiconductor gas sensor, or a solid electrolyte sensor, is used as the odiferous gas sensor 26 for measuring odiferous gas. The gas sensors, such as the semiconductor gas sensor, the solid electrolyte sensor, and the hydrogen gas sensor, react not only to odiferous gas but also to alcohol contained in an aromatic, and a disinfecting sheet.

That is, even when a test subject is absent, detection data of the gas sensors includes environmental noise due to influence of an aromatic, and remaining stool attached to a bowl of a toilet, for example. This kind of influence of an aromatic, and remaining stool attached to a bowl of a toilet, does not vary with time.

When a test subject enters a toilet space, a detection value acquired by each of the gas sensors gradually increases due to influence of a body odor of a test subject, and stink gas components attached to a body and clothes of the test subject, such as perfume and hair liquid used by the test subject, and when the test subject sits on the seat, a portion above the bowl is covered with the test subject and the clothes, so that a data value detected by each of the gas sensors becomes stable, or gradually increases.

If a test subject cleans a seat with a disinfecting sheet, the amount of gas measured by a semiconductor gas sensor rapidly increases at the moment when the disinfecting sheet is used, and after the test subject sits on the seat, or after a while when the disinfecting sheet is used, a detection value measured by the gas sensor does not increase due to influence of the disinfecting sheet.

That is, after the test subject sits on the seat, a detection value of the gas sensor may gradually increase due to influence of stink gas attached to the body of the test subject, but does not rapidly increase.

In contrast, once the test subject starts an excretory act, the gas sensor reacts to odiferous gas and hydrogen gas, contained in defecation gas, at the time when each excretory act is performed, so that a detection value of the gas sensor rapidly increases to a peak, and then decreases.

Thus, the present inventors think that there is no sudden increase of a detection value of a gas sensor after a test subject sits on a seat, and that if the detection value is set as a reference value, odiferous gas and hydrogen gas, contained in defecation gas can be detected as a sudden increase from the reference value.

Then, in the present embodiment, as described with reference to FIG. 9, the data analyzer 60 sets a detection value of the gas sensor as a reference value, the detection value being acquired during a period of a non-excretory act after time t₂ at which the seating detection sensor 36 detects that a test subject sits on the seat 4, and before time t₅ at which an excretory act is started. Next, the data analyzer 60 sets a time point at which a rate of change in a difference between a detection value of the gas sensor and the reference value becomes a positive predetermined value or more as the time t₅ of a starting point of the excretory act. Then, the data analyzer 60 integrates with time a difference between a detection value of the gas sensor and the reference value during the excretory act from the starting point to a finishing point of the excretory act (or acquires an area of a portion in which a detection value is more than the reference value of the amount of gas during the excretory act), and estimates the integrated value as the amount of defecation gas. A finishing point of an excretory act may be set at a time point at which a detection value of the gas sensor returns to a reference value again, or at a time point at which a rate of change in a difference between a detection value of the gas sensor and the reference value changes from a positive value to a negative value after a starting point.

As with the odiferous gas sensor 26, the hydrogen gas sensor 24 and the carbon dioxide sensor 28 may be affected by stink noise other than defecation gas. Thus, also when the amount of hydrogen gas and carbon dioxide gas is estimated on the basis of detection data of the hydrogen gas sensor 24 and the carbon dioxide sensor 28, the estimate may be performed as with the estimate of defecation gas.

A method of estimating the amount of gas is not limited to the method described above. Then, a method of estimating the amount of gas in a biological information measurement system of a second embodiment will be described below. The second embodiment is only different in the method of estimating the amount of gas as compared with the first embodiment.

Also in the system of the present embodiment, as with the first embodiment, a semiconductor gas sensor or a solid electrolyte sensor is used as the odiferous gas sensor 26 for measuring odiferous gas. Since the semiconductor gas sensor or the solid electrolyte sensor measures the amount of gas by detecting a reaction of a detecting portion heated, its sensitivity is low. The hydrogen gas sensor 24 also has a low sensitivity as with the semiconductor gas sensor. In a case where this kind of gas sensor with a low sensitivity is used, there is the following problem. The following problem is not unique to the semiconductor gas sensor, and the same applies to the solid electrolyte sensor and the hydrogen gas sensor.

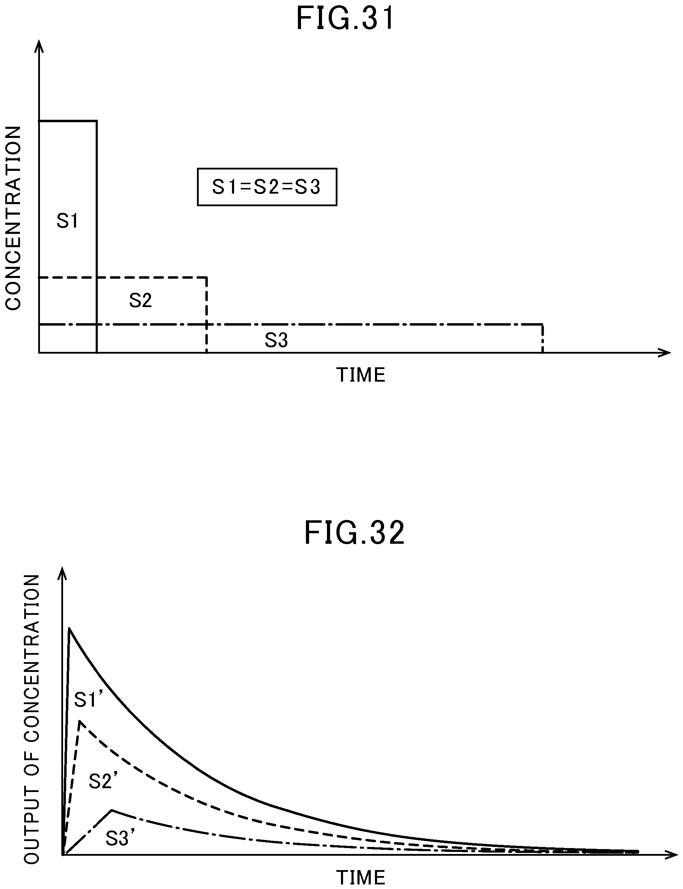

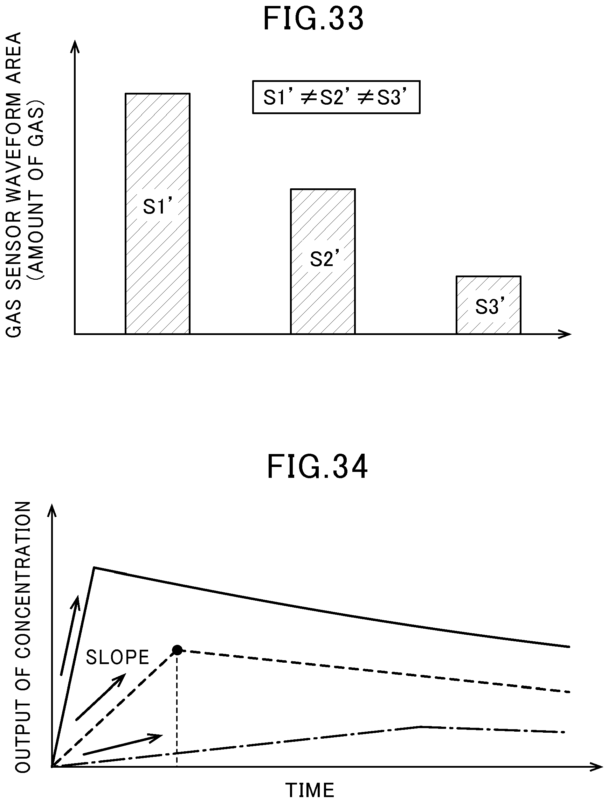

For example, as shown in FIG. 31, there is considered a case where the semiconductor gas sensor is used as the odiferous gas sensor 26 to detect odiferous gas for each of the conditions S1, S2, and S3, in which a total amount of discharged defecation gas is identical, but a discharge time and a discharge rate per unit time are different. FIG. 32 shows detection waveforms of a gas sensor in a case where a discharge time as well as a discharge rate per unit time is changed, and FIG. 33 shows the amount of gas calculated on the basis of the detection waveforms of the gas sensor. FIGS. 32 and 33 show S1', S2', and S3', which corresponds to S1, S2, and S3, shown in FIG. 31, respectively.

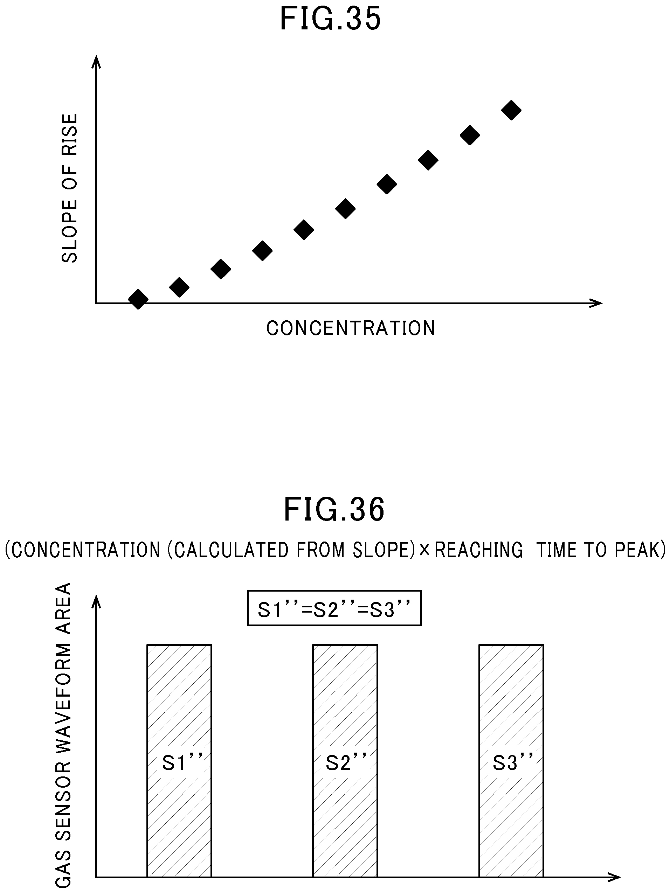

As shown in FIG. 32, if a total amount of discharged defecation gas is identical but a discharge time is different, a waveform of discharged gas needs to converge in a similar time due to a time constant of the gas sensor. Thus, the present inventors focus on an inclination of the waveform of discharged gas when the gas is discharged. FIG. 34 shows initial portions of the detection waveforms of the gas sensor shown in FIG. 32 by being enlarged in a time axis. As shown in FIG. 34, if a discharge rate per unit time (discharge concentration) is different, an inclination of the waveform from a start of discharge to a peak value, and a time by which the discharge rate reaches a peak value, are different. Then, as the discharge rate per unit time (discharge concentration) increases, an inclination of the waveform to a peak value increases, and as a gas discharge time increases, a reaching time to a peak value increases. In addition, FIG. 35 is a graph showing a relationship between a discharge rate per unit time (discharge concentration) and an inclination of a rising edge of each of the waveforms of detection data acquired by the sensor. As shown in FIG. 35, it can be said that there is a substantially proportional relationship between the discharge rate per unit time (discharge concentration) and the inclination of a rising edge of the waveform of detection data acquired by the semiconductor gas sensor.

On the basis of findings described above that an inclination of a detection waveform acquired by the semiconductor gas sensor is associated with a discharge rate of discharged gas per unit time (discharge concentration), and a reaching time to a peak of the detection waveform acquired by the semiconductor gas sensor is associated with a discharge time, the present inventors estimate the amount of gas on the basis of the product (gas sensor waveform area) of the inclination of the detection waveform acquired by the semiconductor gas sensor and the reaching time to the peak of the detection waveform. FIG. 36 shows the amount of gas estimated in this way on the basis of the product (gas sensor waveform area) of an inclination of a detection waveform acquired by a semiconductor gas sensor, and a reaching time to a peak, for each of the conditions S1, S2, and S3, in which a discharge time as well as a discharge rate per unit time (discharge concentration) is different. As shown in FIG. 36, the amounts of gas S1", S2", and S3" estimated on the basis of the product of the inclination of the waveform of the amount of gas and the reaching time to the peak are the same amount, so that it is found that the amount of gas can be accurately estimated on the basis of an inclination of a waveform of the amount of gas and a reaching time to a peak of the waveform.

Thus, in the present embodiment, as with the first embodiment described above, a reference value is set on the basis of detection data acquired by the odiferous gas sensor 26 in a period after a time point at which the seating detection sensor 36 detects that a test subject sits on a seat, and before an excretory act is started. Then, as shown in FIG. 10A, a time point, at which a rate of change in a difference between a detection value measured by the odiferous gas sensor 26 and a reference value exceeds a preset threshold value of a start, is set as a starting point of estimating the amount of defecation gas (or a starting point of an excretory act). Next, as shown in FIG. 10A, a time point, at which the rate of change in a difference between the detection data acquired by the odiferous gas sensor 26 and the reference value becomes negative (or a time point at which the detection data of the odiferous gas sensor 26 becomes a peak), is set as a finishing point of estimating the amount of defecation gas (or a finishing point of the excretory act).

Subsequently, the data analyzer 60 calculates a rate of change in a difference between detection data and the reference value, from the starting point to the finishing point of the excretory act. The data analyzer 60 also calculates a discharge time of defecation gas from the starting point to the finishing point of the excretory act. Then, the data analyzer 60 integrates the rate of change in a difference between detection data acquired from the starting point to the finishing point of the excretory act and the reference value, and the discharge time of defecation gas, and then estimates the integrated value as the amount of gas. Likewise, an estimation of the amount of hydrogen gas based on detection data of the hydrogen gas sensor 24, as well as an estimation of the amount of carbon dioxide gas based on detection data of the carbon dioxide sensor 28, can be performed. According to the method of estimating the amount of gas, described above, it is possible to more accurately estimate the amount of defecation gas without influence of a time constant of the gas sensor.

In addition, a study by the present inventors on a relationship between a discharge rate of defecation gas per unit time and a discharge time reveals that there is a little personal difference in a relationship between a discharge rate and a discharge time. That is, if a discharge rate of defecation gas per unit time is large, a discharge time becomes a certain relatively short time regardless of a test subject, and if the discharge rate of discharged defecation gas per unit time is small, a discharge time becomes a certain long time regardless of a test subject. Thus, the present inventors think that a discharge time of defecation gas (odiferous gas) can be estimated on the basis of a discharge rate per unit time of odiferous gas in defecation gas (a rate of change in a detection value acquired by the odiferous gas sensor 26). Likewise, a discharge time of defecation gas (hydrogen gas and carbon dioxide) can be estimated on the basis of a discharge rate per unit time of each of hydrogen gas and carbon dioxide (a rate of change in a detection value acquired by each of the hydrogen gas sensor 24 and the carbon dioxide sensor 28). In the present embodiment, although an area is estimated so that a correlation between the amount of healthy-state gas and the amount of odiferous gas is acquired, there is a correlation only between concentration of healthy-state gas and concentration of odiferous gas so that a similar result can be acquired, whereby it may be configured to acquire concentration from an inclination of a waveform of measurement values of each sensor. In this case, eliminating an estimation of an area enables measurement to be further simplified.

Then, a method of estimating the amount of gas in a biological information measurement system of a third embodiment, based on the findings described above, will be described below. The third embodiment is only different in the method of estimating the amount of gas as compared with the first and second embodiments. In the data analyzer 60, data on a rate of change versus a discharge period, related to a correspondence between a rate of change in a difference, and a discharge time of gas, is set in addition to a threshold value of a start of a rate of change in a difference, described in the embodiments above.

A reference value is set on the basis of detection data acquired by the odiferous gas sensor 26 in a period after a time point at which the seating detection sensor 36 detects that a test subject sits on a seat, and before an excretory act is started. Then, a time point, at which a rate of change in a difference between a detection value measured by the odiferous gas sensor 26 and a reference value exceeds a preset threshold value of a start, is set as a starting point of estimating the amount of defecation gas (or a starting point of an excretory act). Subsequently, the data analyzer 60 acquires data on a discharge period corresponding to a rate of change in a difference between a detection value at the starting point and the reference value, with reference to the data on a rate of change versus a discharge period. Then, the data analyzer 60 integrates the rate of change in a difference between detection data acquired at the starting point of the excretory act and the reference value, and the discharge time, and then estimates the integrated value as the amount of gas. Likewise, an estimation of the amount of hydrogen gas based on detection data of the hydrogen gas sensor 24, as well as an estimation of the amount of carbon dioxide gas based on detection data of the carbon dioxide sensor 28, can be performed. It is also possible to more accurately estimate the amount of defecation gas without influence of a time constant of the gas sensor, in accordance with the method of estimating the amount of gas, described above. While a case of using a semiconductor gas sensor as the odiferous gas sensor 26 is described in the method of estimating the amount of gas of each of the embodiments, a case of using a solid electrolyte sensor instead of the semiconductor gas sensor enables the amount of gas to be estimated. In the embodiments described above, while the data analyzer 60 acquires a rate of change in a difference between a detection value at the starting point and the reference value to acquire data on a discharge period corresponding to the rate of change in the difference, with reference to the data on a rate of change versus a discharge period, and then estimates the amount of gas on the basis of the rate of change and the discharge period, the present invention is not limited to the way above. For example, data on a rate of change versus the amount of gas, in which a rate of change in the difference and the amount of gas are associated with each other, may be previously stored so that a rate of change in the difference is acquired to directly estimate the amount of gas with reference to the data on a rate of change versus the amount of gas.

In the biological information measurement system of the first embodiment described with reference to FIG. 1, although it is described that the measuring device 6 is assembled inside the seat 4 mounted on the flush toilet 2 installed in the toilet installation room R, the measuring device is not required to be always assembled inside the seat in the biological information measurement system of the present invention.

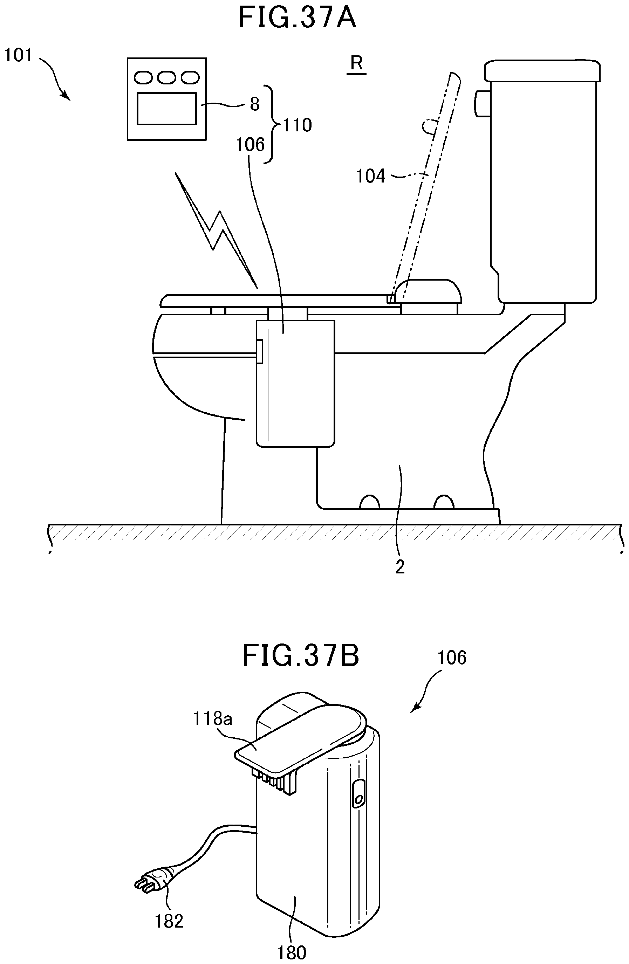

FIG. 37A shows a state in which a device on a test subject side of a biological information measurement system in accordance with a fourth embodiment is attached to a flush toilet installed in a toilet installation room, and FIG. 37B is a perspective view showing a measuring device of the device on a test subject side shown in FIG. 37A. The fourth embodiment is only different in a configuration of the device on a test subject side as compared with the first embodiment. As shown in FIG. 37A, a biological information measurement system 101 of the present embodiment has the same configuration as that of the first embodiment, except that only a measuring device 106 of a device 110 on a test subject side is different. The measuring device 106 of the present embodiment is provided separately from a seat 104.

As shown in FIG. 37B, the measuring device 106 includes a device body 180, a duct 118a that is attached on a top face of the device body 180 so as to extend in a traverse direction, and that is provided with an edge portion bent downward, and a power source code 182 that is connected to the device body 180. As shown in FIG. 37A, the measuring device 106 is fixed while an end of the duct 118a is positioned in the bowl by hanging the edge portion of the duct 118a on a sidewall of a bowl of the flush toilet 2.

The device body 180, as with the first embodiment, includes a hydrogen gas sensor, an odiferous gas sensor, a carbon dioxide sensor, a humidity sensor, a temperature sensor, an entrance detection sensor, a seating detection sensor, a defecation/urination detection sensor, a suction device, a sensor heater, and a transmitter-receiver. Gas sucked through the duct 118a is deodorized and is discharged through a deodorized air outlet provided in a bottom face of the device body 180. In the duct 118a, there are provided the hydrogen gas sensor, the odiferous gas sensor, the carbon dioxide sensor, the humidity sensor, the temperature sensor, the sensor heater, and a fan. Arrangement of the sensors in the duct 118a is the same as that of the first embodiment, so that description thereof is omitted. According to this kind of configuration, the measuring device 106 of the present embodiment is also capable of acquiring detection data corresponding to the amount of odiferous gas, hydrogen gas, and carbon dioxide, contained in defecation gas, by using the odiferous gas sensor, the hydrogen gas sensor, and the carbon dioxide sensor.

It is desirable that the seat 104 to be used along with the measuring device 106 of the present embodiment is a seat with a cleaning function that includes a toilet lid opening/closing device, a nozzle driving device, a nozzle cleaning device, a toilet cleaning device, and a toilet disinfection device, the seat being capable of communicating with the measuring device 106. Using the measuring device 106 along with this kind of seat enables various cleaning operations and disinfecting operation to be performed when stink gas is detected.

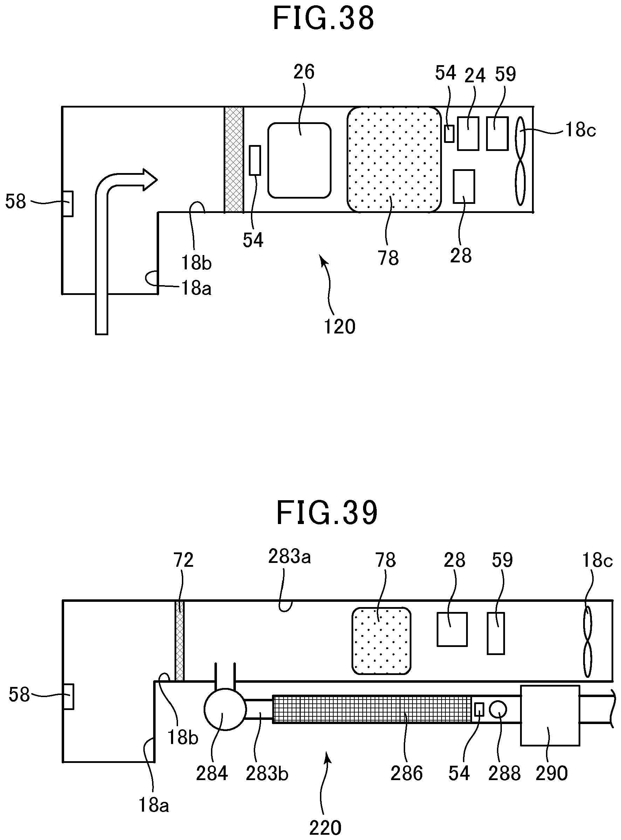

In the first embodiment, as shown in FIG. 3, although the gas detector 20 is configured so that the hydrogen gas sensor 24 is provided upstream of the deodorant filter 78, this kind of configuration is not always required. FIG. 38 shows a configuration of a gas detector provided in a biological information measurement system of a fifth embodiment. The fifth embodiment is only different in a configuration of the gas detector as compared with the first embodiment. As shown in FIG. 39, arrangement of the hydrogen gas sensor 24 in the gas detector 120 in the present embodiment is different from that in the embodiment shown in FIG. 3. In the present embodiment, the hydrogen gas sensor 24 is provided downstream of the deodorant filter 78 in the air intake passage 18b. According to this kind of configuration, even if a sensor sensitive to odiferous gas as well as to hydrogen gas is used as the hydrogen gas sensor 24, it is possible to remove influence of odiferous gas from data to be outputted from the hydrogen gas sensor 24.

In the first embodiment, although a detection value of odiferous gas is calculated by subtracting a detection value acquired by the hydrogen gas sensor 24 from a detection value acquired by the odiferous gas sensor 26 to separate influence of hydrogen gas, the present invention is not limited to the way above. For example, as described below, influence of hydrogen gas can be also separated by varying a reaching time of each of hydrogen gas and odiferous gas to the odiferous gas sensor 26.

FIG. 39 shows a structure of a gas detector of a sixth embodiment of the present invention. While the first embodiment of the present invention allows respective gas sensors to directly detect hydrogen gas and odiferous gas, the present embodiment allows a single gas sensor to detect short-chain fatty acid gas in addition to hydrogen gas and odiferous gas while gas components is separated through a column. The sixth embodiment is only different in the configuration of a gas detector as compared with the first embodiment. As shown in FIG. 39, in the present embodiment, there is provided a branch passage 283b that branches from a main passage 283a of the air intake passage 18b in the duct 18a. While a hydrogen gas sensor and an odiferous gas sensor are separately provided in the first embodiment, the present embodiment is configured to detect hydrogen gas, odiferous gas, and short-chain fatty acid gas, by using one semiconductor gas sensor.

Here, a measurement principle of physical condition in the biological information measurement system of the present embodiment will be described.

As described above, since gas of short-chain fatty acid is created only in a case where pH in intestine is low and an intestinal environment is good, it is possible to reliably determine that an intestinal environment is good by detecting the gas. In this way, in a good intestinal environment, bad bacteria, which create deleterious components inducing disease such as colorectal cancer, tend to be difficult to survive, so that it can be said that a test subject having a good intestinal environment with a low pH value is in a condition where resistance to serious disease, such as colorectal cancer, or immune strength, is high. In contrast, in an intestinal environment in which there are many bad bacteria, deleterious components created by the bad colon bacilli are likely to induce disease such as colorectal cancer to increase a risk of serious disease.

Here, defecation gas discharged during defecation includes nitrogen, oxygen, argon, water vapor, carbon dioxide, hydrogen, methane, acetic acid, trimethylamine, ammonia, propionic acid, butyric acid, methyl disulfide, methyl trisulfide, and the like, along with hydrogen sulfide and methyl mercaptan. The defecation gas includes short-chain fatty acids created by good bacteria in intestine, such as acetic acid, propionic acid, and butyric acid. Most of the short-chain fatty acids are absorbed in the large intestine, so that a slight amount of the short-chain fatty acids remains in stool. Then, the present inventors find that a part of a slight amount of the short-chain fatty acids remaining without being absorbed is vaporized to be contained in defecation gas. The biological information measurement system 1 of the present embodiment succeeds in detecting a very trace amount of vaporized short-chain fatty acid contained in defecation gas, as well as in measuring health condition in intestine of a test subject, and immune strength of the test subject, against disease of the large intestine, on the basis of the detected amount of short-chain fatty acid. The present embodiment, as described later, guides a part of defecation gas into a tube called a column so that acetic acid gas and propionic acid gas of short-chain fatty acid is separated from another gas on the basis of a difference in time in which each gas component passes through the column to be detected.

As described above, although acetic acid of short-chain fatty acid exists in defecation gas when physical condition is good, the present inventors find that there is a very large amount of discharge of acetic acid when a test subject has diarrhea. That is, if a test subject has diarrhea, digested material fed into the large intestine stays in the large intestine for a very short time, so that most of short-chain fatty acid, such as acetic acid, contained in the digested material is discharged along with stool while being absorbed little. This kind of increase in the amount of short-chain fatty acid gas due to diarrhea of a test subject is a very large value which is impossible in a condition without diarrhea, so that it is possible to definitely distinguish it from short-chain fatty acid gas discharged when physical condition is good on the basis of measurement data on the short-chain fatty acid gas. That is, it is possible to detect diarrhea of a test subject on the basis of the amount of short-chain fatty acid gas (acetic acid gas) contained in defecation gas measured.

As shown in FIG. 39, in the present embodiment, as with the first embodiment, the air intake passage 18b includes the filter 72, the deodorant filter 78 provided downstream of the filter 72, and the suction fan 18c, and the branch passage 283b branches on the downstream side of the filter 72. The filter 72 does not have a deodorizing function, and allows odiferous gas, hydrogen gas, and short-chain fatty acid gas to pass therethrough, but prevents foreign material, such as urine, and a cleaner from passing therethrough. As with the first embodiment, the deodorant filter 78 is also a catalyst that adsorbs gas components of odiferous gas or the like.

Defecation gas in the bowl 2a of the toilet is sucked into the air intake passage 18b at a fixed flow rate by the suction fan 18c. The defecation gas sucked into the air intake passage 18b passes through the filter 72 so that foreign material, such as urine, and a cleaner, is removed, and then is returned into the bowl 2a of the toilet after gas components of odiferous gas or the like are removed by the deodorant filter 78.

The branch passage 283b includes a flow channel changeover valve 284, a column 286, a semiconductor gas sensor 288, and a pump 290, in order from an upstream side toward a downstream side.

The flow channel changeover valve 284 is opened in a partial time (a very short time) during an excretory act to allow a part of defecation gas flowing through the air intake passage 18b (for the partial time during the excretory act of a test subject) to be drawn into the branch passage 283b. The flow channel changeover valve 284 is provided at the most upstream portion of the branch passage 283b.

The column 286 is provided downstream of the flow channel changeover valve 284, and is formed by filling elongated piping with thin fibers and the like, for example. The column 286 has a mechanism in which passing time of gas varies in accordance with molecule size (molecular weight), according to a principle of gas chromatography.

The sensor heater 54 is provided upstream of the semiconductor gas sensor 288 to heat a detecting portion of the semiconductor gas sensor 288 to a predetermined temperature as well as remove stink gas components attached to the semiconductor gas sensor 288.

The flow channel changeover valve 284 allows defecation gas in trace amounts flowing through the air intake passage 18b after passing through the filter 72 to flow into the branch passage 283b. Then, when the pump 290 is driven, each of hydrogen and odiferous gas, contained in the defecation gas, passes through the column 286 for a different time in accordance with molecular weight, according to the principle of gas chromatography, to reach the semiconductor gas sensor 288. That is, hydrogen with a small molecular weight tends to easily pass through the column 286 to reach the semiconductor gas sensor 288 in a short time, and odiferous gas with a large molecular weight tends to be difficult to pass through the column 286 to reach the semiconductor gas sensor 288 in a longer time as compared with the hydrogen. The pump 290 is configured to suck defecation gas at a fixed flow velocity.

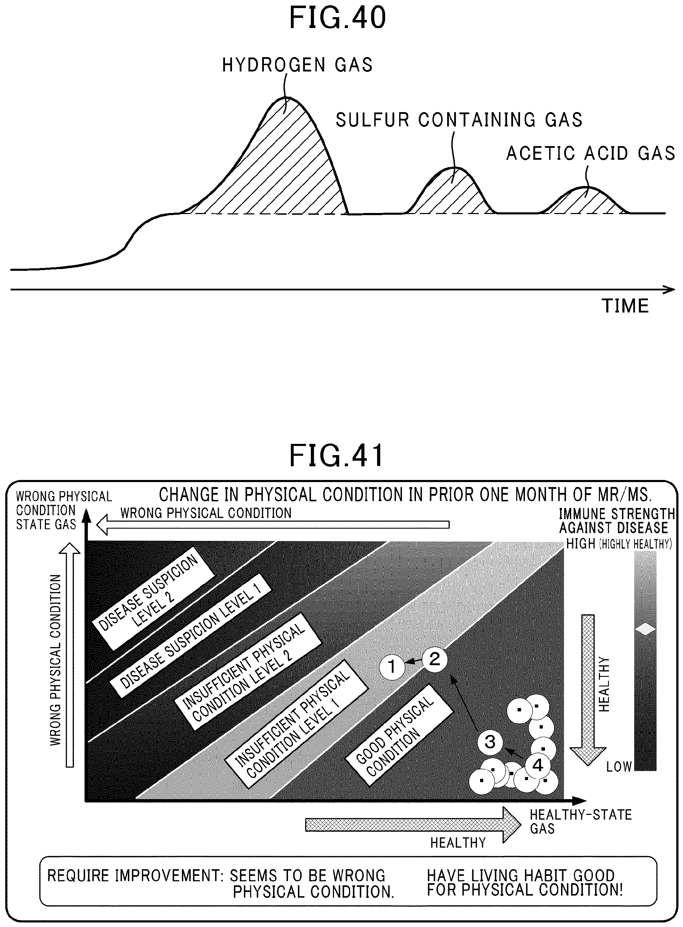

FIG. 40 shows a detection waveform acquired by a semiconductor gas sensor of a gas detector, shown in FIG. 39. As shown in FIG. 40, according to a configuration of a gas detector 220 of the present embodiment, the semiconductor gas sensor 288 reacts to short-chain fatty acid gas, hydrogen gas, and odiferous gas, which are temporally separated. In particular, an excretory act is performed in a short time, and defecation gas containing hydrogen and odiferous gas is also discharged only in a short time. In this way defecation gas is discharged in a short time, and thus providing the column 286 upstream of the semiconductor gas sensor 288 enables a time by which each of short-chain fatty acid gas, hydrogen gas, and odiferous gas reaches the semiconductor gas sensor to be varied, whereby it is possible to detect the amount of each of short-chain fatty acid gas, hydrogen gas, and odiferous gas, by using one semiconductor gas sensor 288. This is also based on technical findings made by the present inventors that if a method of determining physical condition using a correlation between healthy-state gas and odiferous gas, as well as the amount of short-chain fatty acid gas, without measuring all of the amount of methyl mercaptan gas in correlation with cancer, is adopted, gas only in a specific period can be measured in this kind of method. If a reduction sensor is used, the sensor is inexpensive but it is difficult to separate a large amount of hydrogen contained in defecation gas. In contrast, since the present embodiment allows a small amount of gas to be measured only in a specific period, separation of hydrogen becomes easy so that practicality can be achieved with a very inexpensive sensor.

While the present embodiment allows the column 286 to vary a reaching time of each of short-chain fatty acid gas, hydrogen gas, and odiferous gas, to the semiconductor gas sensor 288, it is a matter of course that it is also possible to vary a reaching time of methane contained in defecation gas. Accordingly, it is also possible to separate influence of not only hydrogen but also methane from detection data acquired by a semiconductor gas sensor.

Subsequently, with reference to FIG. 41, a physical condition display table in the present embodiment will be described. The physical condition display table is to be displayed by pressing the button of "detailed screen" in the display screen shown in FIG. 5 in the first embodiment.

As shown in FIG. 41, the physical condition display table is displayed on the left side of a display screen. The physical condition display table is a graph in which the vertical axis represents an index related to the amount of odiferous gas (referred to as wrong physical condition state gas in the display), referred to as a first index, and the horizontal axis represents an index related to the amount of healthy-state gas, referred to as a second index. In addition, a graph of immune strength against disease, estimated on the basis of detection data on short-chain fatty acid gas, is displayed in a right edge portion of the display screen, as a third index. That is, a level of immune strength of a test subject, acquired from detection data on short-chain fatty acid gas, is plotted in a longitudinal display bar for immune strength. The first index relates to the amount of odiferous gas based on first detection data acquired by the gas detector 220. The second index relates to the amount of hydrogen gas of healthy-state gas based on second detection data acquired by the gas detector 220. In addition, the third index relates to the amount of short-chain fatty acid gas based on third detection data acquired by the gas detector 220. As the second index, the amount of carbon dioxide gas or methane gas, which is healthy-state gas, is also available. The display device 68 of the remote control 8 displays the physical condition display table with the vertical axis and the horizontal axis as above, in which a measurement result of defecation gas of a test subject is plotted in a time-dependent manner.

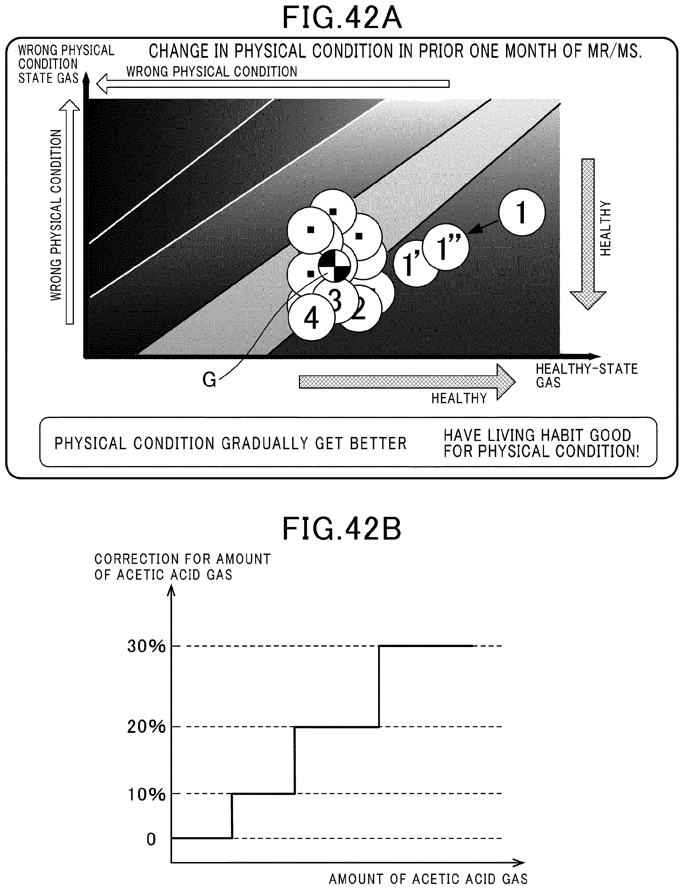

Next, with reference to FIG. 42, a biological information measurement system of a seventh embodiment of the present invention will be described.

In the sixth embodiment described above, immune strength of a test subject is displayed in the display bar for immune strength in the display device on the basis of acquired detection data on short-chain fatty acid gas, and a plotted point showing physical condition is displayed in the physical condition display table on the basis of acquired detection data on odiferous gas and hydrogen gas. The present embodiment is different from the sixth embodiment described above only in that a position of a plotted point to be displayed in the physical condition display table is corrected on the basis of detection data on short-chain fatty acid gas. Here, only a difference in the present embodiment from the sixth embodiment will be described, and description of a similar configuration, and the like, is omitted.

FIG. 42A shows an example of correction of a plotted point to be displayed in the physical condition display table. FIG. 42B shows an example of the amount of correction based on the amount of acetic acid gas of short-chain fatty acid detected.

As described in the sixth embodiment, a plotted point based on previous detection data is also displayed in the physical condition display table, along with a plotted point based on detection data acquired in a present defecation act. In addition, a display position of the present plotted point is corrected on the basis of reliability of the present measurement so as to be close to a position G of the center of gravity of the previous data (refer to FIG. 17) to be displayed. That is, in FIG. 42A, if a plotted point based on raw data of present detection data is indicated as "1", a display position of the plotted point is corrected to a position indicated as "1"' so as to be close to the position G of the center of gravity of the previous data to be displayed (the plotted point "1" is not actually displayed). In addition, the position of the plotted point "1" is corrected more as reliability of the present measurement decreases to be close to the position G of the center of gravity (the plotted point "1"' is displaced to near the position G of the center of gravity). Meanwhile, in the sixth embodiment, immune strength of a test subject is displayed in the display bar for immune strength on the basis of detection data on short-chain fatty acid gas, in addition to the physical condition display table.

In contrast, in the present embodiment, the position of the plotted point "1"' corrected on the basis of reliability is further corrected on the basis of detection data on short-chain fatty acid gas. That is, short-chain fatty acid gas, such as acetic acid gas, is created by good bacteria, such as bifidobacteria, in the large intestine, and is hardly created in a bad intestinal condition in which bad bacteria are predominant. Thus, even if the present measurement acquires detection data on odiferous gas and hydrogen gas that does not show better physical condition, it can be determined that condition in intestine is not always poor if some amount of acetic acid gas, and the like, is discharged. Then, the present embodiment corrects the amount of correction (the amount of correction between the plotted points "1" and "1"') in the present detection data on the basis of detection data on acetic acid gas so that the plotted point "1"' is displaced to a good physical condition side (a health side of a right side in FIG. 42A).

FIG. 42B shows an example of the amount of correction based on the amount of acetic acid gas in defecation gas.

For example, if the amount of acetic acid gas detected corresponds to a correction of "20%", a plotted point is displayed at a position indicated as "1"" to which a position of the plotted point "1"' is displaced to the health side (the right side in FIG. 42A) by a distance equivalent to 20% of a distance between the plotted points "1" and "1"' (the plotted points "1" and "1"' are not actually displayed). As shown in FIG. 42B, the amount of correction of a plotted point to the health side (a good physical condition side) increases as the amount of acetic acid gas detected increases. In this way, if a position of a plotted point to be displayed in the display device is corrected to the good physical condition side on the basis of the amount of acetic acid gas, condition in intestine of a test subject reflects a plotted point in the physical condition display table in a more multi-faceted manner, whereby it is possible to increase reliability of physical condition to be presented to a test subject.

Although the present embodiment allows the display device to display only the physical condition display table, a display bar for immune strength, showing immune strength may be displayed along with the physical condition display table as a variation, as with the first embodiment.

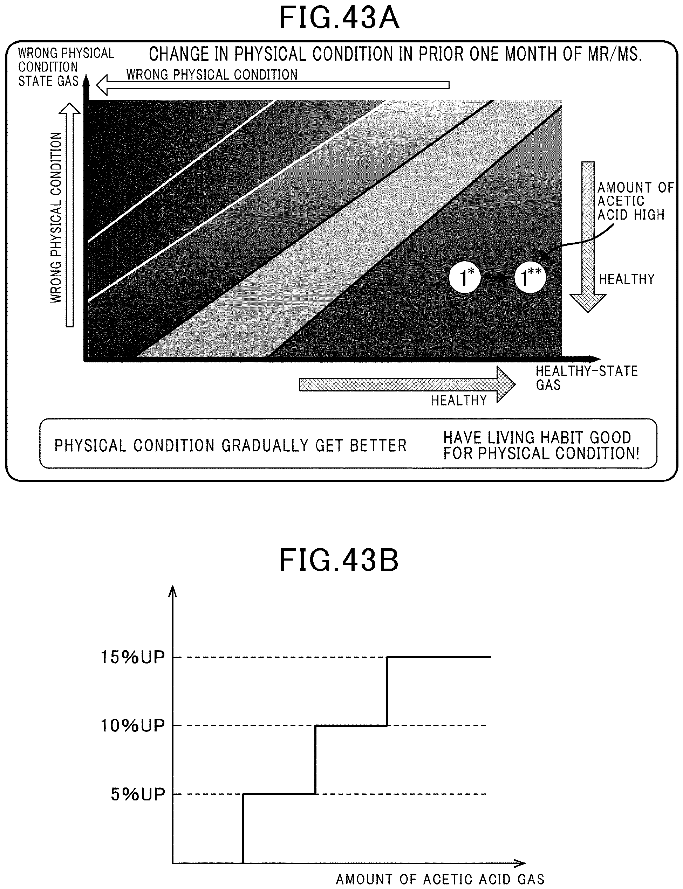

Next, with reference to FIG. 43, another variation of the biological information measurement system of the seventh embodiment of the present invention will be described.

The seventh embodiment described above corrects the amount of correction (correction of the plotted point "1" to the plotted point "1"') based on reliability of a plotted point of the present detection data on the basis of detection data on acetic acid gas so that the plotted point "1"" is displaced to the health side (correction of the plotted point "1"' to the plotted point "1""). In contrast, the present variation is different from the seventh embodiment in that a plotted point after correction based on reliability (the plotted point "1"' in FIG. 42A) is directly corrected on the basis of detection data on acetic acid gas.

As shown in FIG. 43A, the present variation directly corrects a plotted point in the physical condition display table on the basis of the amount of correction that is set on the basis of FIG. 43B. That is, if acquired detection data on the amount of acetic acid gas is equivalent to "10% up" in FIG. 43B, a value of the amount of hydrogen gas (the amount of healthy-state gas) at a plotted point after correction based on reliability (equivalent to "1*" in FIG. 43A, and to "1"' in FIG. 42A) is corrected to a value increased by 10%. Accordingly, the plotted point "1*" in FIG. 43A is displaced parallel along an axis of the amount of hydrogen gas (horizontal axis in FIG. 43A) to a plotted point "1**". In this way, if a position of a plotted point to be displayed in the display device is corrected to a health side (a right side in FIG. 43A) on the basis of the amount of acetic acid gas, condition in intestine of a test subject reflects a plotted point in the physical condition display table in a more multi-faceted manner, whereby it is possible to increase reliability of physical condition to be presented to a test subject. Since the present variation allows a plotted point in the physical condition display table to be simply displaced parallel along an axis of the amount of healthy-state gas, it is possible to perform correction with a simple calculation.

According to the biological information measurement system of each of the embodiments of the present invention, first detection data acquired by the odiferous gas sensor 26 sensitive to methyl mercaptan gas of odiferous gas, containing a sulfur component, as well as to odiferous gas other than the methyl mercaptan gas, is stored for each test subject, and physical condition of a test subject is analyzed on the basis of time-dependent change in a plurality of first detection data items in a defecation act performed multiple times in a predetermined period. As a result, it is possible to chronologically grasp the amount of odiferous gas in defecation gas for a long period even if there is used detection data acquired by the gas detector 20 using a general gas sensor sensitive to methyl mercaptan gas of odiferous gas, as well as to odiferous gas other than the methyl mercaptan gas, whereby it is possible to notify a wrong physical condition to a test subject in a state of ahead-disease before having a serious disease, such as colorectal cancer. Then, it is possible to provide a biological information measurement system at a cost, allowing general consumers to readily purchase it. According to the biological information measurement system of the present embodiment, it is possible to prevent people from having a serious disease, such as a cancer by measuring defecation gas at home, or to urge people to present to a hospital to receive treatment under a moderate condition.

In the biological information measurement system of the present embodiment, the gas detector 20 is configured to output second detection data related to hydrogen gas, and the data analyzer 60 analyzes physical condition of a test subject on the basis of a relationship between the first index related to odiferous gas and the second index related to hydrogen gas. The hydrogen gas is called healthy-state gas, and it is known that a large amount of the hydrogen gas is discharged when condition in intestine is good. The present inventors find that if a test subject is in a wrong physical condition, as physical condition deteriorates, odiferous gas in defecation gas increases in amount, but the amount of healthy-state gas decreases. Since the biological information measurement system 1 of the present embodiment analyzes physical condition of a test subject on the basis of time-dependent change in a relationship between odiferous gas and healthy-state gas, in a defecation act performed multiple times, it is possible to reliably notify a wrong physical condition to the test subject in a state of ahead-disease before having colorectal cancer. Even if data analysis is based on detection data acquired by a gas detector with less measurement accuracy, it is possible to extract useful information by the data analysis because a state of defecation gas is evaluated on the basis of a relationship between odiferous gas and healthy-state gas.

In addition, since the biological information measurement system of the present embodiment analyzes physical condition of a test subject on the basis of information on a test subject in addition to first detection data, and second detection data, it is possible to accurately measure the physical condition. The biological information measurement system 1 of the present embodiment analyzes physical condition of a test subject in consideration of information on the test subject, so that a personal difference in the amount of odiferous gas containing a sulfur component can be absorbed to enable accurately measuring physical condition, as well as enable preventing an unnecessary mental burden from being applied to a test subject due to notification of a wrong result.

Since the biological information measurement system of the present embodiment allows the display device 68 to display a plurality of analysis results related to every defecation act (at time t1 to time t8, in FIG. 9) in a time-dependent manner (refer to FIG. 6), a test subject can grasp his or her own physical condition as time-dependent change, whereby it is possible to urge the test subject to perform health management, such as improvement in a living habit.

In addition, the biological information measurement system of the present embodiment displays a plurality of analysis results (plotted points in FIG. 6) in a time-dependent manner in the physical condition display table (refer to FIG. 6), provided with the first axis (a vertical axis of FIG. 6) related to odiferous gas containing a sulfur component, and the second axis (a horizontal axis of FIG. 6) related to healthy-state gas, a test subject can visually grasp his or her own change in physical condition, whereby it is possible to easily determine his or her own physical condition.

According to the biological information measurement system of the present embodiment, with respect to whether physical condition is good or bad, a plurality of analysis results are plotted in a time-dependent manner in the physical condition display table divided into a plurality of stage areas ("disease suspicion level 2", "disease suspicion level 1", etc., in FIG. 6), so that a test subject can visually determine what level of physical condition corresponds to his or her own physical condition, whereby it is possible to make an effort for health management.

Since the biological information measurement system of the present embodiment acquires detection data even before a test subject is identified, a test subject is released from reluctance to input information on a test subject before defecation, whereby it is possible to reduce an operating burden when physical condition is measured. Accordingly, a test subject easily continues to measure physical condition without receiving an excessive operating burden.

In addition, since the biological information measurement system of the present embodiment notifies a test subject if the test subject does not input specific information on a test subject for a predetermined time, it is possible to prevent input of specific information on a test subject from being forgotten, whereby it is possible to easily continue to measure physical condition.

Since the biological information measurement system of the present embodiment does not allow cleaning of the flush toilet 2 if a test subject does not input specific information on a test subject, the test subject is urged to input the specific information on a test subject, whereby it is possible to continue to reliably measure physical condition.

In addition, since the biological information measurement system of the present embodiment plots a corrected analysis result in the physical condition display table (refer to FIG. 7A), it is possible to prevent a large variation of physical condition to be displayed due to a large error to be included in detection data, or a temporary wrong physical condition.

Further, since the biological information measurement system of the present embodiment corrects an analysis result to be plotted in the physical condition display table to a good physical condition side (refer to FIG. 7A), it is possible to prevent physical condition to be displayed from remarkably deteriorating to apply an excessive mental burden to a test subject, due to a large error to be included in detection data, or a temporary wrong physical condition.

Furthermore, since the biological information measurement system of the present embodiment reduces the amount of correction (refer to FIG. 7B) if an analysis result showing a wrong physical condition continues predetermined times or more, a result showing a wrong physical condition is displayed for a continual wrong physical condition and the wrong physical condition can be notified to a test subject before greatly deteriorating, whereby it is possible to urge the test subject to manage health, to present to a hospital, or the like.

The biological information measurement system of the present embodiment displays a message related to health management of a test subject (refer to a middle stage in FIG. 5), so that the test subject can take an appropriate action on the basis of his or her own physical condition displayed, whereby it is possible to early address improvement in physical condition.

Since the biological information measurement system of the present embodiment corrects an analysis result on the basis of reliability (refer to FIG. 17), it is possible to prevent a plotted point to be displayed in the physical condition display table from greatly varying due to an analysis result with low reliability to prevent an unnecessary mental burden from being applied to a test subject.

In addition, since the biological information measurement system of the present embodiment assigns a higher weight coefficient to detection data acquired by detecting defecation gas discharged early than that to later detection data (refer to a portion of a correction value in FIG. 12, and FIG. 16), it is possible to perform further accurate measurement. Further, measuring defecation gas discharged early enables presenting an analysis result to a test subject at an end of one defecation act, or immediately after the one defecation act, so that it is possible to present a measurement result of physical condition without allowing a test subject to excessively wait.

According to the biological information measurement system of the present embodiment, a different evaluation of health condition is made depending on a value in the first axis (the vertical axis in FIG. 6) representing the first index even if a value in the second axis (the horizontal axis of FIG. 6) representing the second index is identical, so that it is possible to perform more accurate measurement of physical condition.

In addition, since the biological information measurement system of the present embodiment includes the hydrogen gas sensor 24 for detecting hydrogen gas, and the carbon dioxide sensor 28 for detecting carbon dioxide gas, it is possible to evaluate healthy-state gas indicating good physical condition on the basis of two kinds of gas, whereby it is possible to more accurately measure physical condition.

Although the embodiments described above of the present invention are provided to suck defecation gas discharged into a bowl of a toilet for analysis, defecation gas also can be collected from a portion other than a bowl of a toilet if physical condition of a test subject, such as a bedridden patient, is analyzed. For example, in the embodiment shown in FIG. 37, if a pipe for suction is connected to the end of the duct 118a, defecation gas can be directly collected from a test subject through the pipe for suction. In this case, if a sheet-like defecation gas collecting fixture (not shown) is connected to an end of the pipe for suction, and is placed in bedclothes (a sleeping mat and a comforter) of a test subject, defecation gas discharged from the test subject can be sucked. The sucked defecation gas is sucked from the duct 118a through the pipe for suction, and then a gas sensor assembled in the device body 180 acquires detection data on the gas. Alternatively, the defecation gas collecting fixture may be in placed in underwear or a diaper of a test subject. It is also possible to directly place a necessary gas sensor in bedclothes, underwear, a diaper, or the like, of a test subject, to measure defecation gas to analyze physical condition of the test subject. In this case, preferably, detection data acquired by the gas sensor is wirelessly transmitted to a device on a test subject side, or a server.

In the embodiments described above, although healthy-state gas, such as hydrogen gas, methane gas, or carbon dioxide gas, is detected, research by the present inventors reveals that while many test subjects include hydrogen gas in defecation gas as healthy-state gas but no methane gas, a part of test subjects includes methane gas in defecation gas but no hydrogen gas. Thus, if healthy-state gas is measured, it is preferable to provide a gas detector capable of detecting both hydrogen gas and methane gas. In a case of a device targeting a specific test subject who is known for what kind of healthy-state gas is discharged, the device may be configure to be able to detect only any one of kinds of gas.

### [Reference Signs List]

R toilet installation room
1 biological information measurement system in accordance with first embodiment of the present invention
2 flush toilet
2a bowl
4 seat
6 measuring device
8 remote control
10 device on a test subject side
12 server
14 terminal for test subject
16 medical facilities terminal
18 suction device
18a duct
18b air intake passage
18c suction fan
20 gas detector
22 control device
22a CPU
22b storage device
24 hydrogen gas sensor
26 odiferous gas sensor
28 carbon dioxide sensor
30 humidity sensor
32 temperature sensor
34 entrance detection sensor
36 seating detection sensor
38 defecation/urination detection sensor
40 toilet lid opening/closing device
42 nozzle driving device
44 nozzle cleaning device
46 toilet cleaning device
48 toilet disinfection device
50 aromatic sprayer
52 deodorizing air supply device
54 sensor heater
56 transmitter-receiver
58 duct cleaner
59 humidity adjuster
60 data analyzer
62 test subject identification device
64 input device
66 transmitter-receiver
68 display device
70 speaker
72 filter
78 deodorant filter
101 biological information measurement system of fourth embodiment
104 seat
106 measuring device
118a duct
180 device body
182 power source code
120 gas detector of fifth embodiment
283a main passage
283b branch passage
284 flow channel changeover valve
286 column
288 semiconductor gas sensor
290 pump

## Claims

1. A biological information measurement system (1) that measures physical condition of a test subject on the basis of defecation gas discharged into a bowl (2a) of a flush toilet (2) installed in a toilet installation space (R), the biological information measurement system (1) comprising:
a test subject identification device (62) for identifying the test subject who uses the flush toilet (2);
a suction device (18) that sucks gas in the bowl (2a) into which the defecation gas is discharged by the test subject;
a gas detector (20) provided with a gas sensor (26) that is sensitive to methyl mercaptan gas, as well as to odiferous gas containing a sulfur component other than the methyl mercaptan gas, included in gas sucked by the suction device (18);
a control device (22) that controls the suction device (18) and the gas detector (20);
a storage device (22b) that stores first detection data acquired by the gas detector (20) for each of test subjects identified by the subject identification device (62), wherein the first detection data are related to the methyl mercaptan gas and the odiferous gas;
a data analyzer (60) that analyzes physical condition of the test subject on the basis of time-dependent change in a plurality of first detection data items that are detected in defecation acts performed multiple times in a predetermined period, and that is stored in the storage device (22b); and
an output device (66, 68, 70) that outputs an analysis result acquired by the data analyzer,
wherein the gas detector (20) is configured to sense a healthy-state gas composed of at least one of hydrogen gas, carbon dioxide gas, and methane gas, contained in gas sucked by the suction device (18), to output second detection data which are related to the healthy-state gas, and wherein the data analyzer (60) acquires a relationship between a first index related to odiferous gas containing a sulfur component, acquired on the basis of first detection data, and a second index related to healthy-state gas, acquired on the basis of second detection data in one defecation act, to analyze physical condition of the test subject on the basis of time-dependent change in the acquired relationship in a defecation act performed multiple times.

2. The biological information measurement system according to claim 1, further comprising:
a test-subject-information storage device that stores information on weight, age, and sex of the test subject, or information on elapsed time from a previous defecation act,
wherein the data analyzer (60) analyzes physical condition of the test subject on the basis of the first detection data, the second detection data, and the information on the test subject stored in the test-subject-information storage device.

3. The biological information measurement system according to any one of the preceding claims, wherein the output device displays a plurality of analysis results related to every defecation act analyzed by the data analyzer (60) in time-dependent manner.

4. The biological information measurement system according to any one the preceding claims, wherein the output device (66, 68) displays a plurality of analysis results in the time-dependent manner in a physical condition display table, provided with a first axis representing the first index acquired on the basis of the first detection data, and a second axis representing the second index acquired on the basis of the second detection data.

5. The biological information measurement system according to claim 4, wherein the physical condition display table is divided into a plurality of stage areas with respect to whether physical condition is good or bad, and the plurality of analysis results is plotted in a time-dependent manner in the physical condition display table divided into the areas.

6. The biological information measurement system according to claim 4 or 5, wherein the output device (66, 68, 70) corrects the analysis result most recently acquired by the data analyzer to plot the corrected result in the physical condition display table.

7. The biological information measurement system according to any one of claims 4 to 6, wherein if the most recent analysis result is changed to analysis result representing wrong physical condition, the output device (66, 68, 70) corrects the analysis result to be plotted in the physical condition display table to good physical condition side to display the corrected analysis result.

8. The biological information measurement system according to any one of claims 4 to 7, wherein if the analysis result showing wrong physical condition continues predetermined times or more, the output device (66, 68, 70) reduces an amount of correction with respect to the analysis result to display the corrected analysis result.

9. The biological information measurement system according to any one of claims 4 to 8, wherein the data analyzer (60) calculates reliability of detected data, on the basis of the first detection data and the second detection data, and wherein the output device plots the analysis result corrected on the basis of the reliability of the data in the physical condition display table.

10. The biological information measurement system according to any one of claims 4 to 9, wherein if reliability of data, calculated by the data analyzer (60), is low, the output device (66, 68, 70) increases the amount of correction for allowing the analysis result to be plotted in the physical condition display table to be closer to previous plotted points side than a case where reliability of data is high.

11. The biological information measurement system according to claim 9, wherein the data analyzer (60) calculates smaller reliability of data when noise included in the first detection data and the second detection data is larger.

12. The biological information measurement system according to any one of the preceding claims, wherein the output device (66, 68, 70) displays a message related to health management of the test subject on the basis of the analysis result acquired by the data analyzer (60).

13. The biological information measurement system according to any one of the preceding claims, wherein the suction device (18) and the gas detector (20) are configured to suck gas and detect the first detection data, respectively, even before the test subject identification device (62) identifies the test subject who uses the toilet (2), and wherein the storage device (22b) stores first detection data in association with the test subject identified by the test subject identification device (62) after the first detection data is acquired.

14. The biological information measurement system according to any one of the preceding claims, wherein if the test subject does not input information of identifying the test subject into the test subject identification device (62) for a predetermined time after the gas detector acquires first detection data, the output device (66, 68, 70) outputs notification of urging the test subject to input the information.

15. The biological information measurement system according to any one of the preceding claims, wherein the data analyzer (60) performs analysis by assigning a higher weight coefficient to the first detection data and the second detection data, acquired by detecting defecation gas discharged early in one defecation act of the test subject, than that to the first detection data and the second detection data acquired by detecting defecation gas discharged later in the one defecation act.

16. The biological information measurement system according to any one of the preceding claims, wherein the data analyzer (60) performs analysis by using only the first detection data and the second detection data, acquired by detecting defecation gas discharged first in one defecation act of the test subject.

17. The biological information measurement system according to any one of the preceding claims, wherein the gas detector (20) is configured to be able to detect also vaporized short-chain fatty acid contained in defecation gas sucked by the suction device (18), and wherein the data analyzer (60) analyzes the first index based on detection data on odiferous gas, the second index based on detection data on healthy-state gas, and a third index based on detection data on short-chain fatty acid, as physical condition of the test subject.

18. The biological information measurement system according to claim 17, wherein the data analyzer (60) is configured to analyze whether physical condition is good or bad on the basis of the first and second indexes, and to output the analysis result to the output device (66, 68, 70), and wherein when a value of the third index is large, the data analyzer (60) outputs the analysis result based on the first and second indexes is corrected to a good physical condition side greater than when the value of the third index is small.

19. The biological information measurement system according to claim 17 or 18, wherein the gas detector (20) is configured to be able to detect acetic acid or propionic acid of short-chain fatty acid, and wherein the data analyzer (60) analyzes physical condition of the test subject on the basis of the time-dependent tendency of change in detection data on acetic acid or propionic acid.

20. The biological information measurement system according to any one of claims 17 to 19, further comprising:
diarrhea determination means for detecting whether the test subject has diarrhea or not, wherein the diarrhea determination means detect diarrhea based on short-chain fatty acid gas contained in defecation gas sucked by the suction device (18), and wherein if the diarrhea determination means determines that the test subject has diarrhea, detection data on short-chain fatty acid, acquired in the defecation act, is not used for analysis of physical condition, or weighting of the detection data is reduced.

21. The biological information measurement system according to any one of the preceding claims, wherein the data analyzer (60) analyzes current health condition of the test subject as well as analyzes resistance to deterioration of a condition in the intestine of the test subject on the basis of detection data on short-chain fatty acid.

## Patentansprüche

1. Messsystem (1) für biologische Informationen, das den physischen Zustand einer Testperson auf Grundlage von Defäkationsgas misst, das in eine Schüssel (2a) einer Spültoilette (2) abgegeben wird, die in einem Toiletteninstallationsraum (R) installiert ist, wobei das Messsystem (1) für biologische Informationen umfasst:
eine Testperson-Identifikationsvorrichtung (62), um die Testperson zu identifizieren, die die Spültoilette (2) benutzt;
eine Absaugvorrichtung (18), die Gas in der Schüssel (2a) absaugt, in die das Defäkationsgas von der Testperson abgegeben wird;
einen Gasdetektor (20), der mit einem Gassensor (26) versehen ist, der für Methylmercaptangas sowie für ein eine Schwefelkomponente enthaltendes Geruchsgas außer dem Methylmercaptangas sensitiv ist, die in dem Gas enthalten sind, das durch die Absaugvorrichtung (18) abgesaugt wurde;
eine Steuervorrichtung (22), welche die Absaugvorrichtung (18) und den Gasdetektor (20) steuert;
eine Speichervorrichtung (22b), die erste Detektionsdaten speichert, die durch den Gasdetektor (20) für jede von durch die Testperson-Identifikationsvorrichtung (62) identifizierten Testpersonen erfasst wurden, wobei die ersten Detektionsdaten auf das Methylmercaptangas und das Geruchsgas bezogen sind;
einen Datenanalysator (60), der einen physischen Zustand der Testperson auf Grundlage einer zeitabhängigen Änderung bei mehreren ersten Detektionsdatenelementen analysiert, die in mehrfach in einem vorbestimmten Zeitraum erfolgten Defäkationsakten erfasst werden, und was in der Speichervorrichtung (22b) gespeichert ist; und
eine Ausgabevorrichtung (66, 68, 70), die ein durch den Datenanalysator erlangtes Analyseergebnis ausgibt,
wobei der Gasdetektor (20) dazu ausgelegt ist, ein Gas eines gesunden Zustands abzufühlen, das sich aus Sauerstoffgas, Kohlendioxidgas und/oder Methangas zusammensetzt, das in dem Gas enthalten ist, das durch die Absaugvorrichtung (18) abgesaugt wurde, um zweite Detektionsdaten auszugeben, die auf das Gas des gesunden Zustands bezogen sind, und wobei der Datenanalysator (60) ein Verhältnis zwischen einem ersten Index, der sich auf Geruchsgas bezieht, das eine Schwefelkomponente enthält, der auf Grundlage der ersten Detektionsdaten erfasst wird, und einem zweiten Index erfasst, der sich auf das Gas des gesunden Zustands bezieht, der auf Grundlage der zweiten Detektionsdaten in einem Defäkationsakt erfasst wird, um den physischen Zustand der Testperson auf Grundlage der zeitabhängigen Änderung bei dem erfassten Verhältnis in einem mehrfach erfolgten Defäkationsakt zu analysieren.

2. Messsystem für biologische Informationen nach Anspruch 1, darüber hinaus umfassend:
eine Testperson-Informationsspeichervorrichtung, die Informationen über Gewicht, Alter und Geschlecht der Testperson oder Informationen über eine ab einem vorherigen Defäkationsakt vergangene Zeit speichert,
wobei der Datenanalysator (60) den physischen Zustand der Testperson auf Grundlage der ersten Detektionsdaten, der zweiten Detektionsdaten und der Informationen über die Testperson analysiert, die in der Testperson-Informationsspeichervorrichtung gespeichert sind.

3. Messsystem für biologische Informationen nach einem der vorhergehenden Ansprüche, wobei die Ausgabevorrichtung mehrere sich auf jeden Defäkationsakt beziehende Analyseergebnisse, die durch den Datenanalysator (60) analysiert wurden, zeitabhängig anzeigt.

4. Messsystem für biologische Informationen nach einem der vorhergehenden Ansprüche, wobei die Ausgabevorrichtung (66, 68) mehrere Analyseergebnisse zeitabhängig in einer Anzeigetabelle für den physischen Zustand anzeigt, die mit einer ersten Achse, die den auf Grundlage der ersten Detektionsdaten erfassten ersten Index darstellt, und einer zweiten Achse versehen ist, die den auf Grundlage der zweiten Detektionsdaten erfassten zweiten Index darstellt.

5. Messsystem für biologische Informationen nach Anspruch 4, wobei die Anzeigetabelle für den physischen Zustand in mehrere Stufenbereiche im Hinblick darauf unterteilt ist, ob der physische Zustand gut oder schlecht ist, und die mehreren Analyseergebnisse zeitabhängig in der in die Bereiche unterteilten Anzeigetabelle für den physischen Zustand grafisch dargestellt sind.

6. Messsystem für biologische Informationen nach Anspruch 4 oder 5, wobei die Ausgabevorrichtung (66, 68, 70) die zuletzt durch den Datenanalysator erfassten Analyseergebnisse korrigiert, um das korrigierte Ergebnis in der Anzeigetabelle für den physischen Zustand grafisch darzustellen.

7. Messsystem für biologische Informationen nach einem der Ansprüche 4 bis 6, wobei, wenn das letzte Analyseergebnis auf ein Analyseergebnis abgeändert wird, das einen falschen physischen Zustand darstellt, die Ausgabevorrichtung (66, 68, 70) das in der Anzeigetabelle für den physischen Zustand grafisch darzustellende Analyseergebnis auf die Seite eines guten physischen Zustands abzuändern, um das korrigierte Analyseergebnis anzuzeigen.

8. Messsystem für biologische Informationen nach einem der Ansprüche 4 bis 7, wobei, wenn das Analyseergebnis, das einen falschen physischen Zustand zeigt, sich eine vorbestimmte Anzahl von Malen oder mehr fortsetzt, die Ausgabevorrichtung (66, 68, 70) einen Korrekturbetrag im Hinblick auf das Analyseergebnis reduziert, um das korrigierte Analyseergebnis anzuzeigen.

9. Messsystem für biologische Informationen nach einem der Ansprüche 4 bis 8, wobei der Datenanalysator (60) die Zuverlässigkeit erfasster Daten auf Grundlage der ersten Detektionsdaten und der zweiten Detektionsdaten berechnet, und wobei die Ausgabevorrichtung das auf Grundlage der Zuverlässigkeit der Daten korrigierte Analyseergebnis in der Anzeigetabelle für den physischen Zustand grafisch darstellt.

10. Messsystem für biologische Informationen nach einem der Ansprüche 4 bis 9, wobei, wenn die durch den Datenanalysator (60) berechnete Zuverlässigkeit von Daten gering ist, die Ausgabevorrichtung (66, 68, 70) den Korrekturbetrag erhöht, um zu ermöglichen, dass das in der Anzeigetabelle für den physischen Zustand grafisch darzustellende Analyseergebnis näher auf der Seite vorheriger grafisch dargestellter Punkte als ein Fall liegt, in dem die Zuverlässigkeit von Daten hoch ist.

11. Messsystem für biologische Informationen nach Anspruch 9, wobei der Datenanalysator (60) eine kleinere Zuverlässigkeit von Daten berechnet, wenn ein in den ersten Detektionsdaten und den zweiten Detektionsdaten enthaltenes Rauschen größer ist.

12. Messsystem für biologische Informationen nach einem der vorhergehenden Ansprüche, wobei die Ausgabevorrichtung (66, 68, 70) eine auf das Gesundheitsmanagement der Testperson bezogene Nachricht auf Grundlage des durch den Datenanalysator (60) erfassten Analyseergebnisses anzeigt.

13. Messsystem für biologische Informationen nach einem der vorhergehenden Ansprüche, wobei die Absaugvorrichtung (18) und der Gasdetektor (20) dazu ausgelegt sind, Gas abzusaugen bzw. die ersten Detektionsdaten zu erfassen, und zwar sogar, bevor die Testperson-Identifikationsvorrichtung (62) die Testperson identifiziert, die die Toilette (2) benutzt, und wobei die Speichervorrichtung (22b) erste Detektionsdaten in Verbindung mit der durch die Testperson-Identifikationsvorrichtung (62) identifizierten Testperson speichert, nachdem die ersten Detektionsdaten erfasst wurden.

14. Messsystem für biologische Informationen nach einem der vorhergehenden Ansprüche, wobei, wenn die Testperson eine vorbestimmte Zeit lang, nachdem der Gasdetektor erste Detektionsdaten erfasst, keine Informationen zum Identifizieren der Testperson in die Testperson-Identifikationsvorrichtung (62) eingibt, die Ausgabevorrichtung (66, 68, 70) eine Mitteilung ausgibt, um die Testperson dringend zu ersuchen, die Informationen einzugeben.

15. Messsystem für biologische Informationen nach einem der vorhergehenden Ansprüche, wobei der Datenanalysator (60) eine Analyse durchführt, indem er den ersten Detektionsdaten und den zweiten Detektionsdaten, die durch Erkennen von früh in einem Defäkationsakt der Testperson abgegebenen Defäkationsgas erfasst wurden, einen höheren Gewichtungskoeffizienten zuteilt als den ersten Detektionsdaten und den zweiten Detektionsdaten, die durch Erkennen von später in einem Defäkationsakt abgegebenem Defäkationsgas erfasst wurden.

16. Messsystem für biologische Informationen nach einem der vorhergehenden Ansprüche, wobei der Datenanalysator (60) eine Analyse durchführt, indem er nur die ersten Detektionsdaten und die zweiten Detektionsdaten verwendet, die durch Erkennen von in einem Defäkationsakt der Testperson zuerst abgegebenem Defäkationsgas erfasst wurden.

17. Messsystem für biologische Informationen nach einem der vorhergehenden Ansprüche, wobei der Gasdetektor (20) dazu ausgelegt ist, auch eine verdampfte kurzkettige Fettsäure erkennen zu können, die in dem durch die Absaugvorrichtung (18) abgesaugten Defäkationsgas enthalten ist, und wobei der Datenanalysator (60) den ersten Index auf Grundlage von Detektionsdaten über Geruchsgas, den zweiten Index auf Grundlage von Detektionsdaten über einen gesunden Zustand, und einen dritten Index auf Grundlage von Detektionsdaten über eine kurzkettige Fettsäure als physischen Zustand der Testperson analysiert.

18. Messsystem für biologische Informationen nach Anspruch 17, wobei der Datenanalysator (60) dazu ausgelegt ist, auf Grundlage des ersten und zweiten Indexes zu analysieren, ob der physische Zustand gut oder schlecht ist, und das Analyseergebnis an die Ausgabevorrichtung (66, 68, 70) auszugeben, und wobei, wenn ein Wert des dritten Indexes groß ist, der Datenanalysator (60) das Analyseergebnis auf Grundlage des ersten und zweiten Indexes ausgibt, das mehr auf die Seite eines guten physischen Zustands abgeändert ist, als wenn der Wert des dritten Indexes klein ist.

19. Messsystem für biologische Informationen nach Anspruch 17 oder 18, wobei der Gasdetektor (20) dazu ausgelegt ist, eine Essigsäure oder Propionsäure einer kurzkettigen Fettsäure erkennen zu können, und wobei der Datenanalysator (60) den physischen Zustand der Testperson auf Grundlage der zeitabhängigen Änderungstendenz bei Detektionsdaten über Essigsäure oder Propionsäure analysiert.

20. Messsystem für biologische Informationen nach einem der Ansprüche 17 bis 19, darüber hinaus umfassend:
Diarrhoe-Bestimmungseinrichtungen, um zu erkennen, ob die Testperson Diarrhoe hat oder nicht, wobei die Diarrhoe-Bestimmungseinrichtungen eine Diarrhoe auf Grundlage von Gas kurzkettiger Fettsäuren erkennen, das in dem durch die Absaugvorrichtung (18) abgesaugten Defäkationsgas enthalten ist, und wobei, wenn die Diarrhoe-Bestimmungseinrichtungen bestimmen, dass die Testperson eine Diarrhoe hat, Detektionsdaten über eine kurzkettige Fettsäure, die im Defäkationsakt erfasst wurden, nicht zur Analyse des physischen Zustands verwendet werden oder eine Gewichtung der Detektionsdaten reduziert wird.

21. Messsystem für biologische Informationen nach einem der vorhergehenden Ansprüche, wobei der Datenanalysator (60) den momentanen Gesundheitszustand der Testperson sowie eine Widerstandsfähigkeit gegen eine Verschlechterung eines Zustands im Darm der Testperson auf Grundlage von Detektionsdaten über eine kurzkettige Fettsäure analysiert.

## Revendications

1. Système de mesure d'informations biologiques (1) qui mesure un état physique d'un sujet test sur la base d'un gaz de défécation évacué dans une cuvette (2a) de W.C. (2) installée dans un espace d'installation de W.C. (R), le système de mesure d'informations biologiques (1) comprenant :
un dispositif d'identification de sujet test (62) destiné à identifier le sujet test qui utilise les W.C. (2) ;
un dispositif d'aspiration (18) qui aspire du gaz dans la cuvette (2a) dans laquelle le gaz de défécation est évacué par le sujet test ;
un détecteur de gaz (20) pourvu d'un capteur de gaz (26) qui est sensible à du gaz de mercaptan méthylique, ainsi qu'à du gaz odoriférant contenant une composante de soufre autre que le gaz de mercaptan méthylique, contenu dans du gaz aspiré par le dispositif d'aspiration (18) ;
un dispositif de commande (22) qui commande le dispositif d'aspiration (18) et le détecteur de gaz (20) ;
un dispositif de stockage (22b) qui stocke des premières données de détection acquises par le détecteur de gaz (20) pour chacun de sujets tests identifiés par le dispositif d'identification de sujet (62), sachant que les premières données de détection sont relatives au gaz de mercaptan méthylique et au gaz odoriférant ;
un analyseur de données (60) qui analyse un état physique du sujet test sur la base d'un changement en fonction du temps dans une pluralité de premiers éléments de données de détection qui sont détectés lors d'actes de défécation effectués de multiples fois au cours d'une période prédéterminée, et ce qui est stocké dans le dispositif de stockage (22b) ; et
un dispositif de sortie (66, 68, 70) qui sort un résultat d'analyse acquis par l'analyseur de données,
sachant que le détecteur de gaz (20) est configuré pour capter un gaz d'état sain composé d'au moins l'un d'un gaz d'hydrogène, d'un gaz de dioxyde de carbone, et d'un gaz de méthane, contenu dans du gaz aspiré par le dispositif d'aspiration (18), pour sortir des deuxièmes données de détection qui sont relatives au gaz d'état sain, et sachant que l'analyseur de données (60) acquiert une relation entre un premier indice relatif à un gaz odoriférant contenant une composante de soufre, acquis sur la base de premières données de détection, et un deuxième indice relatif à un gaz d'état sain, acquis sur la base de deuxièmes données de détection lors d'un acte de défécation, pour analyser un état physique du sujet test sur la base d'un changement en fonction du temps dans la relation acquise lors d'un acte de défécation effectué de multiples fois.

2. Le système de mesure d'informations biologiques selon la revendication 1, comprenant en outre :
un dispositif de stockage d'informations de sujet test qui stocke des informations sur le poids, l'âge, et le sexe du sujet test, ou des informations sur un temps écoulé depuis un précédent acte de défécation,
sachant que l'analyseur de données (60) analyse un état physique du sujet test sur la base des premières données de détection, des deuxièmes données de détection, et des informations sur le sujet test stockées dans le dispositif de stockage d'informations de sujet test.

3. Le système de mesure d'informations biologiques selon l'une quelconque des revendications précédentes, sachant que le dispositif de sortie affiche une pluralité de résultats d'analyse relatifs à chaque acte de défécation analysés par l'analyseur de données (60) en fonction du temps.

4. Le système de mesure d'informations biologiques selon l'une quelconque des revendications précédentes, sachant que le dispositif de sortie (66, 68) affiche une pluralité de résultats d'analyse en fonction du temps dans un tableau d'affichage d'état physique, pourvu d'un premier axe représentant le premier indice acquis sur la base des premières données de détection, et d'un deuxième axe représentant le deuxième indice acquis sur la base des deuxièmes données de détection.

5. Le système de mesure d'informations biologiques selon la revendication 4, sachant que le tableau d'affichage d'état physique est divisé en une pluralité de zones étagées par rapport au fait de savoir si un état physique est bon ou mauvais, et la pluralité de résultats d'analyse est tracée en fonction du temps dans le tableau d'affichage d'état physique divisé en lesdites zones.

6. Le système de mesure d'informations biologiques selon la revendication 4 ou 5, sachant que le dispositif de sortie (66, 68, 70) corrige le résultat d'analyse le plus récemment acquis par l'analyseur de données pour tracer le résultat corrigé dans le tableau d'affichage d'état physique.

7. Le système de mesure d'informations biologiques selon l'une quelconque des revendications 4 à 6, sachant que si le résultat d'analyse le plus récent est changé en résultat d'analyse représentant un mauvais état physique, le dispositif de sortie (66, 68, 70) modifie le résultat d'analyse à tracer dans le tableau d'affichage d'état physique vers le côté bon état physique pour afficher le résultat d'analyse corrigé.

8. Le système de mesure d'informations biologiques selon l'une quelconque des revendications 4 à 7, sachant que si le résultat d'analyse présentant un mauvais état physique se poursuit un nombre prédéterminé de fois ou plus, le dispositif de sortie (66, 68, 70) réduit un taux de correction par rapport au résultat d'analyse pour afficher le résultat d'analyse corrigé.

9. Le système de mesure d'informations biologiques selon l'une quelconque des revendications 4 à 8, sachant que l'analyseur de données (60) calcule une fiabilité de données détectées, sur la base des premières données de détection et des deuxièmes données de détection, et sachant que le dispositif de sortie trace le résultat d'analyse corrigé sur la base de la fiabilité des données dans le tableau d'affichage d'état physique.

10. Le système de mesure d'informations biologiques selon l'une quelconque des revendications 4 à 9, sachant que si une fiabilité de données, calculée par l'analyseur de données (60), est faible, le dispositif de sortie (66, 68, 70) augmente le taux de correction pour faire en sorte que le résultat d'analyse à tracer dans le tableau d'affichage d'état physique soit plus proche du côté de précédents points tracés que dans un cas où une fiabilité de données est élevée.

11. Le système de mesure d'informations biologiques selon la revendication 9, sachant que l'analyseur de données (60) calcule une fiabilité plus petite de données lorsque du bruit inclus dans les premières données de détection et les deuxièmes données de détection est plus grand.

12. Le système de mesure d'informations biologiques selon l'une quelconque des revendications précédentes, sachant que le dispositif de sortie (66, 68, 70) affiche un message relatif à une gestion de santé du sujet test sur la base du résultat d'analyse acquis par l'analyseur de données (60).

13. Le système de mesure d'informations biologiques selon l'une quelconque des revendications précédentes, sachant que le dispositif d'aspiration (18) et le détecteur de gaz (20) sont configurés pour aspirer du gaz et détecter les premières données de détection, respectivement, avant même que le dispositif d'identification de sujet test (62) identifie le sujet test qui utilise les W.C. (2), et sachant que le dispositif de stockage (22b) stocke des premières données de détection en association avec le sujet test identifié par le dispositif d'identification de sujet test (62) après que les premières données de détection sont acquises.

14. Le système de mesure d'informations biologiques selon l'une quelconque des revendications précédentes, sachant que si le sujet test n'entre pas d'informations d'identification du sujet test dans le dispositif d'identification de sujet test (62) pendant un temps prédéterminé après que le détecteur de gaz a acquis des premières données de détection, le dispositif de sortie (66, 68, 70) sort une notification visant à demander au sujet test d'entrer les informations.

15. Le système de mesure d'informations biologiques selon l'une quelconque des revendications précédentes, sachant que l'analyseur de données (60) effectue une analyse en assignant un coefficient de pondération plus élevé aux premières données de détection et aux deuxièmes données de détection, acquises par détection de gaz de défécation évacué précocement lors d'un acte de défécation du sujet test, qu'aux premières données de détection et aux deuxièmes données de détection acquises par détection de gaz de défécation évacué plus tard lors dudit acte de défécation.

16. Le système de mesure d'informations biologiques selon l'une quelconque des revendications précédentes, sachant que l'analyseur de données (60) effectue une analyse en utilisant uniquement les premières données de détection et les deuxièmes données de détection, acquises par détection de gaz de défécation évacué en premier lieu lors d'un acte de défécation du sujet test.

17. Le système de mesure d'informations biologiques selon l'une quelconque des revendications précédentes, sachant que le détecteur de gaz (20) est configuré pour être capable de détecter également un acide gras à chaîne courte évaporé contenu dans du gaz de défécation aspiré par le dispositif d'aspiration (18), et sachant que l'analyseur de données (60) analyse le premier indice sur la base de données de détection sur un gaz odoriférant, le deuxième indice sur la base de données de détection sur un gaz d'état sain, et un troisième indice sur la base de données de détection sur un acide gras à chaîne courte, comme état physique du sujet test.

18. Le système de mesure d'informations biologiques selon la revendication 17, sachant que l'analyseur de données (60) est configuré pour analyser si un état physique est bon ou mauvais sur la base du premier et du deuxième indice, et pour sortir le résultat d'analyse à l'attention du dispositif de sortie (66, 68, 70), et sachant que lorsqu'une valeur du troisième indice est grande, l'analyseur de données (60) sort le résultat d'analyse sur la base du premier et du deuxième indice qui est modifié plus vers le côté de bon état physique que lorsque la valeur du troisième indice est petite.

19. Le système de mesure d'informations biologiques selon la revendication 17 ou 18, sachant que le détecteur de gaz (20) est configuré pour être capable de détecter de l'acide acétique ou de l'acide propionique d'un acide gras à chaîne courte, et sachant que l'analyseur de données (60) analyse un état physique du sujet test sur la base d'une tendance au changement en fonction du temps dans des données de détection sur de l'acide acétique ou de l'acide propionique.

20. Le système de mesure d'informations biologiques selon l'une quelconque des revendications 17 à 19, comprenant en outre :
des moyens de détermination de diarrhée destinés à détecter si le sujet test a la diarrhée ou non, sachant que les moyens de détermination de diarrhée détectent une diarrhée sur la base d'un gaz d'acide gras à chaîne courte contenu dans du gaz de défécation aspiré par le dispositif d'aspiration (18), et sachant que si les moyens de détermination de diarrhée déterminent que le sujet test a la diarrhée, des données de détection sur un acide gras à chaîne courte, acquises lors de l'acte de défécation, ne sont pas utilisées pour une analyse d'un état physique, ou une pondération des données de détection est réduite.

21. Le système de mesure d'informations biologiques selon l'une quelconque des revendications précédentes, sachant que l'analyseur de données (60) analyse un état de santé actuel du sujet test et analyse également une résistance à une détérioration d'un état dans l'intestin du sujet test sur la base de données de détection sur un acide gras à chaîne courte.
